(19) Europäisches Patentamt
European Patent Office
Office européen des brevets

(11) EP 4 570 798 A1

(12) EUROPEAN PATENT APPLICATION
published in accordance with Art. 153(4) EPC

(43) Date of publication:
**18.06.2025 Bulletin 2025/25**

(21) Application number: **23851751.0**

(22) Date of filing: **07.08.2023**

(51) International Patent Classification (IPC):
***C07D 417/12*** (2006.01) ***C07D 211/06*** (2006.01)
***C07D 277/60*** (2006.01) ***C07D 221/00*** (2006.01)
***A61K 31/39*** (2006.01) ***A61K 31/4427*** (2006.01)
***A61P 25/16*** (2006.01) ***A61P 25/24*** (2006.01)
***A61P 25/22*** (2006.01)

(52) Cooperative Patent Classification (CPC):
**A61K 31/39; A61K 31/4427; A61P 25/16; A61P 25/22; A61P 25/24; C07D 211/06; C07D 221/00; C07D 277/60; C07D 417/12**

(86) International application number:
**PCT/CN2023/111410**

(87) International publication number:
**WO 2024/032530 (15.02.2024 Gazette 2024/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.08.2022 CN 202210961906**

(71) Applicants:
- **Jiangsu NHWA Pharmaceutical Co., Ltd**
  **Xuzhou, Jiangsu 221000 (CN)**
- **Shanghai Institute of Pharmaceutical Industry Co., Ltd.**
  **Shanghai, 200040 (CN)**

(72) Inventors:
- **WANG, Guan**
  **Xuzhou, Jiangsu 221000 (CN)**
- **HU, Zhijing**
  **Xuzhou, Jiangsu 221000 (CN)**
- **DU, Xinli**
  **Xuzhou, Jiangsu 221000 (CN)**
- **GE, Yuqiang**
  **Xuzhou, Jiangsu 221000 (CN)**
- **JIE, Peng**
  **Xuzhou, Jiangsu 221000 (CN)**
- **MA, Liang**
  **Xuzhou, Jiangsu 221000 (CN)**
- **LI, Jianqi**
  **Xuzhou, Jiangsu 221000 (CN)**
- **WAN, Zehong**
  **Xuzhou, Jiangsu 221000 (CN)**

(74) Representative: **Lavoix**
**Bayerstraße 83**
**80335 München (DE)**

(54) **AROMATIC HETEROCYCLIC CYCLOHEXYL AMINOALKYL PIPERIDINE DERIVATIVE, PREPARATION METHOD AND USE THEREOF**

(57) The present invention relates to an aromatic heterocycle-fused cyclohexyl aminoalkyl piperidine derivative, a preparation method and use thereof. Specifically, the present invention provides a compound of general formula (I), a stereoisomer thereof, a tautomer thereof, or a pharmaceutically acceptable salt thereof, and a preparation method therefor, use thereof for activating the activities of a 5-$HT_{1A}$ receptor and dopamine $D_2$ and $D_3$ receptors, and use thereof in the preparation of a medicament for Parkinson's disease.

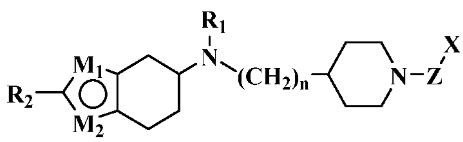

(I) .

EP 4 570 798 A1

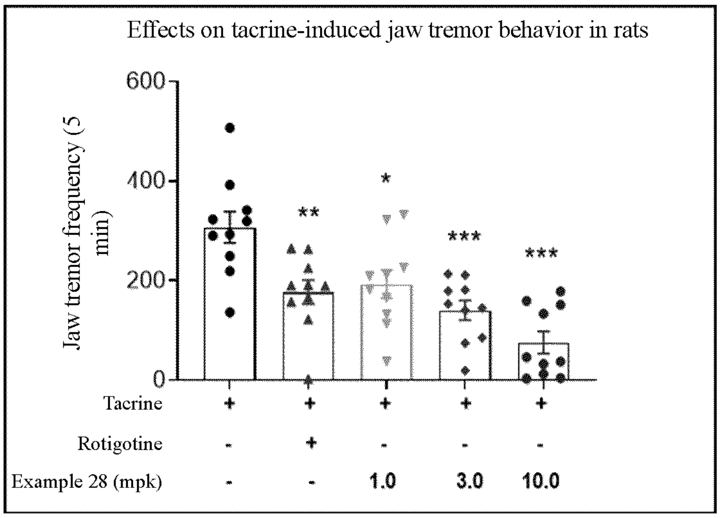
Effects on tacrine-induced jaw tremor behavior in rats
Jaw tremor frequency (5 min)
600
400
200
0
**
*
***
***
Tacrine
+ + + + +
Rotigotine
- + - - -
Example 28 (mpk)
- - 1.0 3.0 10.0

FIG. 1

## Description

The present application claims priority to Chinese Patent Application No. 2022109619064 filed on Aug. 11, 2022, which is incorporated herein by reference in its entirety.

## TECHNICAL FIELD

The present invention belongs to the field of pharmaceuticals, and particularly relates to an aromatic heterocycle-fused cyclohexyl aminoalkyl piperidine derivative, a preparation method and use thereof. More specifically, the present invention relates to an aromatic heterocycle-fused cyclohexyl aminoalkyl piperidine derivative, a preparation method therefor, a pharmaceutical composition comprising the aromatic heterocycle-fused cyclohexyl aminoalkyl piperidine derivative, and use of the aromatic heterocycle-fused cyclohexyl aminoalkyl piperidine derivative or the pharmaceutical composition thereof in the preparation of a medicament for preventing and/or treating a central nervous system disease in a mammal.

## BACKGROUND

Parkinson's disease (PD), the second most common neurodegenerative disorder in the world after Alzheimer's disease, often occurs in the elderly, with a prevalence rate of about 2% in the population over the age of 60 (Lilienfeld, D. E. Neuroepidemiology, 1993, 12, 219-228.). It is clinically characterized mainly by resting tremor, muscular rigidity, bradykinesia, and postural balance disturbance (Armstrong MJ. JAMA. 2020; 323(6):548-560.), and may be accompanied by non-motor symptoms such as autonomic dysfunction (Jost WH. J Neurol. 2003 Feb; 250 Suppl 1:128-30.), cognitive disorder (Halliday, G.M. Mov. Disord. 2014, 29, 634-650.), sleep disorder and mood disorder (Gallagher, D.A. Neurobiol. Dis. 2012, 46, 581-589.), and the like. From a pathological perspective, the degeneration of dopamine neurons in the substantia nigra pars compacta leads to a significant decrease in dopamine levels in the striatum, and the accumulation of $\alpha$-synuclein forms Lewy bodies (Schapira, A. H. Neurol. Clin. 2009, 27, 583-603.), thereby causing various motor disorders. Non-motor disorders, in addition to being affected by the dopamine pathway, are also associated with the degeneration of cholinergic, noradrenergic, serotonergic, and other neural pathways (Titova N. Med J Aust 2018; 208:404-409.) (Titova N. J Neural Transm (Vienna) 2017; 124:907-914.).

Currently, drug therapy for Parkinson's focuses primarily on dopamine strategies, including levodopa (L-DOPA), monoamine oxidase B inhibitors (MAOIs), catechol-O-methyltransferase inhibitors (COMTIs), and dopamine agonists (DAs). L-DOPA has long been the most commonly used drug for Parkinson's disease and remains the gold standard for treating this disease. MAOIs and COMTIs are commonly used as adjuncts to L-DOPA, prolonging its therapeutic effect by blocking dopamine metabolism (Jankovic J. Journal of Neurology, Neurosurgery & Psychiatry 2020; 91:795-808.) (Armstrong MJ. JAMA. 2020; 323(6):548-560.). Despite the clear benefits for symptomatic treatment, long-term use of L-DOPA often leads to motor fluctuations (on-off phenomenon) and dyskinesias (Marsden, C. D. Lancet 1976, 307, 292-296.).

Dopamine agonists directly stimulate $D_2$-like receptors ($D_2$, $D_3$, and $D_4$) or $D_1$ receptors to improve motor symptoms (Hisahara S. Int J Med Chem. 2011; 2011:403039.). Although dopamine agonists are less effective than L-DOPA, monotherapy with dopamine agonists can adequately control motor symptoms in the early stages of Parkinson's, so most patients choose dopamine agonists over L-DOPA for their initial treatment (Jankovic J. Journal of Neurology, Neurosurgery & Psychiatry 2020; 91:795-808.). In the advanced stages of the disease, dopamine agonists are used in combination with L-DOPA to reduce the "off" time (Marsden, C. D. Lancet 1976, 307,292-296.). Dopamine agonists can also alleviate some non-motor symptoms. For example, pramipexole can effectively treat depressive symptoms, while ropinirole has beneficial effects on sleep, anxiety, and depression. The efficacy of rotigotine in ameliorating swallowing dysfunction has been demonstrated, and apomorphine is effective for mood, gastrointestinal, and urinary dysfunctions (Torti M. Drugs. 2019 May; 79(7):693-703.). 5-$HT_{1A}$ receptors also play an important role in the drug therapy for Parkinson's, mainly reflecting in three aspects. Firstly, 5-$HT_{1A}$ receptor agonists, such as eltoprazine and NLX-112, can ameliorate extrapyramidal disorders caused by the degeneration of dopamine neurons and dyskinesias caused by L-DOPA (Cerri S. Expert Opin Investig Drugs. 2017 Jul; 26(7):777-791.). Secondly, the activation of 5-$HT_{1A}$ receptors contributes to the amelioration of cognitive disorder and has anti-anxiety and anti-depression effects, such as when treated with aripiprazole (Ohno Y. Prog Neurobiol. 2015 Nov; 134:104-21.). In addition, various models have shown that 5-$HT_{1A}$ receptor agonists, such as BAY-639044, can prevent neurotoxicity, which means that they can control disease progression by protecting nerves from damage. Miyazaki et al. have demonstrated that the activation of 5-$HT_{1A}$ receptors can induce astrocyte proliferation and increase antioxidant molecule levels in the striatum, thereby preventing progressive dopaminergic neuronal degeneration (Miyazaki I. Curr Med Chem. 2016; 23(7):686-700.).

Furthermore, 5HT has been found in the aqueous humor of the human eye (Martin et al, Ophthalmol., 95:1221-1226, 1988.). Receptor binding sites for [$^3$H]5HT have been demonstrated and pharmacologically characterized

in the iris-ciliary body (ICB) of rabbits (Mallorga and Sugrue, Curr. Eye Res., 6:527-532, 1987 and Chidlow et al., Invest. Ophthalmol. Vis. Sci., 36:2238-2245, 1995.). It has been shown that these 5HT binding sites are functionally coupled to second messengers produced by rabbits (Tobin and Osborne, J. Neurochem., 53:686-601, 1989 and Tobin et al., J. Neurosci, supra.). In the human ICB, these binding sites are characterized as $5HT_{1A}$ and $5HT_2$ receptors (Barnet and Osborne, Exp. Eye Res., 57:209-216, 1993.). Additionally, the presence of mRNAs for $5HT_{1A}$ receptors in the ICB of rabbits has been reported (Chidlow et al., Invest. Ophthalmol. Vis. Sci., supra and Osborne and Chidlow, Ophthalmologica, 210:308-314, 1996.).

Studies conducted on rabbits with 8-hydroxyl-DPAT and MKC-242 ($5HT_{1A}$ agonists) have shown that these $5HT_{1A}$ agonists are capable of lowering IOP (Osborne and Chidlow, Ophthalmologica, 210:308-319, 1996, EP 0771563A2.). In addition, 5-methylurapidil (a $5HT_{1A}$ agonist) is capable of lowering IOP in monkeys suffering from glaucoma (Wang, et al., Curr. Eye Res., 16:679-775, 1997.). U.S. Patent 5693654 discloses a $5HT_1$ receptor agonist for lowering IOP. WO 92/20333 discloses certain $5HT_{1A}$ agonists for treating glaucoma.

Eltoprazine is a $5HT_{1A/1B}$ agonist ($EC_{50}$ = 148 nM), approved by the U.S. FDA in 2016 as an orphan drug for treating L-DOPA-induced dyskinesia (LID), and is currently in phase II clinical trials, showing good therapeutic effect and safety in the treatment of cognitive disorder and dyskinesia (Cabedo N. Journal of Medicinal Chemistry, 2001, 44(11): 1794-1801).

NLX-112, as a selective $5HT_{1A}$ agonist ($EC_{50}$ = 3.3 nM, $E_{max}$ = 84%), is used for treating L-DOPA-induced dyskinesia (LID) in Parkinson's disease (PD), and is currently in phase II clinical trials conducted by Neurolixis. The literature (Noureddine El Aouad. European Journal of Medicinal Chemistry, 44(11):4616-4621.) reports that NLX-112 exhibits novel therapeutic properties, with significant anti-dyskinesia functions without compromising the therapeutic efficacy of L-DOPA, and also has ameliorating effects on non-motor mental-emotional symptoms, such as anti-depression and anti-anxiety effects, and the like.

Furthermore, $5\text{-}HT_{1A}$ receptor agonists primarily alleviate motor and non-motor disorders through non-dopaminergic mechanisms, which implies that a multi-target approach combining both dopaminergic and serotonergic receptor therapeutic effects will provide dual benefits for the treatment of Parkinson's (Ohno Y. Prog Neurobiol. 2015 Nov; 134:104-21.) (Shimizu S. Aging Dis. 2013 Feb; 4(1):1-13.). Currently, molecules with such multi-target characteristics have demonstrated clinical efficacy.

SOMCL-171 ($D_2$: $EC_{50}$ = 873 nM, $E_{max}$ = 74%; $5\text{-}HT_{1A}$: $EC_{50}$ = 175 nM, $E_{max}$ = 83%) exhibits anti-Parkinson's effects as a $D_2$/$5\text{-}HT_{1A}$ agonist in a 6-OHDA-lesioned rat model, and it can also mitigate the development of LID (L-DOPA-induced dyskinesia) without impairing the therapeutic effect on Parkinson's (Zhao R, Lu W, Fang X, et al. 2014 Sep; 124:204-10.).

Bifeprunox ($D_2$: $EC_{50}$ = 39.8 nM; $D_3$: $IC_{50}$ = 15.2 nM; $5\text{-}HT_{1A}$: $EC_{50}$ = 47.8 nM) is an anti-Parkinson's drug under development by Solvay, and is currently in phase III clinical trials. Clinical trials have shown that it has effects in improving motor symptoms and alleviating psychiatric symptoms such as depression, anxiety, and the like (Wang, Q. Neuropharmacology, 2019, 148:1-10.).

Pardoprunox (SLV-308) ($D_2$: $EC_{50}$ = 10 nM; $D_3$: $EC_{50}$ = 0.6 nM; $5\text{-}HT_{1A}$: $EC_{50}$ = 500 nM) is a $D_2$/$D_3$/$5\text{-}HT_{1A}$ agonist with anti-Parkinson's, anti-depression and anti-anxiety effects (Jones CA. Eur Neuropsychopharmacol. 2010 Aug; 20(8):582-93.). In addition, compared to single-target dopamine agonists, it has a lower tendency to cause side effects such as dyskinesias and the like, and has entered phase III clinical trials for the treatment of Parkinson's (Glennon, J. C. Synapse 2006, 60, 599-608.).

In addition, the article published by Swati Biswas in 2008 (Biswas S. J Med Chem. 2008; 51(10):3005-3019.) discloses a class of 2-aminothiazole derivatives with selective $D_3$ agonistic effect, where compounds (-)31 and (-)33, which are relatively similar to the compound of the present patent, have the following structure:

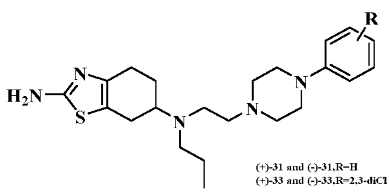

(+)-31 and (-)-31,R=H
(+)-33 and (-)-33,R=2,3-diCl

These compounds exhibit a certain affinity for $D_2$ and $D_3$ receptors and can be used for treating Parkinson's disease. Compound (-)31 has binding affinity $K_i$ values for $D_{2L}$ and $D_3$ receptors of 1979 ± 567 nm and 44.0 ± 10.6 nm, respectively, with a ratio of 58.6; compound (-)33 has agonistic potency $EC_{50}$ values for $D_{2L}$ and $D_3$ receptors of 13.4 ± 2.4 (78.8 ± 0.8%) and 0.06 ± 0.015 (95.7 ± 10.7%), respectively, with a ratio of 223. JP2005104885A discloses a novel thiazole derivative that exhibits an agonistic effect on both dopamine $D_2$ and 5-hydroxytryptamine $5\text{-}HT_{1A}$ receptors, where the compound in Example 19, which is structurally similar to the compound of the present patent, has the following structure, and the technical effects of this compound are not described in the specification:

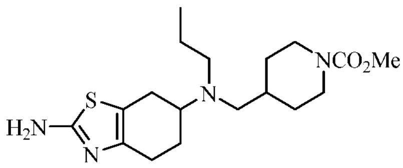

.

In conclusion, given the multi-target synergistic effects on dopamine $D_2$, $D_3$, and/or 5-$HT_{1A}$ receptors, multi-target chemical small molecules with activity for dopamine $D_2$, $D_3$, and/or 5-$HT_{1A}$ receptors are expected to exhibit new clinical therapeutic characteristics in the aspects of overcoming dyskinesias, ameliorating cognitive impairment, and ameliorating mental-emotional symptoms such as anxiety, depression, and the like, while treating the main motor symptoms of Parkinson's. Especially, dopamine $D_2$, $D_3$, and/or 5-$HT_{1A}$ multi-target agonists have become an important direction in the global development of new drugs against Parkinson's, depression, schizophrenia, and other conditions, in terms of the research, development and application of drugs. The research in this field possesses novelty, creativity, and significant scientific value.

## SUMMARY

The technical problem to be solved by the present invention is to provide an aromatic heterocycle-fused cyclohexyl aminoalkyl piperidine derivative, a preparation method and use thereof, and particularly, the present invention aims to develop a novel anti-Parkinson's disease drug that has full agonistic effects on $D_2$, $D_3$, and 5-$HT_{1A}$ receptors simultaneously, so as to effectively treat motor and non-motor disorders, reduce various toxic and side effects, and overcome the main defects of existing drugs, such as the poor efficacy on non-motor disorders and the presence of LID side effect in treatment with $D_2$ and $D_3$ dual full agonists, the limited anti-Parkinson's dyskinesia effect of selective $D_3$ agonists, the poor therapeutic effect on Parkinson's of $D_2$ and $D_3$ dual partial agonists, and the like.

The present invention aims to provide a compound of general formula (I), a stereoisomer thereof, a tautomer thereof, or a pharmaceutically acceptable salt thereof:

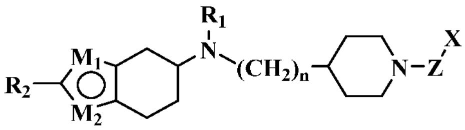

**(I)**

wherein

X is selected from amino, $C_{1-6}$ alkyl, $C_{2-6}$ alkenyl, $C_{2-6}$ alkynyl, $C_{3-8}$ cycloalkyl, 3- to 8-membered heterocyclyl, $C_{6-10}$ aryl, 5- to 10-membered heteroaryl, $C_{6-10}$ aryl-fused $C_{3-8}$ cycloalkyl, $C_{6-10}$ aryl-fused 3- to 8-membered heterocyclyl, $C_{6-10}$ aryl-fused 5- to 10-membered heteroaryl, 5- to 10-membered heteroaryl-fused $C_{3-8}$ cycloalkyl, or 5- to 10-membered heteroaryl-fused 3- to 8-membered heterocyclyl, and optionally further substituted with one or more R;

R is selected from hydrogen, deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy, wherein the $C_{1-6}$ alkyl, $C_{1-6}$ haloalkyl, $C_{1-6}$ alkoxy, or $C_{1-6}$ haloalkoxy is optionally further substituted with one or more substituents selected from deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy;

Z is selected from a bond, $-(CR_{aa}R_{bb})_m-$, $-C(O)(CR_{aa}R_{bb})_m-$, $-O(CR_{aa}R_{bb})_m-$, - $(CR_{aa}R_{bb})_mO-$, $-(CR_{aa}R_{bb})_mN(R_{cc})-$, $-S(O)(CR_{aa}R_{bb})_m-$, $-S(O)_2(CR_{aa}R_{bb})_m-$, - $C(O)(CR_{aa}R_{bb})_mN(R_{cc})-$, $-C(S)(CR_{aa}R_{bb})_mN(R_{cc})-$, or $-C(S)(CR_{aa}R_{bb})_m-$;

$M_1$ and $M_2$ are each independently selected from $CR_{aa}$, N, O, or S;

$R_{aa}$ and $R_{bb}$ are each independently selected from hydrogen, deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy, wherein the $C_{1-6}$ alkyl, $C_{1-6}$ haloalkyl, $C_{1-6}$ alkoxy, or $C_{1-6}$ haloalkoxy is optionally further substituted with one or more substituents selected from deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy;

$R_{cc}$ is selected from hydrogen, deuterium, $C_{1-6}$ alkyl, $C_{1-6}$ haloalkyl, $C_{1-6}$ alkoxy, or $C_{1-6}$ haloalkoxy, wherein the $C_{1-6}$ alkyl, $C_{1-6}$ haloalkyl, $C_{1-6}$ alkoxy, or $C_{1-6}$ haloalkoxy is optionally further substituted with one or more substituents

selected from deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy;

$R_1$ is selected from hydrogen, deuterium, $C_{1-6}$ alkyl, $C_{2-6}$ alkenyl, $C_{2-6}$ alkynyl, $C_{1-6}$ haloalkyl, $C_{1-6}$ alkoxy, or $C_{1-6}$ haloalkoxy, wherein the $C_{1-6}$ alkyl, $C_{2-6}$ alkenyl, $C_{2-6}$ alkynyl, $C_{1-6}$ haloalkyl, $C_{1-6}$ alkoxy, or $C_{1-6}$ haloalkoxy is optionally further substituted with one or more substituents selected from deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy;

$R_2$ is selected from hydroxyl, amino, sulfydryl, $C_{1-6}$ alkyl, $C_{1-6}$ haloalkyl, $C_{1-6}$ alkoxy, or $C_{1-6}$ haloalkoxy;

n is selected from 0, 1, 2, 3, or 4; and

m is selected from 0, 1, 2, 3, 4, 5, or 6.

In a further preferred embodiment of the present invention,

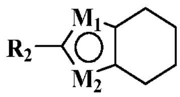

in general formula (I) is selected from the following groups:

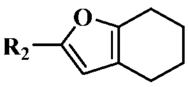, 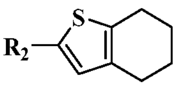, 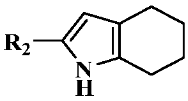, 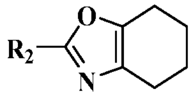, 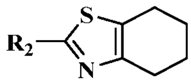,

or

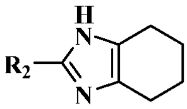,

preferably

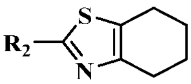.

In a further preferred embodiment of the present invention, X is selected from amino, $C_{3-8}$ cycloalkyl, 3- to 8-membered heterocyclyl, $C_{6-10}$ aryl, 5- to 10-membered heteroaryl, or $C_{6-10}$ aryl-fused 5- to 10-membered heteroaryl, and optionally further substituted with one or more R, and preferably X is selected from amino, $C_{4-6}$ cycloalkyl, 4- to 6-membered heterocyclyl, $C_{6-10}$ aryl, 5- to 10-membered heteroaryl, or benzo-fused 5- to 6-membered heteroaryl, and optionally further substituted with one or more R.

In a further preferred embodiment of the present invention, R is selected from hydrogen, deuterium, halogen, hydroxyl, amino, cyano, $C_{1-6}$ alkyl, $C_{1-6}$ haloalkyl, $C_{1-6}$ alkoxy, or $C_{1-6}$ haloalkoxy, preferably hydrogen, deuterium, halogen, cyano, or $C_{1-6}$ alkyl, more preferably hydrogen, deuterium, fluorine, chlorine, bromine, cyano, or $C_{1-3}$ alkyl, and further preferably hydrogen, deuterium, fluorine, chlorine, cyano, or methyl.

In a further preferred embodiment of the present invention, Z is selected from a bond, - $(CH_2)_m$-, -C(O)-, -C(O)$(CH_2)_m$-, -S(O)2-, -$S(O)_2(CH_2)_m$-, or -C(O)N($CH_3$)-, preferably a bond, -C(O)-, -$S(O)_2$-, or -C(O)N($CH_3$)-.

In a further preferred embodiment of the present invention, $R_1$ is selected from hydrogen, deuterium, $C_{1-6}$ alkyl, $C_{2-6}$ alkenyl, $C_{2-6}$ alkynyl, or $C_{1-6}$ alkoxy, preferably hydrogen, deuterium, or $C_{1-6}$ alkyl, more preferably hydrogen, deuterium, or $C_{1-3}$ alkyl, and further preferably hydrogen, deuterium, methyl, ethyl, or propyl.

In a further preferred embodiment of the present invention, $R_2$ is selected from hydroxyl, amino, $C_{1-6}$ alkyl, or $C_{1-6}$ haloalkyl, preferably hydroxyl, amino, $C_{1-3}$ alkyl, or $C_{1-3}$ haloalkyl, more preferably hydroxyl or amino, and further preferably amino.

In a further preferred embodiment of the present invention, n is selected from 0, 1, 2, or 3, preferably 0, 1, or 2.

In a further preferred embodiment of the present invention, X is selected from the following groups:

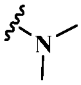, 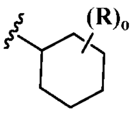, 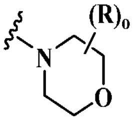, 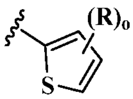, 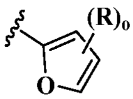, 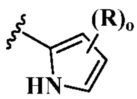,

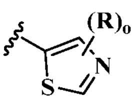, 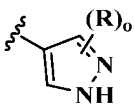, 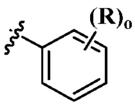, 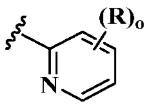, 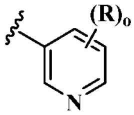, 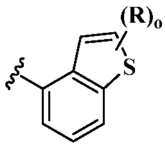,

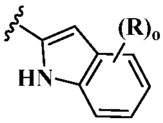, 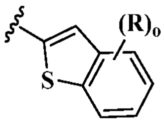, or 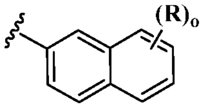,

wherein
o is selected from 0, 1, 2, 3, 4, or 5; and R is as defined above.

In a further preferred embodiment of the present invention, X is selected from the following groups:

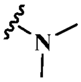, 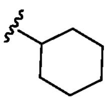, 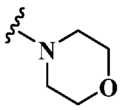, 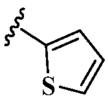, 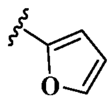, 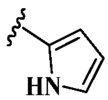, 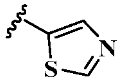,

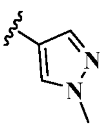, 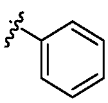, 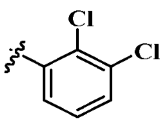, 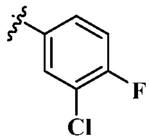, 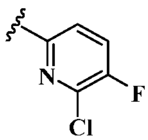, 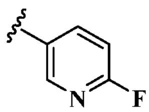, 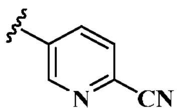,

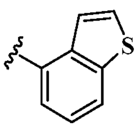, 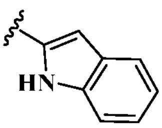, 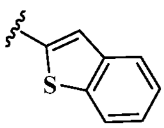, or 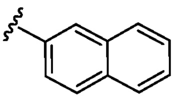.

In a further preferred embodiment of the present invention, general formula (I) further has a structure represented by general formula (II):

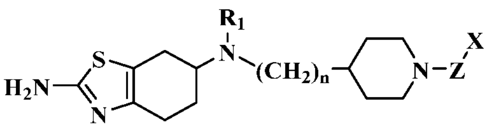

(II)

wherein
X, Z, $R_1$, and n are as defined above.

In a further preferred embodiment of the present invention, general formula (I) further has a structure represented by general formula (III):

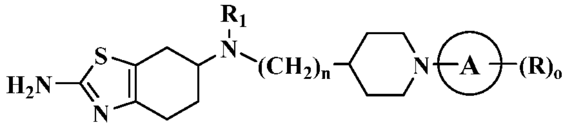

(III)

wherein

ring A is selected from $C_{6-10}$ aryl, 5- to 10-membered heteroaryl, or $C_{6-10}$ aryl-fused 5- to 10-membered heteroaryl, preferably $C_{6-10}$ aryl or benzo-fused 5- to 6-membered heteroaryl, and more preferably phenyl or benzothienyl; and R, $R_1$, n, and o are as defined above.

In a further preferred embodiment of the present invention,

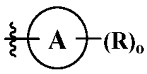

in general formula (III) is selected from the following groups:

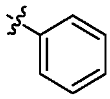 , 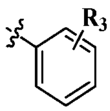 , 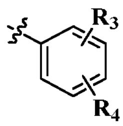 ,

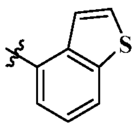 , 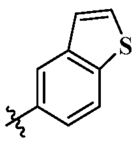 , 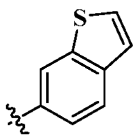 , or 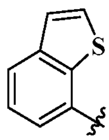 ,

preferably

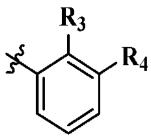 or 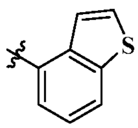 ,

wherein

$R_3$ and $R_4$ are each independently selected from halogen, hydroxyl, amino, cyano, $C_{1-6}$ alkyl, $C_{1-6}$ haloalkyl, $C_{1-6}$ alkoxy, or $C_{1-6}$ haloalkoxy, preferably halogen, cyano, or $C_{1-6}$ alkyl, more preferably fluorine, chlorine, or bromine, and further preferably chlorine.

In a further preferred embodiment of the present invention,

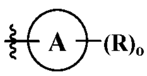

in general formula (III) is selected from the following groups:

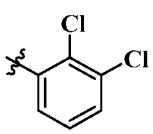 or 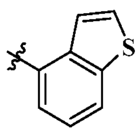 .

In a further preferred embodiment of the present invention, general formula (I) further has a structure represented by general formula (IV):

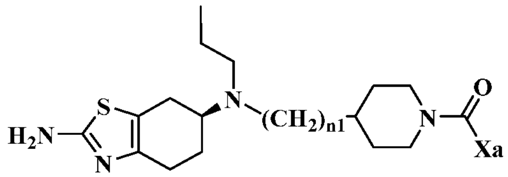

(IV)

wherein

Xa is selected from amino, $C_{3-8}$ cycloalkyl, 3- to 8-membered heterocyclyl, $C_{6-10}$ aryl, or 5- to 10-membered heteroaryl, and optionally further substituted with one or more $R_a$, and preferably Xa is selected from amino, $C_{4-6}$ cycloalkyl, 4- to 6-membered heterocyclyl, $C_{6-10}$ aryl, or 5- to 10-membered heteroaryl, and optionally further substituted with one or more $R_a$;

$R_a$ is selected from hydrogen, deuterium, halogen, hydroxyl, amino, cyano, $C_{1-6}$ alkyl, $C_{1-6}$ haloalkyl, $C_{1-6}$ alkoxy, or $C_{1-6}$ haloalkoxy, preferably hydrogen, deuterium, halogen, cyano, or $C_{1-6}$ alkyl, more preferably hydrogen, deuterium, fluorine, chlorine, bromine, cyano, or $C_{1-3}$ alkyl, and further preferably hydrogen, deuterium, fluorine, chlorine, cyano, or methyl; and

n1 is selected from 0, 1, 2, 3, or 4, preferably 0, 1, or 2, and more preferably 1.

In a further preferred embodiment of the present invention, Xa is selected from the following groups:

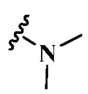, 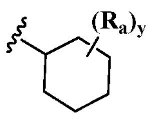, 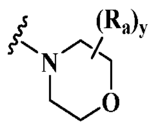, 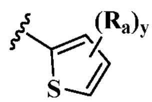, 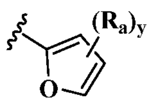,

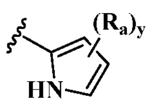, 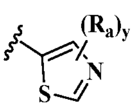, 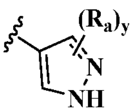, 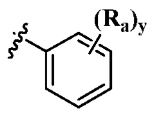, 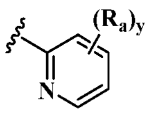, 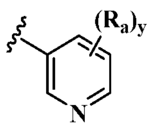,

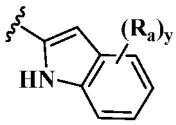, 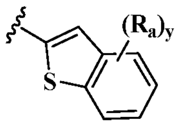, or 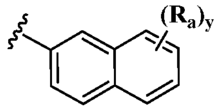,

wherein
y is selected from 0, 1, 2, or 3, preferably 0, 1, or 2.

In a further preferred embodiment of the present invention, Xa is selected from the following groups:

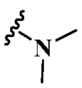, 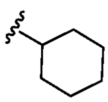, 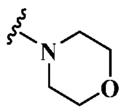, 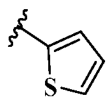, 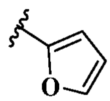, 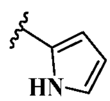, 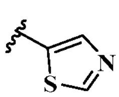,

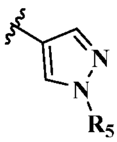, 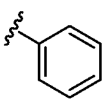, 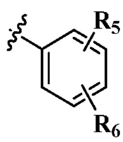, 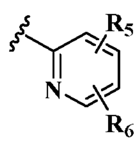, 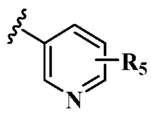, 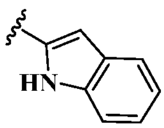, 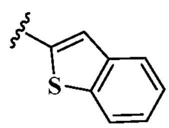,

or

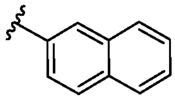,

wherein $R_5$ and $R_6$ are each independently selected from halogen, hydroxyl, amino, cyano, $C_{1-6}$ alkyl, $C_{1-6}$ haloalkyl, $C_{1-6}$ alkoxy, or $C_{1-6}$ haloalkoxy, preferably halogen, cyano, or $C_{1-6}$ alkyl, more preferably fluorine, chlorine, bromine, cyano, or $C_{1-3}$ alkyl, and further preferably fluorine, chlorine, cyano, or methyl.

In a further preferred embodiment of the present invention, Xa is selected from the following groups,:

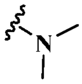, 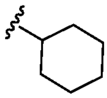, 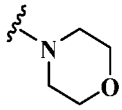, 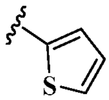, 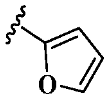, 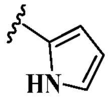, 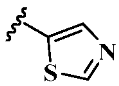,

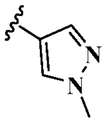, 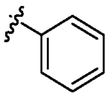, 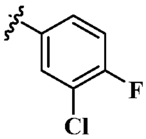, 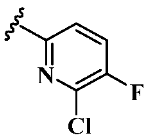, 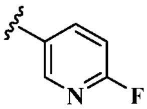, 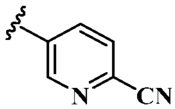, 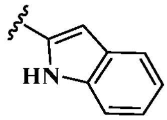,

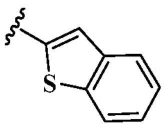, or 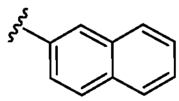.

In a further preferred embodiment of the present invention, the compound is selected from:

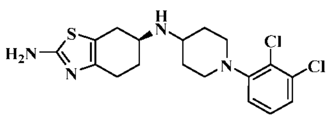
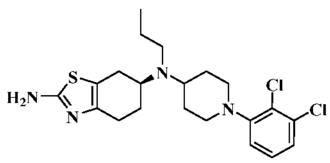
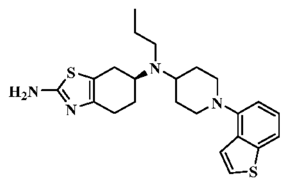
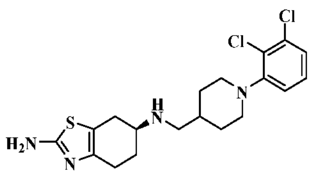
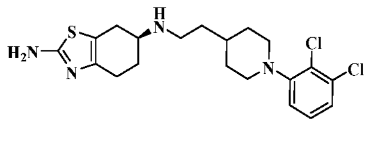
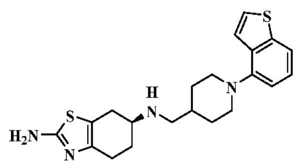
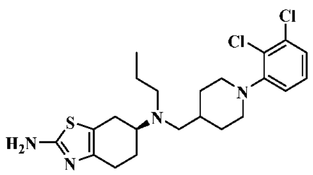
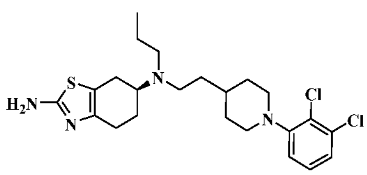
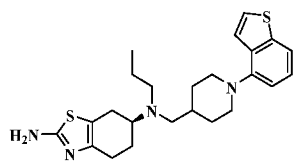
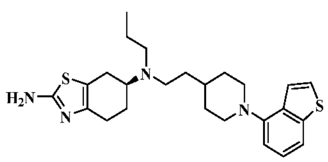
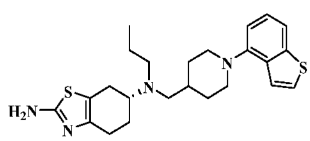
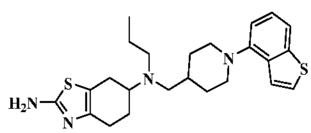

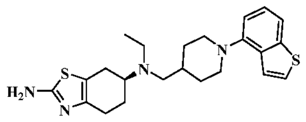
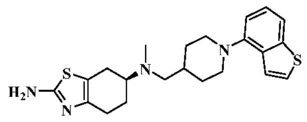
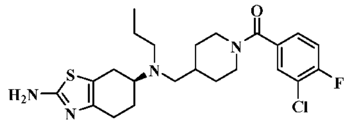
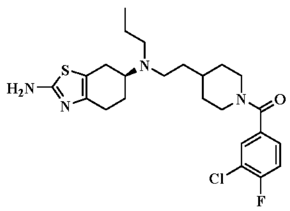
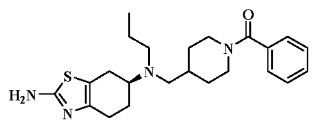
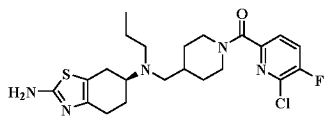
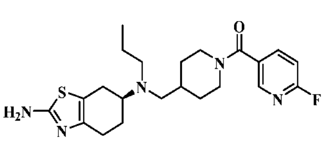
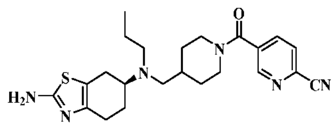
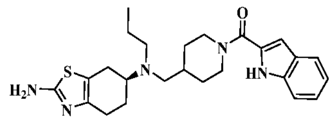
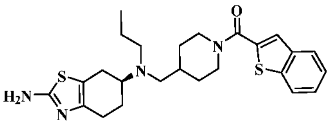
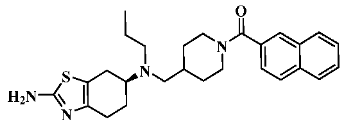
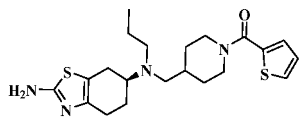
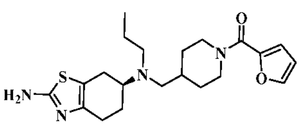
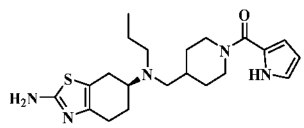
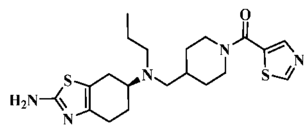
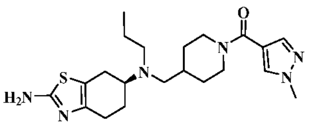
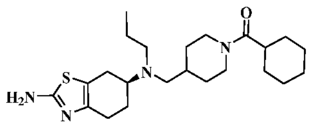
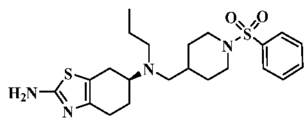
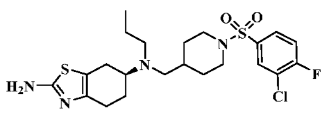
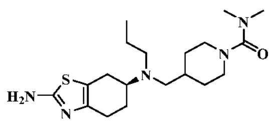
or
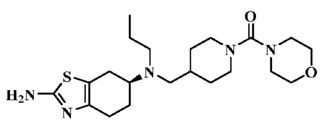
.

The present invention further provides a compound of general formula (V), a stereoisomer thereof, a tautomer thereof, or a pharmaceutically acceptable salt thereof:

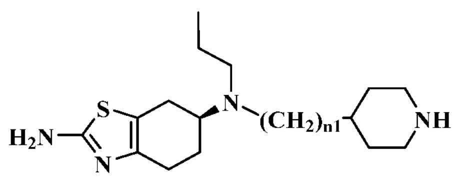
(V)

wherein

n1 is selected from 0, 1, 2, 3, or 4, preferably 0, 1, or 2, and more preferably 1.

The present invention further provides a method for preparing the compound of general formula (IV), the stereoisomer thereof, the tautomer thereof, or the pharmaceutically acceptable salt thereof, comprising the following step:

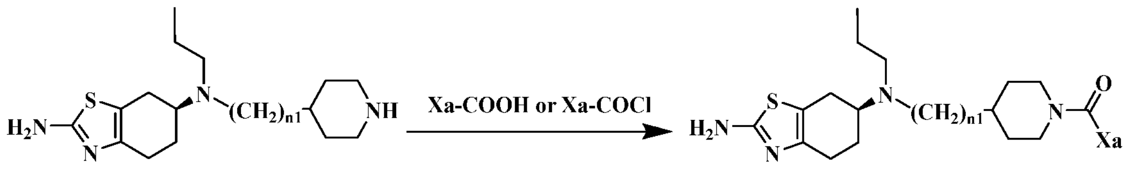


(V)

(IV)

conducting a condensation reaction of a compound of general formula (V) with a substituted carboxylic acid or acyl chloride to obtain the compound of general formula (IV), the stereoisomer thereof, the tautomer thereof, or the pharmaceutically acceptable salt thereof,

wherein n1 and Xa are as described in general formula (IV).

The present invention further provides a pharmaceutical composition comprising a therapeutically effective amount of the compound of each of the general formulas described above, the stereoisomer thereof, the tautomer thereof, or the pharmaceutically acceptable salt thereof, and at least one pharmaceutical adjuvant of a pharmaceutically acceptable carrier, diluent or excipient.

In some embodiments of the present invention, the pharmaceutical composition described above may be prepared according to any method well known in the art. The carrier refers to a carrier conventional in the pharmaceutical field, for example, fillers or binders such as cellulose derivatives, gelatin, polyvinylpyrrolidone, and the like, etc.; diluents such as water and the like; excipients such as starch and the like; disintegrants such as calcium carbonate and sodium bicarbonate; and lubricants such as calcium stearate or magnesium stearate, and the like. In addition, other auxiliary agents such as sweetening agents, fragrances, or colorants may also be added to the composition.

In some embodiments of the present invention, the pharmaceutical composition may be administered in any of the following ways: oral administration, spray inhalation, rectal administration, nasal administration, buccal administration, topical administration, parenteral administration such as subcutaneous, intravenous, intramuscular, intraperitoneal, intrathecal, intraventricular, intrasternal, or intracranial injection or infusion, or administration by means of an implantable reservoir. Oral administration is the preferred way.

When being administered orally, the compound of the present application may be prepared into any orally acceptable formulation forms, including, but not limited to, tablets, capsules, aqueous solutions, aqueous suspensions, etc.

The present invention further provides a preferred embodiment, which relates to use of the compound of each of the general formulas, the stereoisomer thereof, the tautomer thereof, or the pharmaceutically acceptable salt thereof, or the pharmaceutical composition in the preparation of a medicament.

The present invention further provides a preferred embodiment, which relates to use of the compound of each of the general formulas, the stereoisomer thereof, the tautomer thereof, or the pharmaceutically acceptable salt thereof, or the pharmaceutical composition in the preparation of a medicament involving or regulating a 5-hydroxytryptamine receptor and/or a dopamine receptor, preferably in the preparation of a medicament involving or regulating a 5-$HT_{1A}$ receptor, a dopamine $D_2$ receptor, and/or a dopamine $D_3$ receptor.

The present invention further provides use of the compound of each of the general formulas described above, the stereoisomer thereof, the tautomer thereof, or the pharmaceutically acceptable salt thereof, or the pharmaceutical composition in the preparation of a medicament for treating a central nervous system disease.

The present invention further provides use of the compound of each of the general formulas described above, the stereoisomer thereof, the tautomer thereof, or the pharmaceutically acceptable salt thereof, or the pharmaceutical composition in the treatment of a central nervous system disease.

In a preferred embodiment of the present invention, the central nervous system disease is selected from one or more of Parkinson's disease, schizophrenia, bipolar disorder, depression, anxiety, mania, Huntington's disease, Alzheimer's disease, senile dementia, dementia of the Alzheimer's type, memory disorder, loss of executive function, vascular dementia, neuropathic pain and dysfunctional diseases related to intelligence, learning, or memory, glaucoma, age-related macular degeneration, optic neuritis, ischemic disorder, and retinal edema, more preferably Parkinson's disease.

Detailed Description of the Present Invention

Unless otherwise stated, the terms used in the specification and claims have the following meanings.

The compound of the present invention may exist in the form of a specific geometric isomer or stereoisomer. All such compounds are contemplated herein, including *cis* and *trans* isomers, (-)- and (+)-enantiomers, (R)- and (S)-enantiomers, diastereoisomers, (D)-isomers, (L)-isomers, and racemic mixtures and other mixtures thereof, such as enantiomer or diastereomer enriched mixtures, all of which are encompassed within the scope of the present invention. Additional asymmetric carbon atoms may be present in substituents such as alkyl. All these isomers and mixtures thereof

are encompassed within the scope of the present invention. In certain embodiments, preferred compounds are those isomer compounds that exhibit superior biological activity. Purified or partially purified isomers and stereoisomers, or racemic or diastereomeric mixtures of the compounds of the present invention are also encompassed within the scope of the present invention. Purification and separation of such substances can be accomplished by standard techniques known in the art.

Unless otherwise stated, the term "enantiomer" or "optical isomer" refers to stereoisomers that are mirror images of each other.

Unless otherwise stated, the term *"cis-trans* isomer" or "geometric isomer" results from the inability of a single bond of a ring carbon atom or a double bond to rotate freely. Unless otherwise stated, the term "diastereomer" refers to stereoisomers whose molecules have two or more chiral centers and are not mirror images of each other.

The compound of the present invention may be present in a particular form. Unless otherwise stated, the term "tautomer" or "tautomeric form" means that different functional isomers are in dynamic equilibrium at room temperature and can be rapidly converted into each other. If tautomers are possible (e.g., in solution), the chemical equilibrium of the tautomers can be achieved. For example, a proton tautomer, also known as a prototropic tautomer, includes the interconversion by proton transfer, such as keto-enol isomerization and imine-enamine isomerization. A valence tautomer includes the interconversion by recombination of some bonding electrons. A specific example of the keto-enol tautomerization is the interconversion between tautomers pentane-2,4-dione and 4-hydroxypent-3-en-2-one.

The term "alkyl" refers to a linear or branched saturated aliphatic hydrocarbon group consisting of carbon atoms and hydrogen atoms, which is attached to the rest of the molecule through a single bond. The "alkyl" may have 1-8 carbon atoms, that is, it may be "C1-C8 alkyl", such as C1-4 alkyl, C1-3 alkyl, C1-2 alkyl, C3 alkyl, C4 alkyl, C1-6 alkyl, or C3-6 alkyl. Non-limiting examples of the alkyl include, but are not limited to, methyl, ethyl, propyl, butyl, pentyl, hexyl, isopropyl, isobutyl, sec-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof. The alkyl may be optionally substituted or unsubstituted. When it is substituted, the substituent may attach at any available attachment site, and the substituent is preferably one or more of the following groups independently selected from deuterium, alkyl, alkoxy, haloalkyl, haloalkoxy, halogen, sulfydryl, hydroxyl, nitryl, amino, or cyano, wherein the alkyl, haloalkyl, alkoxy, or haloalkoxy is optionally further substituted with one or more substituents selected from deuterium, halogen, hydroxyl, amino, sulfydryl, nitryl, cyano, alkyl, haloalkyl, alkoxy, or haloalkoxy.

The term "cycloalkyl" refers to a saturated or partially unsaturated monocyclic or polycyclic hydrocarbon substituent. The cycloalkyl contains 3 to 20 carbon atoms, that is, it may be "C3-C20 cycloalkyl", such as C3-18 cycloalkyl, C3-16 cycloalkyl, C3-12 cycloalkyl, C3-8 cycloalkyl, C3-6 cycloalkyl, C3-5 cycloalkyl, C3-4 cycloalkyl, C4-8 cycloalkyl, C4-6 cycloalkyl, or C5-6 cycloalkyl, preferably C3-8 cycloalkyl, C3-6 cycloalkyl, C3-5 cycloalkyl, or C3-4 cycloalkyl. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like; polycyclic cycloalkyl includes spiro cycloalkyl, fused cycloalkyl, and bridged cycloalkyl. The cycloalkyl may be optionally substituted or unsubstituted. When it is substituted, the substituent is preferably one or more of the following groups independently selected from deuterium, alkyl, haloalkyl, alkoxy, haloalkoxy, halogen, sulfydryl, hydroxyl, nitryl, amino, or cyano, wherein the alkyl, haloalkyl, alkoxy, or haloalkoxy is optionally further substituted with one or more substituents selected from deuterium, halogen, hydroxyl, amino, sulfydryl, nitryl, cyano, alkyl, haloalkyl, alkoxy, or haloalkoxy.

The term "heterocyclyl" refers to a saturated or unsaturated monocyclic or polycyclic hydrocarbon substituent containing 3 to 20 ring atoms, wherein one or more of the ring atoms are heteroatoms selected from nitrogen, oxygen, and $S(O)_m$ (wherein m is an integer of 0-2), excluding a ring portion of -O-O-, -O-S-, or -S-S-, and the remaining ring atoms are carbon atoms. That is, the heterocyclyl may be "3- to 20-membered heterocyclyl", such as 3- to 18-membered heterocyclyl, 3- to 16-membered heterocyclyl, 3- to 12-membered heterocyclyl, 3- to 8-membered heterocyclyl, 3- to 6-membered heterocyclyl, 3- to 5-membered heterocyclyl, 3- to 4-membered heterocyclyl, 4- to 8-membered heterocyclyl, 4- to 6-membered heterocyclyl, or 5- to 6-membered heterocyclyl, preferably 3- to 8-membered heterocyclyl, 3- to 6-membered heterocyclyl, 3- to 5-membered heterocyclyl, 3- to 4-membered heterocyclyl, 4- to 8-membered heterocyclyl, 4- to 6-membered heterocyclyl, or 5- to 6-membered heterocyclyl, optionally containing 1-4 heteroatoms, 1-3 heteroatoms, or 1-2 heteroatoms, wherein the heteroatom is optionally N, O, or $S(O)_m$ (wherein m is an integer of 0-2), excluding a ring portion of -O-, -O-S-, or -S-S-; the heterocyclyl is preferably 3- to 8-membered heterocyclyl containing 1-4 heteroatoms selected from N, O, or S, and more preferably 4- to 6-membered heterocyclyl containing 1-3 heteroatoms selected from N, O, or S. Non-limiting examples of monocyclic heterocyclyl include oxetanyl, thietanyl, pyrrolidinyl, imidazolidinyl, tetrahydrofuranyl, tetrahydrothienyl, tetrahydropyranyl, dihydroimidazolyl, dihydrofuranyl, dihydropyrazolyl, piperidinyl, piperazinyl, morpholinyl, 1,3-dioxolanyl, 2,2-difluoro-1,3-dioxolanyl, azepinyl, or the like. Non-limiting examples of polycyclic heterocyclyl include spiro heterocyclyl, fused heterocyclyl, and bridged heterocyclyl. The heterocyclyl may be optionally substituted or unsubstituted. When it is substituted, the substituent may attach at any available attachment site, and the substituent is preferably one or more of the following groups independently selected from deuterium, alkyl,

haloalkyl, alkoxy, haloalkoxy, halogen, sulfydryl, hydroxyl, nitryl, amino, or cyano, wherein the alkyl, haloalkyl, alkoxy, or haloalkoxy is optionally further substituted with one or more substituents selected from deuterium, halogen, hydroxyl, amino, sulfydryl, nitryl, cyano, alkyl, haloalkyl, alkoxy, or haloalkoxy.

The term "aryl" refers to a 6- to 14-membered, preferably 6- to 10- membered all-carbon monocyclic or fused polycyclic (i.e., rings that share a pair of adjacent carbon atoms) group having a conjugated electron system, such as phenyl and naphthyl.

The aryl may be fused to a heteroaryl, heterocyclyl, or cycloalkyl ring, wherein the ring attached to the parent structure is an aryl ring, such as aryl-fused cycloalkyl, aryl-fused heterocyclyl, or aryl-fused heteroaryl, preferably $C_{6-10}$ aryl-fused $C_{3-8}$ cycloalkyl, $C_{6-10}$ aryl-fused 3- to 8-membered heterocyclyl, or $C_{6-10}$ aryl-fused 5- to 10-membered heteroaryl, more preferably $C_{6-10}$ aryl-fused 5- to 10-membered heteroaryl, and further preferably benzo-fused 5- to 6-membered heteroaryl, wherein the heterocyclyl is heterocyclyl containing 1-3 heteroatoms selected from nitrogen, oxygen, and sulfur atoms, and the heteroaryl is heteroaryl containing 1-3 heteroatoms selected from nitrogen, oxygen, and sulfur atoms. Its non-limiting examples (wherein the ring attached to the parent structure is an aryl ring) include:

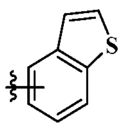, 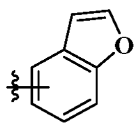, 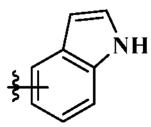, 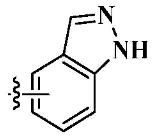, and 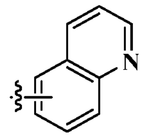;

The aryl, aryl-fused cycloalkyl, aryl-fused heterocyclyl, or aryl-fused heteroaryl may be substituted or unsubstituted. When it is substituted, the substituent is preferably one or more of the following groups independently selected from deuterium, alkyl, haloalkyl, alkoxy, haloalkoxy, halogen, sulfydryl, hydroxyl, nitryl, amino, or cyano, wherein the alkyl, haloalkyl, alkoxy, or haloalkoxy is optionally further substituted with one or more substituents selected from deuterium, halogen, hydroxyl, amino, sulfydryl, nitryl, cyano, alkyl, haloalkyl, alkoxy, or haloalkoxy.

The term "heteroaryl" refers to a heteroaromatic system containing 1 to 4 heteroatoms and 5 to 14 ring atoms, wherein the heteroatoms are selected from oxygen, sulfur, and nitrogen. The heteroaryl is preferably 5- to 10-membered heteroaryl containing 1-4 heteroatoms selected from N, O, or S, more preferably 5- to 6-membered heteroaryl containing 1-3 heteroatoms selected from N, O, or S, or benzo-fused 5- to 6-membered heteroaryl containing 1-3 heteroatoms selected from N, O, or S, such as imidazolyl, furanyl, thienyl, thiazolyl, pyrazolyl, oxazolyl, pyrrolyl, triazolyl, tetrazolyl, pyridinyl, pyrimidinyl, thiadiazole, pyrazinyl, or benzo-fused derivatives thereof, preferably furanyl, triazolyl, thienyl, thiazolyl, imidazolyl, pyrazolyl, pyrrolyl, pyridinyl, pyrimidinyl, thiazolyl, or benzo derivatives thereof; and more preferably furanyl, thienyl, thiazolyl, pyrazolyl, pyrrolyl, pyridinyl, or benzo derivatives thereof. Its non-limiting examples include:

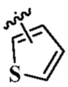, 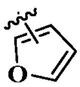, 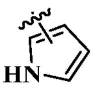, 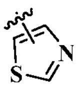, 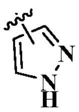, 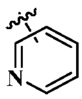, 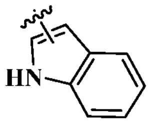, 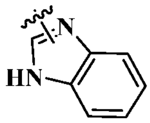, 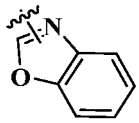,

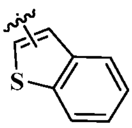, and 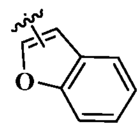.

The heteroaryl may be fused to a heterocyclyl or cycloalkyl ring, wherein the ring attached to the parent structure is a heteroaryl ring, such as heteroaryl-fused cycloalkyl or heteroaryl-fused heterocyclyl, preferably 5- to 10-membered heteroaryl-fused $C_{3-8}$ cycloalkyl or 5- to 10-membered heteroaryl-fused 3- to 8-membered heterocyclyl, wherein the heterocyclyl is heterocyclyl containing 1-4 heteroatoms selected from nitrogen, oxygen, and sulfur atoms, and the heteroaryl is heteroaryl containing 1-4 heteroatoms selected from nitrogen, oxygen, and sulfur atoms.

The heteroaryl, heteroaryl-fused cycloalkyl, or heteroaryl-fused heterocyclyl may be optionally substituted or unsubstituted. When it is substituted, the substituent is preferably one or more of the following groups independently selected from deuterium, alkyl, haloalkyl, alkoxy, haloalkoxy, halogen, sulfydryl, hydroxyl, nitryl, amino, or cyano, wherein the alkyl, haloalkyl, alkoxy, or haloalkoxy is optionally further substituted with one or more substituents selected from deuterium, halogen, hydroxyl, amino, sulfydryl, nitryl, cyano, alkyl, haloalkyl, alkoxy, or haloalkoxy.

The term "alkoxy" refers to -O-(alkyl) and -O-(unsubstituted cycloalkyl), wherein the alkyl and the cycloalkyl are as defined above, preferably alkoxy containing 1 to 8 carbon atoms, more preferably alkoxy of 1 to 6 carbon atoms, and most preferably alkoxy of 1 to 3 carbon atoms. Non-limiting examples of alkoxy include: methoxy, ethoxy, propoxy, butoxy,

cyclopropyloxy, cyclobutoxy, cyclopentyloxy, and cyclohexyloxy. The alkoxy may be optionally substituted or unsubstituted. When it is substituted, the substituent is preferably one or more of the following groups independently selected from deuterium, halogen, hydroxyl, amino, sulfydryl, nitryl, cyano, alkyl, haloalkyl, alkoxy, or haloalkoxy.

The term "haloalkyl" refers to alkyl substituted with one or more halogens, wherein the alkyl is as defined above. Non-limiting examples of halomethyl include: fluoromethyl, chloromethyl, bromomethyl, iodomethyl, difluoromethyl, chlorofluoromethyl, dichloromethyl, bromofluoromethyl, trifluoromethyl, chlorodifluoromethyl, dichlorofluoromethyl, trichloromethyl, bromodifluoromethyl, bromochlorofluoromethyl, dibromofluoromethyl, and the like, preferably fluoromethyl, difluoromethyl, and trifluoromethyl. Non-limiting examples of haloethyl include: 2-fluoroethyl, 2-chloroethyl, 2-bromoethyl, 2,2-difluoroethyl, 2-chloro-2-fluoroethyl, 2,2-dichloroethyl, 2-bromo-2-fluoroethyl, 2,2,2-trifluoroethyl, 2-chloro-2,2-difluoroethyl, 2,2-dichloro-2-fluoroethyl, 2,2,2-trichloroethyl, 2-bromo-2,2-difluoroethyl, 2-bromo-2-chloro-2-fluoroethyl, 2-bromo-2,2-dichloroethyl, 1,1,2,2-tetrafluoroethyl, pentafluoroethyl, 1-chloro-1,2,2,2-tetrafluoroethyl, 2-chloro-1,1,2,2-tetrafluoroethyl, 1,2-dichloro-1,2,2-trifluoroethyl, 2-bromo-1,1,2,2-tetrafluoroethyl, and the like, preferably 2-fluoroethyl, 2-chloroethyl, 2-bromoethyl, and 2,2-difluoroethyl. The haloalkyl may be optionally substituted or unsubstituted. When it is substituted, the substituent is preferably one or more of the following groups independently selected from deuterium, halogen, hydroxyl, amino, sulfydryl, nitryl, cyano, alkyl, haloalkyl, alkoxy, or haloalkoxy.

The term "haloalkoxy" refers to alkoxy substituted with one or more halogens, wherein the alkoxy is as defined above. Non-limiting examples of halomethoxy include: fluoromethoxy, chloromethoxy, bromomethoxy, iodomethoxy, difluoromethoxy, chlorofluoromethoxy, dichloromethoxy, bromofluoromethoxy, trifluoromethoxy, chlorodifluoromethoxy, dichlorofluoromethoxy, trichloromethoxy, bromodifluoromethoxy, bromochlorofluoromethoxy, dibromofluoromethoxy, and the like, preferably fluoromethoxy, difluoromethoxy, and trifluoromethoxy. Non-limiting examples of haloethoxy include: 2-fluoroethoxy, 2-chloroethoxy, 2-bromoethoxy, 2,2-difluoroethoxy, 2-chloro-2-fluoroethoxy, 2,2-dichloroethoxy, 2-bromo-2-fluoroethoxy, 2,2,2-trifluoroethoxy, 2-chloro-2,2-difluoroethoxy, 2,2-dichloro-2-fluoroethoxy, 2,2,2-trichloroethoxy, 2-bromo-2,2-difluoroethoxy, 2-bromo-2-chloro-2-fluoroethoxy, 2-bromo-2,2-dichloroethoxy, 1,1,2,2-tetrafluoroethoxy, pentafluoroethoxy, 1-chloro-1,2,2,2-tetrafluoroethoxy, 2-chloro-1,1,2,2-tetrafluoroethoxy, 1,2-dichloro-1,2,2-trifluoroethoxy, 2-bromo-1,1,2,2-tetrafluoroethoxy, and the like, preferably 2-fluoroethoxy, 2-chloroethoxy, 2-bromoethoxy, and 2,2-difluoroethoxy. The haloalkoxy may be optionally substituted or unsubstituted. When it is substituted, the substituent is preferably one or more of the following groups independently selected from deuterium, halogen, hydroxyl, amino, sulfydryl, nitryl, cyano, alkyl, haloalkyl, alkoxy, or haloalkoxy. The term "alkenyl" refers to alkyl as defined above consisting of at least two carbon atoms and at least one carbon-carbon double bond, such as vinyl, 1-propenyl, 2-propenyl, 1-, 2- or 3-butenyl, and the like. The alkenyl may be substituted or unsubstituted. When it is substituted, the substituent is preferably one or more of the following groups independently selected from deuterium, alkyl, haloalkyl, alkoxy, haloalkoxy, halogen, sulfydryl, hydroxyl, nitryl, amino, or cyano, wherein the alkyl, haloalkyl, alkoxy, or haloalkoxy is optionally further substituted with one or more substituents selected from deuterium, halogen, hydroxyl, amino, sulfydryl, nitryl, cyano, alkyl, haloalkyl, alkoxy, or haloalkoxy.

The term "alkynyl" refers to alkyl containing at least one carbon-carbon triple bond in the molecule, wherein the alkyl is as defined above and has 2 to 6 (e.g., 2, 3, 4, 5, or 6) carbon atoms (i.e., C2-6 alkynyl). Non-limiting examples include: ethynyl, propynyl, butynyl, pentynyl, hexynyl, and the like. The alkynyl may be substituted or unsubstituted. When it is substituted, the substituent is preferably one or more of the following groups independently selected from deuterium, alkyl, haloalkyl, alkoxy, haloalkoxy, halogen, sulfydryl, hydroxyl, nitryl, amino, or cyano, wherein the alkyl, haloalkyl, alkoxy, or haloalkoxy is optionally further substituted with one or more substituents selected from deuterium, halogen, hydroxyl, amino, sulfydryl, nitryl, cyano, alkyl, haloalkyl, alkoxy, or haloalkoxy.

"Hydroxyl" refers to the -OH group.

"Halogen" refers to fluorine, chlorine, bromine, or iodine.

"Amino" refers to $-NH_2$.

"Cyano" refers to -CN.

"Nitryl" refers to $-NO_2$.

"Sulfydryl" refers to -SH.

The terms "comprise", "include", "have", "contain", or "involve" and other variations thereof herein are inclusive or open-ended and do not exclude additional unrecited elements or method steps. It should be understood by those skilled in the art that terms described above such as "comprise" encompass the meaning of "consist of".

The term "one or more" or similar expressions "at least one" may indicate, for example, 1, 2, 3, 4, 5, 6, 7, 8, 9, 10, or more.

When the lower and upper limits of a range of numerical values are disclosed, any numerical value falling within the range and any included range are specifically disclosed. In particular, each range of values disclosed herein is to be understood as indicating each numerical value and range encompassed within a relatively broad range.

As used herein, "Z" and "-Z-" both refer to the same particular group, which can be used interchangeably.

The expression "m-n" as used herein refers to the range of m to n as well as to sub-ranges consisting of point values therein and the point values. For example, the expression "C2-C8" or "C2-8" encompasses a range of 2-8 carbon

atoms and should be understood as also encompassing any sub-range and each point value therein, such as C2-C5, C3-C4, C2-C6, C3-C6, C4-C6, C4-C7, C4-C8, etc., and C2, C3, C4, C5, C6, C7, C8, etc. For example, the expression "C3-C10" or "C3-10" should also be understood in a similar manner, for example, it can encompass any sub-range and point value therein, such as C3-C9, C6-C9, C6-C8, C6-C7, C7-C10, C7-C9, C7-C8, C8-C9, etc., and C3, C4, C5, C6, C7, C8, C9, C10, etc. For another example, the expression "C1-C6" or "C1-6" encompasses a range of 1-6 carbon atoms and should be understood as also encompassing any sub-range and each point value therein, such as C2-C5, C3-C4, C1-C2, C1-C3, C1-C4, C1-C5, C1-C6, etc., and C1, C2, C3, C4, C5, C6, etc. For another example, the expression "three- to ten-membered" should be understood as encompassing any sub-range and each point value therein, such as three- to five-membered, three- to six-membered, three- to seven-membered, three- to eight-membered, four- to five-membered, four- to six-membered, four- to seven-membered, four- to eight-membered, five- to seven-membered, five- to eight-membered, six- to seven-membered, six- to eight-membered, nine- to ten-membered, etc., and three-membered, four-membered, five-membered, six-membered, seven-membered, eight-membered, nine-membered, ten-membered, etc. Other similar expressions herein should also be understood in a similar manner.

The expressions "X is selected from A, B, or C", "X is selected from A, B, and C", "X is A, B, or C", "X is A, B, and C", and the like as used herein all carry the same meaning, i.e., X may be any one or more of A, B, and C.

The expression "-(CY1Y2)n-" as used herein means that each Y1 or Y2 attached to C may be the same or different, i.e., each Y1 may be a different group, each Y2 may be a different group, each Y1 may also be the same group, and each Y2 may also be the same group.

The term "optional" or "optionally" refers to that the subsequently described event or circumstance may occur or may not occur. The description includes instances where the event or circumstance occurs and instances where the event or circumstance does not occur. For example, "cycloalkyl optionally substituted with alkyl" means that alkyl may, but does not necessarily, exist. The description includes instances where the cycloalkyl is substituted with alkyl and instances where the cycloalkyl is not substituted with alkyl. The terms "substitution" and "substituted" mean that one or more (e.g., one, two, three, or four) hydrogens on a specified atom are replaced by a choice from the designated groups, with the proviso that the normal valency of the specified atom in the present case is not exceeded and that the replacement results in a stable compound. A combination of substituents and/or variables is permissible only if the combination results in a stable compound. When it is stated that a certain substituent is absent, it should be understood that the substituent may be one or more hydrogen atoms, provided that the structure enables the compound to reach a stable state. When it is stated that each carbon atom in a group may optionally be replaced by a heteroatom, the proviso is that the normal valency of all atoms in the group in the present case is not exceeded, and that a stable compound is formed.

If a substituent is described as "optionally substituted", the substituent may be unsubstituted or may be substituted. If an atom or group is described as optionally substituted with one or more substituents in the list of substituents, one or more hydrogens on the atom or group can be replaced by an independently selected and optional substituent. When the substituent is oxo (namely =O), it means that two hydrogen atoms are replaced. Unless otherwise specified, as used herein, the attachment point of a substituent may be from any suitable position of the substituted group.

When a bond of a substituent is shown as passing through a bond connecting two atoms in a ring, the substituent may be bonded to any one of the ring-forming atoms in the substitutable ring.

When any variable (e.g., R), as well as labeled variables (e.g., R1, R2, R3, R4, R5, R6, R7, etc.), occur more than once in a composition or structure of a compound, the variables are independently defined in each case at each occurrence. For example, if a group is substituted with 0, 1, 2, 3, or 4 R substituents, the group may optionally be substituted with up to four R substituents, and the options for each R substituent in each case are independent of each other.

The term "pharmaceutically acceptable" substance refers to those substances which are, within the scope of sound medical judgment, suitable for use in contact with the tissues of patients without undue toxicity, irritation, allergic response, and other problems, commensurate with a reasonable benefit to risk ratio, and effective for their intended use. The term "pharmaceutically acceptable salt" refers to salts of the compounds of the present invention, which are safe and effective for use in the body of a mammal and possess the requisite biological activities.

The term "pharmaceutical composition" refers to a mixture containing one or more of the compounds described herein or a physiologically/pharmaceutically acceptable salt or prodrug thereof, and other chemical components, such as physiologically/pharmaceutically acceptable carriers or excipients. The pharmaceutical composition is intended to promote the administration to an organism, so as to facilitate the absorption of the active ingredient, thereby exerting its biological activity.

The term "pharmaceutically acceptable carrier" refers to those substances that do not have a significant irritating effect on organisms and do not impair the biological activity and properties of the active compound. The term "pharmaceutically acceptable carrier" includes, but is not limited to, a glidant, a sweetener, a diluent, a preservative, a dye/colorant, a flavoring agent, a surfactant, a wetting agent, a dispersant, a disintegrant, a stabilizer, a solvent, or an emulsifier.

The terms "administration" or "administering" and the like refer to methods that enable a compound or composition to be delivered to a desired site of biological action. These methods include, but are not limited to, oral

administration or parenteral administration (including intraventricular, intravenous, subcutaneous, intraperitoneal, intramuscular, and intravascular injection or infusion), topical administration, rectal administration, and the like, especially injection or oral administration.

As used herein, the term "treat", "treating" or "treatment" includes alleviating, relieving, or ameliorating a disease or symptom; preventing other symptoms; ameliorating or preventing metabolic factors underlying a symptom; inhibiting a disease or symptom, such as arresting the development of a disease or symptom; relieving a disease or symptom; promoting the alleviation of a disease or symptom; or halting the signs of a disease or symptom, and extends to include prevention. The term "treat", "treating" or "treatment" also includes achieving a therapeutic benefit and/or a prophylactic benefit. The therapeutic benefit refers to eradication or amelioration of a condition being treated. In addition, the therapeutic benefit is achieved by eradicating or ameliorating one or more physiological signs associated with an underlying disease, such that amelioration of the underlying disease in the patient can be observed, although the patient may still be afflicted with the underlying disease. The prophylactic benefit refers to the use of a composition by a patient to prevent the risk of a certain disease or the administration of a composition by a patient when the patient develops one or more physiological conditions of a disease, although the disease has not yet been diagnosed.

The term "active ingredient", "therapeutic agent", "active substance" or "active agent" refers to a chemical entity that is effective in treating or preventing a target disorder, disease, or condition. The term "neuropsychiatric disease" refers to a generic term for neurological diseases and psychiatric diseases, including neurological diseases and/or psychiatric diseases.

For a drug, drug unit or active ingredient, the term "effective amount", "therapeutically effective amount" or "prophylactically effective amount" refers to an amount of a drug or agent that is sufficient to provide the desired effect with acceptable side effects. The determination of the effective amount varies from person to person. It depends on the age and general condition of an individual, as well as the specific active substance used. The appropriate effective amount in a case may be determined by those skilled in the art in the light of routine tests.

As used herein, the term "individual" includes a human or non-human animal. An exemplary human individual includes a human individual (referred to as a patient) with a disease (e.g., a disease described herein) or a normal individual. As used herein, the term "non-human animal" includes all vertebrates, such as non-mammals (e.g., birds, amphibians, and reptiles) and mammals, such as non-human primates, livestock and/or domesticated animals (e.g., sheep, dogs, cats, cows, pigs, etc.).

The following detailed description of the present invention is intended to illustrate non-limiting embodiments and to enable others skilled in the art to more fully understand the technical solutions of the present invention, its principles, and its practical applications, so that others skilled in the art may modify and implement the present invention in various forms to allow it to be optimally adapted to the requirements of particular use.

Beneficial Effects

The compounds of the present invention have novel structures, can act on 5-$HT_{1A}$ receptors, dopamine $D_2$ receptors, and/or dopamine $D_3$ receptors, and show certain agonistic activity. Some of the compounds show an agonistic effect on at least two of the 5-$HT_{1A}$, dopamine $D_2$, and dopamine $D_3$ receptors, and particularly, most of the compounds show triple agonistic activity for the 5-$HT_{1A}$, dopamine $D_2$, and dopamine $D_3$ receptors, suggesting that the compounds have significant therapeutic effect on central nervous system disorders such as Parkinson's and can be used in the preparation of medicaments for treating central nervous system disorders.

## BRIEF DESCRIPTION OF THE DRAWING

FIG. 1: Effects on tacrine-induced jaw tremor behavior in rats by Example 28 at different concentrations.
FIG. 2: Effects on tacrine-induced jaw tremor behavior in rats by Example 32 at different concentrations.
FIG. 3: Effects on tacrine-induced jaw tremor behavior in rats by Example 33 at different concentrations.

## DETAILED DESCRIPTION

Embodiments of the present invention will be described in detail below with reference to examples, but those skilled in the art will appreciate that the following examples are only illustrative of the present invention and should not be construed as limiting the scope of the present invention. Experimental procedures without specified conditions in the examples were conducted according to conventional conditions or conditions recommended by the manufacturers. Reagents or instruments without specified manufacturers used herein are conventional products that are commercially available. The proportions or percentages used herein are calculated by weight, unless otherwise specified.

## Examples

The compound structures of the present invention were determined by nuclear magnetic resonance (NMR) and/or liquid chromatography-mass spectrometry (LC-MS).

The NMR chemical shifts (δ) are given in parts per million (ppm). The NMR determination was conducted by using an AVANCE III600 nuclear magnetic resonance apparatus, with dimethyl sulfoxide (DMSO), deuterated dimethyl sulfoxide (DMSO-$d_6$), deuterated methanol ($CD_3OD$), and deuterated chloroform ($CDCl_3$) as determination solvents, and tetramethylsilane (TMS) as an internal standard.

The LC-MS determination was conducted by using a Japan Shimadzu LCMS2020 mass spectrometer. The HPLC determination was performed by using a Japan Shimadzu LC20A liquid chromatograph.

Yantai Jiangyou silica gel plates were adopted as thin layer chromatography silica gel plates. The specification adopted in the TLC was 0.2 mm ± 0.03 mm, and the specification adopted in the thin layer chromatography for product separation and purification was 0.4 mm-0.5 mm.

### Intermediate 1a

### (S)-N6-(Piperidin-4-ylmethyl)-N6-propyl-4,5,6,7-tetrahydrobenzo[d]thiazole-2,6-diamine 1a

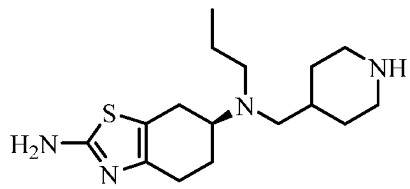

1a

Synthesis scheme:

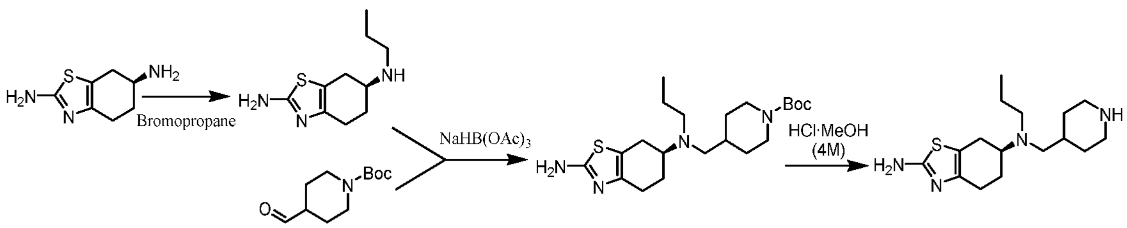


Step A: Synthesis of (S)-N6-propyl-4,5,6,7-tetrahydrobenzo[d]thiazole-2,6-diamine

(S)-4,5,6,7-Tetrahydrobenzo[d]thiazole-2,6-diamine (0.24 mol, 1 eq) was taken and dissolved in 320 mL of NMP, and bromopropane (0.96 mol, 4 eq) was added. The mixture was then stirred at room temperature for 18 h. After the reaction was completed, the mixture was subjected to suction filtration. The filter cake was washed with an appropriate amount of isopropanol to obtain a white solid, which was then placed in a 70 °C air blast drying oven for drying. The dried sample described above was dissolved in an appropriate amount of water (30 mL), and the pH was adjusted to 10 by adding a 20% aqueous NaOH solution. The mixture was extracted with dichloromethane (400 mL × 3), and the organic layers were combined, dried, and concentrated to obtain (S)-N6-propyl-4,5,6,7-tetrahydrobenzo[d]thiazole-2,6-diamine. ESI-MS$[M+H]^+$: m/z 212.1.

Step B: Synthesis of *tert*-butyl (S)-4-(((2-amino-4,5,6,7-tetrahydrobenzo[d]thiazol-6-yl)(propyl)amino)methyl)piperidine-1-carboxylate

(S)-N6-Propyl-4,5,6,7-tetrahydrobenzo[d]thiazole-2,6-diamine (94.6 mmol, 1 eq) and tert-butyl 4-formylpiperidine-1-carboxylate (113.6 mmol, 1.2 eq) were taken and dissolved in 1,2-dichloroethane (150 mL). Then, 3 drops of acetic acid were added at - 20 °C, and the mixture was stirred for 30 min. Sodium triacetoxyborohydride (142.0 mmol, 1.5 eq) was

then added, and the mixture was stirred at -20 °C for 8 h. The reaction was stopped when the starting materials were substantially depleted or by-products began to be produced. Ethyl acetate (100 mL) was then added to dilute the reaction solution. The reaction was quenched by adding 10% hydrochloric acid (150 mL) at -20 °C, and then a saturated $NaHCO_3$ solution (300 mL) was added. Liquid separation was performed. The aqueous layer was extracted with dichloromethane (300 mL × 2), and the organic layers were combined, washed with saturated brine, dried over anhydrous sodium sulfate, and then concentrated under reduced pressure. The crude product was purified by column chromatography to obtain *tert*-butyl (S)-4-(((2-amino-4,5,6,7-tetrahydrobenzo[d]thiazol-6-yl)(propyl)amino)methyl)piperidine-1-carboxylate (15.2 g, 39.3%). ESI-MS[M+H]$^+$: m/z 409.1.

Step C: Synthesis of (S)-$N^6$-(piperidin-4-ylmethyl)-$N^6$-propyl-4,5,6,7-tetrahydrobenzo [d]thiazole-2,6-diamine

*tert*-Butyl (S)-4-(((2-amino-4,5,6,7-tetrahydrobenzo[d]thiazol-6-yl)(propyl)amino)methyl) piperidine-1-carboxylate (37.3 mmol, 1 eq) was taken and dissolved in 20 mL of methanol, and 14 mL of a 4 M solution of hydrogen chloride in methanol (1.5 eq) was added. The mixture was stirred at room temperature for 12 h. After the starting materials were depleted, the solvent was removed by concentration under reduced pressure. After 20 mL of water was added to disperse the sample, the aqueous phase was washed with 50 mL of ethyl acetate, and then the pH was adjusted to 10 with a 10% NaOH solution. The aqueous phase was extracted with dichloromethane (100 mL × 3), and the organic phases were combined. The organic layer was washed with saturated brine, dried over anhydrous sodium sulfate, and then concentrated under reduced pressure to obtain intermediate **1a** (8.5 g, 73.9%). ESI-MS[M+H]$^+$: m/z 309.1.

**Intermediate 1b**

**2-(4-(5-Fluoropyridin-2-yl)-1,9-dioxaspiro[5.5]undecan-4-yl)acetaldehyde 1b**

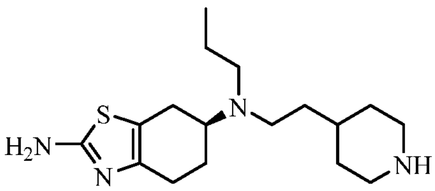

1b

Synthesis scheme:

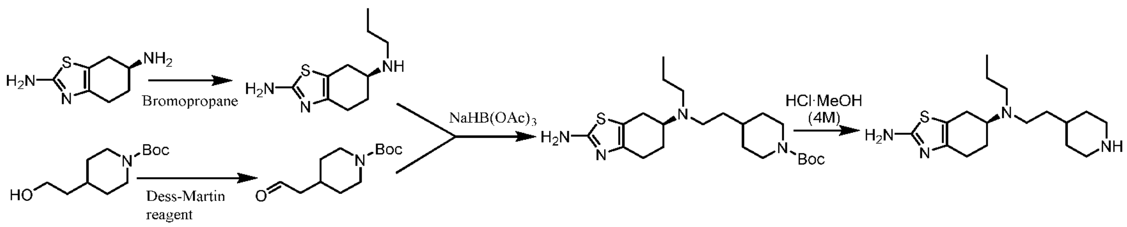


The synthesis method for intermediate 1a was employed, except that tert-butyl 4-formylpiperidine-1-carboxylate used in step B was replaced by tert-butyl 4-(2-oxyethyl)piperidine-1-carboxylate, and intermediate **1b** was prepared by steps A-C. ESI-MS[M+H]$^+$: m/z 323.1.

The intermediate *tert*-butyl 4-(2-oxyethyl)piperidine-1-carboxylate was prepared by the following procedures: *tert*-Butyl 4-(2-hydroxyethyl)piperidine-1-carboxylate (21.8 mmol, 1 eq) was dissolved in 100 mL of dichloromethane, and Dess-Martin reagent (21.8 mmol, 1 eq) was added. The mixture was stirred at room temperature for 1.5 h. After the reaction was completed, a 10% aqueous sodium thiosulfate solution (100 mL) and a saturated aqueous sodium bicarbonate solution (100 mL) were added. The mixture was stirred at room temperature for 1 h. Liquid separation was performed. The aqueous layer was extracted with dichloromethane (100 mL × 2), and the organic layers were combined, washed with a saturated aqueous sodium bicarbonate solution, dried, and concentrated to obtain *tert*-butyl 4-(2-oxyethyl)piperidine-1-carboxylate. ESI-MS[M+H]$^+$: m/z 228.1.

## Example 1

**(S)-$N^6$-(1-(2,3-Dichlorophenyl)piperidin-4-yl)-4,5,6,7-tetrahydrobenzo[d]thiazole-2,6-diamine and dihydrochloride thereof**

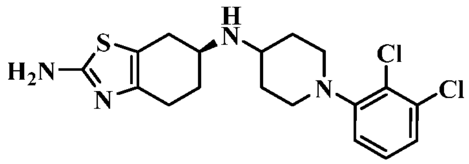

Synthesis scheme of (S)-$N^6$-(1-(2,3-dichlorophenyl)piperidin-4-yl)-4,5,6,7-tetrahydrobenzo [d]thiazole-2,6-diamine:

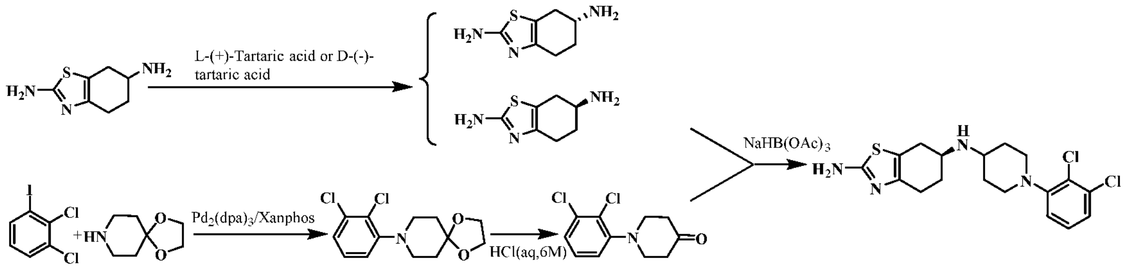


Step A: Synthesis of (S)-4,5,6,7-tetrahydrobenzo[d]thiazole-2,6-diamine

The starting materials 4,5,6,7-tetrahydrobenzo[d]thiazole-2,6-diamine (45.0 g, 0.266 mol) and L-(+)-tartaric acid (40.0 g, 0.266 mol) were added to a 1000-mL reaction flask, and 450 mL of water was added. The mixture was heated and stirred at 80 °C for 1 h, and then cooled to room temperature and stirred for 2 h. The solid was completely precipitated, and the mixture was subjected to suction filtration and drying to obtain an off-white solid (57.0 g). The dried solid described above was taken and recrystallized three times with 8 times the amount of water. The recrystallized product was taken and dispersed in 80 mL of water, and a 20% sodium hydroxide solution was added to adjust the pH to 12. The mixture was stirred at 0-5 °C for 2 h, and subjected to suction filtration and drying to obtain (S)-4,5,6,7-tetrahydrobenzo[d]thiazole-2,6-diamine (15.8 g, yield 62.0%).

ESI-MS$[M+H]^+$: m/z 170.0. $[\alpha]_D^{20}$ -94.5° (C=1, MeOH).

The mother liquor was recovered, and using D-(-)-tartaric acid as a resolving agent, (R)-4,5,6,7-tetrahydrobenzo [d]thiazole-2,6-diamine was obtained by the same method (ESI-MS$[M+H]^+$: m/z 170.0. $[\alpha]_D^{20}$ +99.4° (C = 1, MeOH).).

Step B: Synthesis of 1-(2,3-dichlorophenyl)piperidin-4-one

1,2-Dichloro-3-iodobenzene (22.0 mmol, 1 eq), 4-piperidone ethylene ketal (22.0 mmol, 1 eq), $Pd_2(dba)_3$ (0.6 mmol, 0.025 eq), and Xantphos (2.2 mmol, 0.1 eq) were taken and dissolved in toluene (60 mL), and sodium tert-butoxide (33.0 mmol, 1.5 eq) was added. After being purged with nitrogen, the mixture was heated to 80-110 °C and refluxed for 15 h. After the reaction was completed, suction filtration was performed using celite. The filtrate was subjected to rotary evaporation under reduced pressure to remove the solvent to obtain a crude product of the intermediate (5.1 g) (ESI-MS $[M+H]^+$: m/z 288.1. $^1H$ NMR (400 MHz, DMSO) $\delta$ 7.29 (d, J = 2.0 Hz, 1H), 7.28 (s, 1H), 7.16 (dd, J = 5.7, 3.9 Hz, 1H), 3.92 (s, 4H), 3.05-3.00 (m, 4H), 1.78 (t, J = 5.6 Hz, 4H).).

The crude product of the intermediate was dissolved in acetone (60 mL), and a 6 N hydrochloric acid solution (70 mL) was added. The mixture was stirred at room temperature overnight. After hydrolysis was completed, water was added, and organic impurities were removed by extraction with ethyl acetate. The aqueous layer was adjusted to pH = 10 with $NaHCO_3$, and extracted with ethyl acetate (3 × 100 mL). Liquid separation was performed, and the organic layer was concentrated. The crude product was subjected to silica gel column chromatography to obtain 1-(2,3-dichlorophenyl) piperidin-4-one (2.23 g, yield 51.6%).

ESI-MS$[M+H]^+$: m/z 244.0.

Step C: Synthesis of (S)-*N*6-(1-(2,3-dichlorophenyl)piperidin-4-yl)-4,5,6,7-tetrahydrobenzo [d]thiazole-2,6-diamine

(S)-4,5,6,7-Tetrahydrobenzo[d]thiazole-2,6-diamine (3.7 mmol, 1 eq) and 1-(2,3-dichlorophenyl)piperidin-4-one (4.1 mmol, 1.1 eq) were taken and dissolved in 1,2-dichloroethane (20 mL), and 1 drop of acetic acid was added under an ice bath. The mixture was stirred for 30 min. Sodium triacetoxyborohydride (5.5 mmol, 1.5 eq) was then added, and the mixture was stirred at room temperature for 8 h. The reaction was stopped when the starting materials were substantially depleted or by-products began to be produced. Ethyl acetate (100 mL) was then added to dilute the reaction solution. The reaction was quenched by adding 10% hydrochloric acid (10 mL) under an ice bath, and then a saturated $NaHCO_3$ solution (20 mL) was added. After liquid separation was performed, the aqueous phase was extracted with dichloromethane, and the organic layers were combined, washed with saturated brine, dried over anhydrous sodium sulfate, and then concentrated under reduced pressure. The crude product was purified by column chromatography to obtain the target product.

ESI-MS[M+H]$^+$: m/z 397.0.

Preparation of hydrochloride: Synthesis of (S)-N6-(1-(2,3-dichlorophenyl)piperidin-4-yl)-4,5,6,7-tetrahydrobenzo[d] thiazole-2,6-diamine dihydrochloride

(S)-N6-(1-(2,3-Dichlorophenyl)piperidin-4-yl)-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 2-5 h, and subjected to suction filtration to obtain the dihydrochloride of the compound.

ESI-MS[M+H]$^+$: m/z 397.0.

$^1$H NMR (400 MHz, DMSO) δ 9.63 (s, 1H), 9.44 (s, 1H), 9.17 (s, 2H), 7.36 (d, J = 2.0 Hz, 1H), 7.36 - 7.34 (m, 1H), 7.22 - 7.16 (m, 1H), 3.70 (s, 1H), 3.50 - 3.44 (m, 2H), 3.41 - 3.31 (m, 2H) 3.15 - 3.04 (m, 1H), 2.87 - 2.78 (m, 2H), 2.78 - 2.72 (m, 1H), 2.71 - 2.67 (m, 1H), 2.66 - 2.59 (m, 1H), 2.37 - 2.16 (m, 3H), 2.05 - 1.97 (m, 1H), 1.95 - 1.74 (m, 2H).

**Example 2**

**(S)-N6-(1-(2,3-Dichlorophenyl)piperidin-4-yl)-N6-propyl-4,5,6,7-tetrahydrobenzo[d]thiazole-2,6-diamine and hydrochloride thereof**

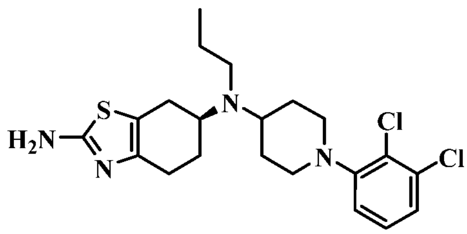

Synthesis scheme of (S)-N6-(1-(2,3-dichlorophenyl)piperidin-4-yl)-N6-propyl-4,5,6,7-tetrahydrobenzo[d]thiazole-2,6-diamine:

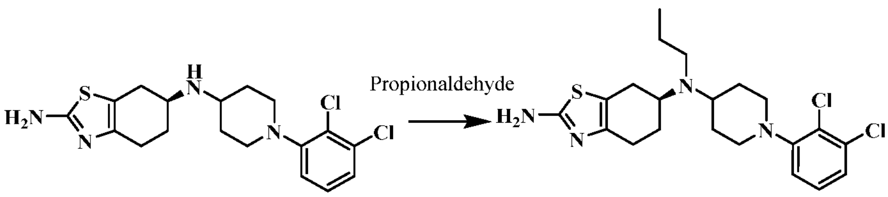


The compound (S)-N6-(1-(2,3-dichlorophenyl)piperidin-4-yl)-4,5,6,7-tetrahydrobenzo [d]thiazole-2,6-diamine (1.26 mmol, 1 eq) and propionaldehyde (1.26 mmol, 1 eq) were taken and dissolved in 1,2-dichloroethane (10 mL), and 1 drop of acetic acid was added under an ice bath. The mixture was stirred for 30 min. Sodium triacetoxyborohydride (1.9 mmol, 1.5 eq) was then added, and the mixture was stirred at room temperature for 6 h. The reaction was stopped when the

starting materials were substantially depleted or by-products began to be produced. Ethyl acetate (20 mL) was then added to dilute the reaction solution. The reaction was quenched by adding 10% hydrochloric acid (5 mL) under an ice bath, and then a saturated $NaHCO_3$ solution (10 mL) was added. After liquid separation was performed, the aqueous phase was extracted with dichloromethane, and the organic layers were combined, washed with saturated brine, dried over anhydrous sodium sulfate, and then concentrated under reduced pressure. The crude product was purified by column chromatography to obtain the target product.

ESI-MS$[M+H]^+$: m/z 439.0.

Preparation of hydrochloride: Synthesis of (S)-N$^6$-(1-(2,3-dichlorophenyl)piperidin-4-yl)-N$^6$-propyl-4,5,6,7-tetrahydrobenzo[d]thiazole-2,6-diamine hydrochloride

(S)-N$^6$-(1-(2,3-Dichlorophenyl)piperidin-4-yl)-N$^6$-propyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 2-5 h, and subjected to suction filtration to obtain the hydrochloride of the compound.

ESI-MS$[M+H]^+$: m/z 439.0.

$^1$H NMR (400 MHz, DMSO) δ10.66 - 10.55 (m, 1H), 9.31 (s, 2H), 7.37 (d, J = 2.3 Hz, 1H), 7.36 - 7.35 (m, 1H), 7.23 - 7.13 (m, 1H), 3.83 (s, 1H), 3.26 - 3.21 (m, 4H), 3.19 - 2.89 (m, 1H), 2.84 - 2.74 (m, 2H), 2.73 - 2.60 (m, 2H), 2.52 - 2.39 (m, 1H), 2.39 - 2.26 (m, 1H), 2.27 - 2.16 (m, 1H), 2.12 (s, 1H), 2.09 - 2.04 (m, 1H), 2.05 - 1.98 (m, 2H), 1.93 - 1.83 (m, 1H), 1.83 - 1.77 (m, 2H), 0.98 (t, J = 7.4 Hz, 3H).

**Example 3**

**(S)-N$^6$-(1-(Benzo[b]thiophen-4-yl)piperidin-4-yl)-N$^6$-propyl-4,5,6,7-tetrahydrobenzo[d]thiazole-2,6-diamine and hydrochloride thereof**

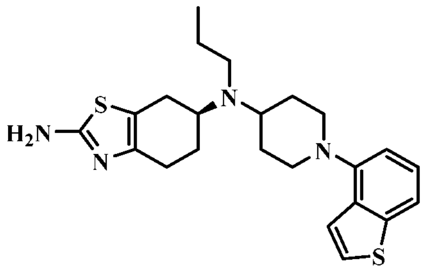

Synthesis scheme of (S)-N$^6$-(1-(benzo[b]thiophen-4-yl)piperidin-4-yl)-N$^6$-propyl-4,5,6,7-tetrahydrobenzo[d]thiazole-2,6-diamine:

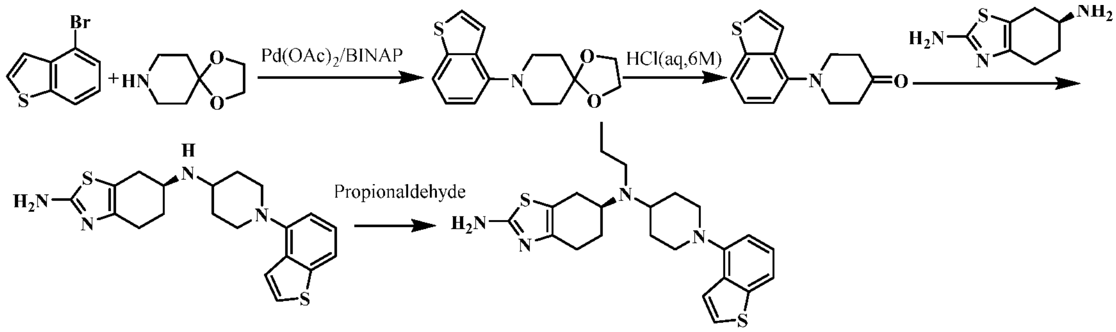


The synthesis method in Example 1 was employed, except that 1,2-dichloro-3-iodobenzene used in step B was

replaced by 4-bromobenzo[b]thiophene, and the compound (S)-$N^6$-(1-(benzo[b]thiophen-4-yl)piperidin-4-yl)-4,5,6,7-tetrahydrobenzo [d]thiazole-2,6-diamine was prepared by steps A-C. Then, the synthesis method in Example 2 was employed, except that the starting material (S)-$N^6$-(1-(2,3-dichlorophenyl)piperidin-4-yl)-4,5,6,7-tetrahydrobenzo[d]thiazole-2,6-diamine was replaced by (S)-$N^6$-(1-(benzo[b]thiophen-4-yl)piperidin-4-yl)-4,5,6,7-tetrahydrobenzo [d]thiazole-2,6-diamine to prepare the target product.

ESI-MS$[M+H]^+$: m/z 427.0.

Preparation of hydrochloride: Synthesis of (S)-$N^6$-(1-(benzo[b]thiophen-4-yl)piperidin-4-yl)-$N^6$ -propyl-4,5,6,7-tetrahydrobenzo[d]thiazole-2,6-diamine hydrochloride

(S)-$N^6$-(1-(Benzo[b]thiophen-4-yl)piperidin-4-yl)-$N^6$-propyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 2-5 h, and subjected to suction filtration to obtain the hydrochloride of the compound.

ESI-MS$[M+H]^+$: m/z 427.0.

$^1$H NMR (600 MHz, DMSO) δ 10.57 (d, J = 18.2 Hz, 1H), 9.35 (s, 2H), 7.75 (d, J = 5.5 Hz, 1H), 7.67 (d, J = 8.0 Hz, 1H), 7.51 - 7.45 (m, 1H), 7.31 (t, J = 7.8 Hz, 1H), 6.95 (d, J = 7.6 Hz, 1H), 3.60 - 3.54 (m, 3H), 3.34 - 3.09 (m, 3H), 3.08 - 2.91 (m, 1H), 2.91 - 2.79 (m, 2H), 2.78 - 2.58 (m, 2H), 2.48 - 2.42 (m, 1H), 2.34 - 2.28 (m, 2H), 2.22 - 2.18 (m, 3H), 2.14 - 2.03 (m, 1H), 1.89 - 1.80 (m, 2H), 0.97 (q, J = 7.1 Hz, 3H).

**Example 4**

**(S)-$N^6$-((1-(2,3-Dichlorophenyl)piperidin-4-yl)methyl)-4,5,6,7-tetrahydrobenzo[d]thiazole-2,6-diamine and dihydrochloride thereof**

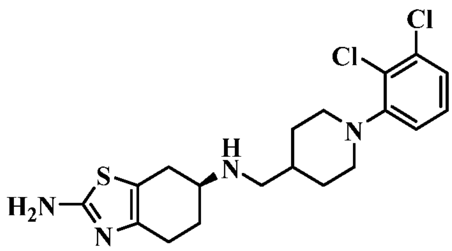

Synthesis scheme of (S)-$N^6$-((1-(2,3-dichlorophenyl)piperidin-4-yl)methyl)-4,5,6,7-tetrahydrobenzo[d]thiazole-2,6-diamine:

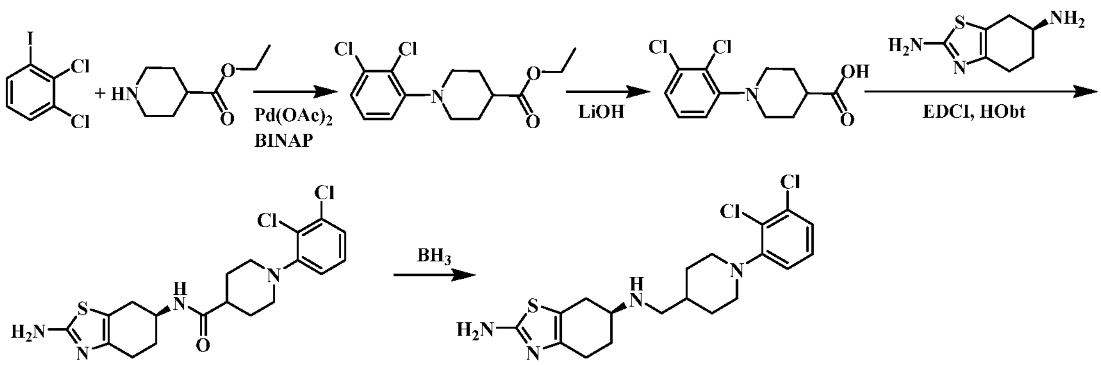


Step A: Synthesis of ethyl 1-(2,3-dichlorophenyl)piperidine-4-carboxylate

1,2-Dichloro-3-iodobenzene (44.3 mmol,1 eq), ethyl 4-piperidine-carboxylate (44.3 mmol,1 eq), $Pd(OAc)_2$ (1.1 mmol, 0.025 eq), BINAP (4.43 mmol, 0.1 eq), and $Cs_2CO_3$ (66.45 mmol, 1.5 eq) were taken and dissolved in toluene (200

mL), and after being purged with nitrogen, the mixture was heated to 110 °C and refluxed for 15 h. After the reaction was completed, suction filtration was performed using celite. The filtrate was concentrated, and the crude product was subjected to silica gel column chromatography to obtain the intermediate ethyl 1-(2,3-dichlorophenyl)piperidine-4-carboxylate (6.1 g, 45.6%).

ESI-MS$[M+H]^+$: m/z 302.1.

$^1$H NMR (600 MHz, DMSO-d6) δ 7.33 - 7.26 (m, 2H), 7.13 (dd, J = 7.0, 2.5 Hz, 1H), 4.10 (q, J = 7.1 Hz, 2H), 3.27 - 3.18 (m, 3H), 2.72 (td, J = 11.6, 2.5 Hz, 2H), 1.98 - 1.91 (m, 2H), 1.79 - 1.70 (m, 2H), 1.21 (t, J = 7.1 Hz, 3H).

Step B: Synthesis of 1-(2,3-dichlorophenyl)piperidine-4-carboxylic acid

The intermediate ethyl 1-(2,3-dichlorophenyl)piperidine-4-carboxylate (20.2 mmol, 1 eq) was dissolved in THF:MeOH (1:1, 60 mL), and an aqueous solution of LiOH $H_2O$ (30.3 mmol, 1.5 eq) (10 mL) was added. The mixture was stirred at room temperature for 18 h. After the reaction was completed, the solvent was removed by evaporation. Then, 10 mL of water was added to dissolve the solid, and the organic impurities were removed by extraction with ethyl acetate. The aqueous layer was taken, adjusted to pH = 2, and extracted with dichloromethane (50 mL × 3), and the organic layers were combined, washed with saturated brine, dried over anhydrous sodium sulfate, concentrated, and then dried to obtain the intermediate 1-(2,3-dichlorophenyl)piperidine-4-carboxylic acid (5.3 g, 95.8%).

ESI-MS$[M+H]^+$: m/z 274.1.

Step C: Synthesis of (S)-N-(2-amino-4,5,6,7-tetrahydrobenzo[d]thiazol-6-yl)-1-(2,3-dichlorophenyl)piperidine-4-carboxamide

The intermediate 1-(2,3-dichlorophenyl)piperidine-4-carboxylic acid (10.9 mmol, 1 eq) produced in the previous step, EDCI (13.1 mmol, 1.2 eq), HObt (13.1 mmol, 1.2 eq), and triethylamine (21.8 mmol, 2 eq) were taken and dissolved in dichloromethane (50 mL), and the mixture was stirred under an ice bath for 20 min. (S)-4,5,6,7-Tetrahydrobenzo[d]thiazole-2,6-diamine (21.8 mmol, 2 eq) was added, and the mixture was stirred at room temperature for 4 h. After the reaction was completed, 20 mL of water was added, and the mixture was extracted with dichloromethane (30 mL × 3). The organic layers were combined, washed with saturated brine, dried over anhydrous sodium sulfate, and concentrated, and the residue was purified by column chromatography to obtain the intermediate (S)-N-(2-amino-4,5,6,7-tetrahydrobenzo[d]thiazol-6-yl)-1-(2,3-dichlorophenyl)piperidine-4-carboxamide (1.5 g, 32.6%).

ESI-MS[M+H]+: m/z 425.2.

Step D: Synthesis of (S)-$N^6$-((1-(2,3-dichlorophenyl)piperidin-4-yl)methyl)-4,5,6,7-tetrahydrobenzo[d]thiazole-2,6-diamine

The intermediate (S)-N-(2-amino-4,5,6,7-tetrahydrobenzo[d]thiazol-6-yl)-1-(2,3-dichlorophenyl)piperidine-4-carboxamide (1.8 g, 4.4 mmol) produced in the previous step was taken and dissolved in anhydrous THF (30 mL), and 1 M $BH_3$ THF (8.7 mL) was added under an ice bath. The mixture was heated and stirred at 60 °C for 3-5 days. TLC monitoring indicated the completion of the reaction. The reaction solution was cooled to room temperature, and 10 mL of methanol was added at 0 °C, followed by concentration of the reaction solution. The sample was acidified using a 4 M solution of hydrogen chloride in methanol and stirred at room temperature for 2 h. The compound was then liberated with a saturated sodium carbonate solution. The aqueous phase was extracted with DCM, and the organic phases were combined, washed with saturated brine, dried over anhydrous sodium sulfate, and concentrated. The residue was purified by column chromatography to obtain the target product.

ESI-MS$[M+H]^+$: m/z 411.0.

Preparation of hydrochloride: Synthesis of (S)-$N^6$-((1-(2,3-dichlorophenyl)piperidin-4-yl)methyl)-4,5,6,7-tetrahydrobenzo[d]thiazole-2,6-diamine dihydrochloride

(S)-$N^6$-((1-(2,3-Dichlorophenyl)piperidin-4-yl)methyl)-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the dihydrochloride of the compound.

ESI-MS$[M+H]^+$: m/z 411.0.

$^1$H NMR (400 MHz, DMSO) δ 8.82 (s, 1H), 8.72 (s, 1H), 7.38 - 7.34 (m, 1H), 7.33 (s, 1H), 7.20 (dd, J = 6.8, 2.9 Hz, 1H), 6.86 (s, 2H), 3.53 - 3.48 (m, 1H), 3.13 - 2.92 (m, 3H), 2.79 - 2.65 (m, 4H), 2.65 - 2.54 (m, 2H), 2.53 - 2.44 (m, 2H), 2.30 -

2.20 (m, 1H), 1.94 - 1.86 (m, 4H), 1.52 - 1.42 (m, 2H).

**Example 5**

**(S)-N$^6$-(2-(1-(2,3-Dichlorophenyl)piperidin-4-yl)ethyl)-4,5,6,7-tetrahydrobenzo[d]thiazole-2,6-diamine and dihydrochloride thereof**

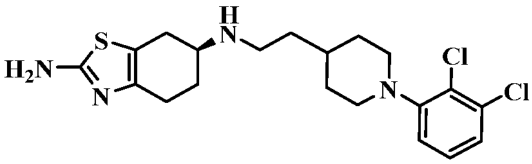

(S)-*N*$^6$-(2-(1-(2,3-Dichlorophenyl)piperidin-4-yl)ethyl)-4,5,6,7-tetrahydrobenzo[*d*]thiazole-2,6-diamine

Synthesis scheme:

The synthesis method in Example 4 was employed, except that ethyl 4-piperidine-carboxylate used in step A was replaced by ethyl 2-(piperidin-4-yl)acetate, and the target product was prepared by steps A-D.

ESI-MS[M+H]$^+$: m/z 425.0.

Preparation of hydrochloride: Synthesis of (S)-N$^6$-(2-(1-(2,3-dichlorophenyl)piperidin-4-yl)ethyl)-4,5,6,7-tetrahydrobenzo[d]thiazole-2,6-diamine dihydrochloride

(S)-N$^6$-(2-(1-(2,3-Dichlorophenyl)piperidin-4-yl)ethyl)-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the dihydrochloride of the compound.

ESI-MS[M+H]$^+$: m/z 425.0.

$^1$H NMR (600 MHz, DMSO-$d_6$) δ 7.33 - 7.28 (m, 1H), 7.30 - 7.26 (m, 1H), 7.14 (dd, J = 7.6, 2.0 Hz, 1H), 6.71 (s, 2H), 3.29 - 3.24 (m, 3H), 2.91 - 2.88 (m, 3H), 2.67 - 2.60 (m, 2H), 2.46 - 2.43 (m, 2H), 2.09 - 2.06 (m, 1H), 1.81 - 1.76 (m, 2H), 1.72 - 1.69 (m, 1H), 1.61 - 1.46 (m, 3H), 1.41 - 1.30 (m, 2H), 1.29 - 1.22 (m, 2H).

**Example 6**

**(S)-N$^6$-((1-(Benzo[b]thiophen-4-yl)piperidin-4-yl)methyl)-4,5,6,7-tetrahydrobenzo[d]thiazole-2,6-diamine and dihydrochloride thereof**

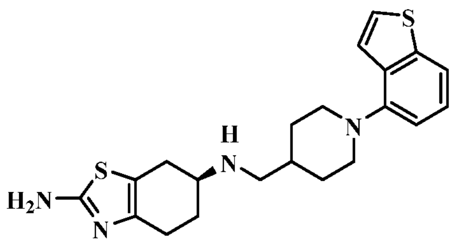

Synthesis scheme of (S)-N$^6$-((1-(benzo[b]thiophen-4-yl)piperidin-4-yl)methyl)-4,5,6,7-tetrahydrobenzo[d]thiazole-2,6-diamine:

The synthesis method in Example 4 was employed, except that 1,2-dichloro-3-iodobenzene used in step A was replaced by 4-bromobenzo[b]thiophene, and the target product was prepared by steps A-D.

ESI-MS[M+H]$^+$: m/z 399.0.

Preparation of hydrochloride: Synthesis of (S)-$N^6$-((1-(benzo[b]thiophen-4-yl)piperidin-4-yl)methyl)-4,5,6,7-tetrahydrobenzo[d]thiazole-2,6-diamine dihydrochloride

(S)-$N^6$-((1-(Benzo[b]thiophen-4-yl)piperidin-4-yl)methyl)-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the dihydrochloride of the compound.

ESI-MS$[M+H]^+$: m/z 399.0.

$^1H$ NMR (400 MHz, DMSO) δ 9.47 (s, 1H), 9.38 (s, 2H), 9.30 (s, 1H), 7.85 - 7.65 (m, 2H), 7.51 (s, 1H), 7.36 (t, J = 7.9 Hz, 1H), 7.11 - 7.07 (m, 1H), 3.62 - 3.53 (m, 3H), 3.53 - 3.50 (m, 1H), 3.50 - 3.42 (m, 3H), 3.15 - 3.06 (m, 1H), 2.98 - 2.78 (m, 2H), 2.77 - 2.68 (m, 1H), 2.64 - 2.57 (m, 1H), 2.38 - 2.31 (m, 1H), 2.10 - 2.03 (m, 4H), 1.79 - 1.53 (m, 2H).

**Example 7**

**(S)-$N^6$-((1-(2,3-Dichlorophenyl)piperidin-4-yl)methyl)-$N^6$-propyl-4,5,6,7-tetrahydrobenzo[d]thiazole-2,6-diamine and hydrochloride thereof**

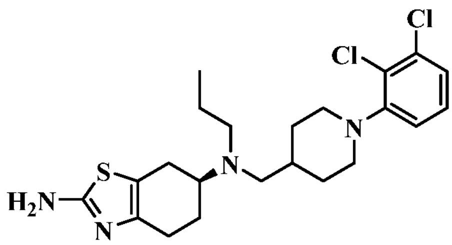

Synthesis scheme of (S)-$N^6$-((1-(2,3-dichlorophenyl)piperidin-4-yl)methyl)-$N^6$-propyl-4,5,6,7-tetrahydrobenzo[d]thiazole-2,6-diamine:

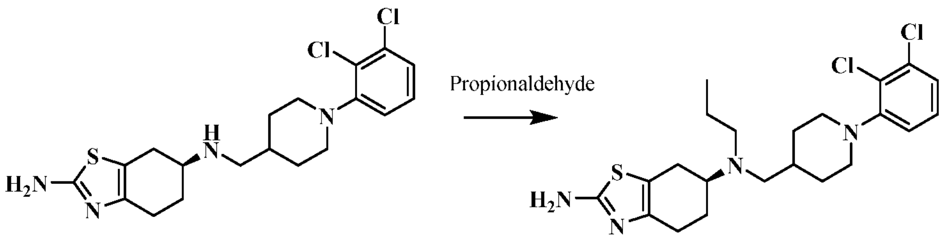


The target product (S)-$N^6$-((1-(2,3-dichlorophenyl)piperidin-4-yl)methyl)-4,5,6,7-tetrahydrobenzo[d]thiazole-2,6-diamine (1.6 mmol, 1 eq) produced in Example 4 and propionaldehyde (1.6 mmol, 1 eq) were taken as starting materials, and the synthesis method in Example 2 was employed to prepare the target product.

ESI-MS$[M+H]^+$: m/z 453.1.

Preparation of hydrochloride: Synthesis of (S)-$N^6$-((1-(2,3-dichlorophenyl)piperidin-4-yl)methyl)-$N^6$-propyl-4,5,6,7-tetrahydrobenzo[d]thiazole-2,6-diamine hydrochloride

(S)-$N^6$-((1-(2,3-Dichlorophenyl)piperidin-4-yl)methyl)-$N^6$-propyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 453.1.

$^1H$ NMR (400 MHz, DMSO) δ 10.56 (s, 1H), 9.81 - 9.24 (m, 2H), 7.38 - 7.30 (m, 2H), 7.18 (dd, J = 7.1, 2.6 Hz, 1H), 3.36 - 3.22 (m, 3H), 3.17 - 3.12 (m, 2H), 3.09 - 2.81 (m, 2H), 2.80 - 2.58 (m, 4H), 2.49 - 2.38 (m, 1H), 2.32 - 2.13 (m, 1H), 2.13 - 1.79 (m, 6H), 1.63 - 1.30 (m, 3H), 1.01 - 0.91 (m, 3H).

**Example 8**

**(S)-$N^6$-(2-(1-(2,3-Dichlorophenyl)piperidin-4-yl)ethyl)-$N^6$-propyl-4,5,6,7-tetrahydrobenzo[d]thiazole-2,6-diamine and hydrochloride thereof**

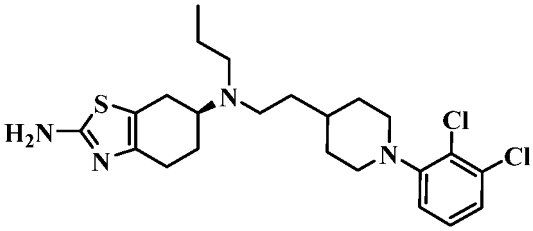

Synthesis scheme of (S)-$N^6$-(2-(1-(2,3-dichlorophenyl)piperidin-4-yl)ethyl)-$N^6$-propyl-4,5,6,7-tetrahydrobenzo[d]thiazole-2,6-diamine:

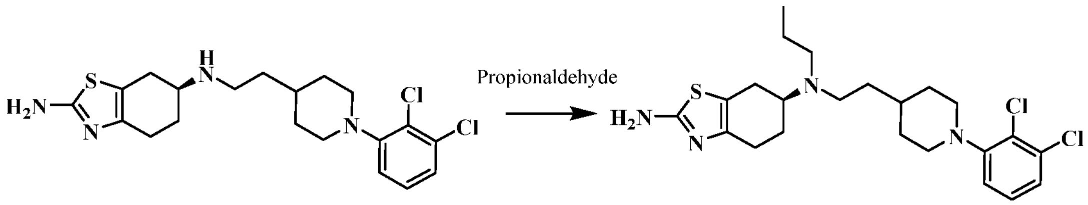


The target product (S)-$N^6$-(2-(1-(2,3-dichlorophenyl)piperidin-4-yl)ethyl)-4,5,6,7-tetrahydrobenzo[d]thiazole-2,6-diamine (0.5 mmol, 1 eq) produced in Example 5 and propionaldehyde (0.5 mmol, 1 eq) were taken as starting materials, and the synthesis method in Example 2 was employed to prepare the target product.
ESI-MS$[M+H]^+$: m/z 467.3.

Preparation of hydrochloride: Synthesis of (S)-$N^6$-(2-(1-(2,3-dichlorophenyl)piperidin-4-yl)ethyl)-$N^6$-propyl-4,5,6,7-tetrahydrobenzo[d]thiazole-2,6-diamine hydrochloride

(S)-$N^6$-(2-(1-(2,3-Dichlorophenyl)piperidin-4-yl)ethyl)-$N^6$-propyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.
ESI-MS$[M+H]^+$: m/z 467.3.
$^1H$ NMR (600 MHz, DMSO-$d_6$) 9.27 - 8.86 (m, 2H), 7.33 - 7.27 (m, 2H), 7.15 (dd, J = 7.5, 2.1 Hz, 1H), 3.62 - 3.47 (m, 2H), 3.29 (s, 1H), 3.27 (s, 1H), 3.07 - 3.01 (m, 3H), 2.76 - 2.69 (m, 1H), 2.68 - 2.62 (m, 2H), 2.61 - 2.54 (m, 1H), 2.48 - 2.42 (m, 2H), 2.28 - 2.23 (m, 1H), 1.95 - 1.92 (m, 1H), 1.83 - 1.77 (m, 2H), 1.72 - 1.65 (m, 2H), 1.64 - 1.55 (m, 2H), 1.55 - 1.50 (m, 1H), 1.42 - 1.32 (m, 2H), 0.93 (t, J = 7.4 Hz, 3H).

**Example 9**

**(S)-$N^6$-((1-(Benzo[b]thiophen-4-yl)piperidin-4-yl)methyl)-$N^6$-propyl-4,5,6,7-tetrahydrobenzo[d]thiazole-2,6-diamine and hydrochloride thereof**

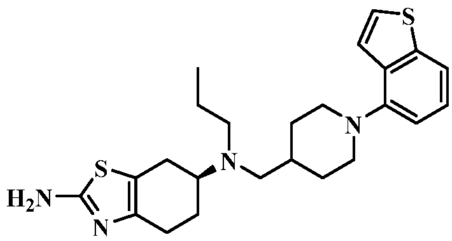

Synthesis scheme of (S)-$N^6$-((1-(benzo[b]thiophen-4-yl)piperidin-4-yl)methyl)-$N^6$-propyl-4,5,6,7-tetrahydrobenzo[d]thiazole-2,6-diamine:

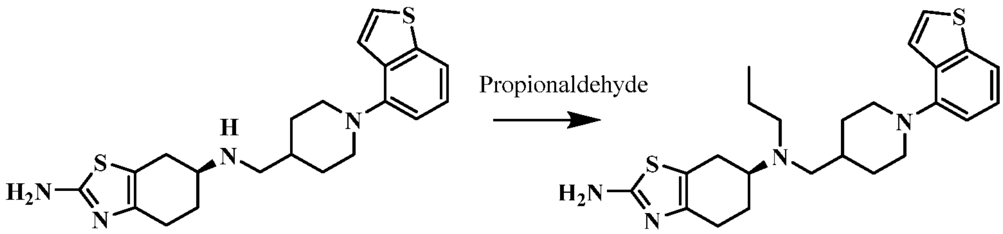


The target product (S)-$N^6$-((1-(benzo[b]thiophen-4-yl)piperidin-4-yl)methyl)-4,5,6,7-tetrahydrobenzo[d]thiazole-2,6-diamine (1.7 mmol, 1 eq) produced in Example 6 and propionaldehyde (1.7 mmol, 1 eq) were taken as starting materials, and the synthesis method in Example 2 was employed to prepare the target product.

ESI-MS$[M+H]^+$: m/z 441.1.

Preparation of hydrochloride: Synthesis of (S)-$N^6$-((1-(benzo[b]thiophen-4-yl)piperidin-4-yl)methyl)-$N^6$-propyl-4,5,6,7-tetrahydrobenzo[d]thiazole-2,6-diamine hydrochloride

(S)-$N^6$-((1-(Benzo[b]thiophen-4-yl)piperidin-4-yl)methyl)-$N^6$-propyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 441.1.

$^1$H NMR (400 MHz, DMSO) δ 10.15 (s, 1H), 9.30 (s, 2H), 7.77 (d, J = 5.5 Hz, 1H), 7.69 (d, J = 8.2 Hz, 1H), 7.44 (s, 1H), 7.33 (t, J = 7.9 Hz, 1H), 7.00 (s, 1H), 3.54 - 3.44 (m, 3H), 3.33 - 3.31 (m, 1H), 3.27 - 3.07 (m, 5H), 2.85 - 2.81 (m, 4H), 2.77 - 2.67 (m, 1H), 2.44 - 2.39 (m, 2H), 2.28 - 2.16 (m, 1H), 2.08 - 2.04 (m, 3H), 1.89 - 1.84 (m, 2H), 1.02 - 0.94 (m, 3H).

**Example 10**

**(S)-$N^6$-(2-(1-(Benzo[b]thiophen-4-yl)piperidin-4-yl)ethyl)-$N^6$-propyl-4,5,6,7-tetrahydrobenzo[d]thiazole-2,6-diamine and hydrochloride thereof**

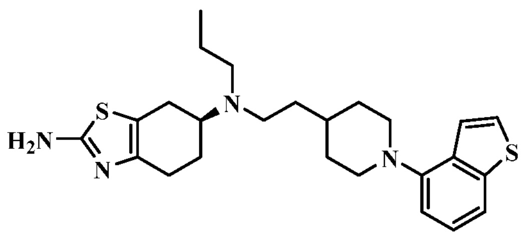

Synthesis scheme of (S)-$N^6$-(2-(1-(benzo[b]thiophen-4-yl)piperidin-4-yl)ethyl)-$N^6$-propyl-4,5,6,7-tetrahydrobenzo[d]thiazole-2,6-diamine:

The synthesis method in Example 4 was employed, except that 1,2-dichloro-3-iodobenzene and ethyl 4-piperidine-carboxylate used in step A were replaced by 4-bromobenzo[b]thiophene and ethyl 2-(piperidin-4-yl)acetate, respectively, and the intermediate (S)-$N^6$-(2-(1-(benzo[b]thiophen-4-yl)piperidin-4-yl)ethyl)-4,5,6,7-tetrahydrobenzo[d]thiazole-2,6-diamine was prepared by steps A-D. The intermediate (1.2 mmol, 1 eq) and propionaldehyde (1.2 mmol, 1 eq) were taken as starting materials, and the synthesis method in Example 2 was employed to prepare the target product. ESI-MS$[M+H]^+$: m/z 455.1.

Preparation of hydrochloride: Synthesis of (S)-$N^6$-(2-(1-(benzo[b]thiophen-4-yl)piperidin-4-yl)ethyl)-$N^6$-propyl-4,5,6,7-tetrahydrobenzo[d]thiazole-2,6-diamine hydrochloride

(S)-$N^6$-(2-(1-(Benzo[b]thiophen-4-yl)piperidin-4-yl)ethyl)-$N^6$-propyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 455.1.

$^1H$ NMR (600 MHz, DMSO-$d_6$) $\delta$ 10.66 (s, 1H), 9.30 (s, 2H), 7.74 (s, 1H), 7.69 (s, 1H), 7.46 (s, 1H), 7.31 (s, 1H), 7.02 (s, 1H), 3.83 - 3.65 (m, 2H), 3.33 - 3.21 (m, 1H), 3.19 - 3.13 (m, 2H), 3.12 - 3.07 (m, 1H), 3.07 - 3.01 (m, 1H), 2.93 - 2.84 (m, 1H), 2.83 - 2.75 (m, 1H), 2.75 - 2.71 (m, 1H), 2.71 - 2.66 (m, 1H), 2.65 - 2.62 (m, 1H), 2.62 - 2.58 (m, 1H), 2.41 - 2.38 (m, 1H), 2.38 - 2.33 (m, 1H), 1.99 - 1.96 (m, 1H), 1.89 - 1.86 (m, 1H), 1.85 - 1.80 (m, 3H), 1.80 - 1.74 (m, 1H), 1.58 (m, 3H), 0.95 (t, J = 7.3 Hz, 3H).

## Example 11

**(R)-$N^6$-((1-(Benzo[b]thiophen-4-yl)piperidin-4-yl)methyl)-$N^6$-propyl-4,5,6,7-tetrahydrobenzo[d]thiazole-2,6-diamine and hydrochloride thereof**

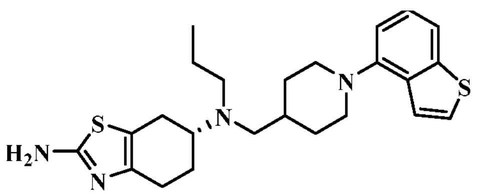

Synthesis scheme of (R)-$N^6$-((1-(benzo[b]thiophen-4-yl)piperidin-4-yl)methyl)-$N^6$-propyl-4,5,6,7-tetrahydrobenzo[d]thiazole-2,6-diamine:

The synthesis method in Example 4 was employed, except that 1,2-dichloro-3-iodobenzene used in step A and (S)-4,5,6,7-tetrahydrobenzo[d]thiazole-2,6-diamine used in step C were replaced by 4-bromobenzo[b]thiophene and (R)-4,5,6,7-tetrahydrobenzo[d]thiazole-2,6-diamine, respectively, and the intermediate (R)-$N^6$-((1-(benzo[b]thiophen-4-yl)piperidin-4-yl)methyl)-4,5,6,7-tetrahydrobenzo[d]thiazole-2,6-diamine was prepared by steps A-D. The intermediate (0.75 mmol, 1 eq) and propionaldehyde (0.75 mmol, 1 eq) were taken as starting materials, and the synthesis method in Example 2 was employed to prepare the target product.

ESI-MS$[M+H]^+$: m/z 441.1.

Preparation of hydrochloride: Synthesis of (R)-$N^6$-((1-(benzo[b]thiophen-4-yl)piperidin-4-yl)methyl)-$N^6$-propyl-4,5,6,7-tetrahydrobenzo[d]thiazole-2,6-diamine hydrochloride

(R)-$N^6$-((1-(Benzo[b]thiophen-4-yl)piperidin-4-yl)methyl)-$N^6$-propyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 441.1.

$^1H$ NMR (600 MHz, DMSO-$d_6$) $\delta$ 7.67 (d, J = 5.5 Hz, 1H), 7.61 - 7.56 (m, 1H), 7.38 (dd, J = 5.5, 0.8 Hz, 1H), 7.26 (t, J

= 7.8 Hz, 1H), 6.89 (dd, J = 7.7, 0.9 Hz, 1H), 6.59 (s, 2H), 3.42 (d, J = 11.5 Hz, 2H), 3.36 - 3.32 (m, 1H), 3.32 - 3.28 (m, 1H), 2.98 - 2.88 (m, 1H), 2.72 - 2.65 (m, 2H), 2.58 - 2.47 (m, 1H), 2.48 - 2.41 (m, 3H), 2.41 - 2.35 (m, 2H), 1.93 - 1.83 (m, 3H), 1.68 - 1.59 (m, 1H), 1.45 - 1.31 (m, 5H), 0.88 (t, J = 7.3 Hz, 3H).

**Example 12**

**$N^6$-((1-(Benzo[b]thiophen-4-yl)piperidin-4-yl)methyl)-$N^6$-propyl-4,5,6,7-tetrahydrobenzo[d]thiazole-2,6-diamine and hydrochloride thereof**

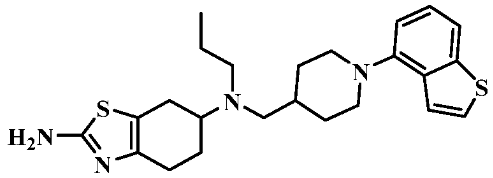

Synthesis scheme of $N^6$-((1-(benzo[b]thiophen-4-yl)piperidin-4-yl)methyl)-$N^6$-propyl-4,5,6,7-tetrahydrobenzo[d]thiazole-2,6-diamine:

The synthesis method in Example 11 was employed, except that (R)-4,5,6,7-tetrahydrobenzo[d]thiazole-2,6-diamine used in step C was replaced by 4,5,6,7-tetrahydrobenzo[d]thiazole-2,6-diamine, and the intermediate $N^6$-((1-(benzo[b]thiophen-4-yl)piperidin-4-yl)methyl)-4,5,6,7-tetrahydrobenzo[d]thiazole-2,6-diamine was prepared by steps A-D. The intermediate (0.5 mmol, 1 eq) and propionaldehyde (0.5 mmol, 1 eq) were taken as starting materials, and the synthesis method in Example 2 was employed to prepare the target product.
ESI-MS$[M+H]^+$: m/z 441.1.

Preparation of hydrochloride: Synthesis of $N^6$-((1-(benzo[b]thiophen-4-yl)piperidin-4-yl)methyl)-$N^6$-propyl-4,5,6,7-tetrahydrobenzo[d]thiazole-2,6-diamine hydrochloride

$N^6$-((1-(Benzo[b]thiophen-4-yl)piperidin-4-yl)methyl)-$N^6$-propyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.
ESI-MS$[M+H]^+$: m/z 441.1.
$^1H$ NMR (400 MHz, DMSO-$d_6$) δ 10.25 (s, 1H), 9.28 (s, 2H), 7.73 (d, J = 5.5 Hz, 1H), 7.65 (d, J = 7.9 Hz, 1H), 7.44 - 7.39 (m, 1H), 7.30 (t, J = 7.8 Hz, 1H), 6.99 - 6.95 (m, 1H), 3.50 - 3.42 (m, 3H), 3.36 - 3.22 (m, 1H), 3.19 - 3.00 (m, 4H), 2.98 - 2.77 (m, 2H), 2.77 - 2.55 (m, 1H), 2.48 - 2.30 (m, 1H), 2.26 - 2.13 (m, 1H), 2.09 - 1.98 (m, 3H), 1.89 - 1.74 (m, 2H), 1.67 - 1.50 (m, 3H), 1.17 (t, J = 7.1 Hz, 1H), 0.98 - 0.90 (m, 3H).

**Example 13**

**(S)-$N^6$-((1-(Benzo[b]thiophen-4-yl)piperidin-4-yl)methyl)-$N^6$-ethyl-4,5,6,7-tetrahydrobenzo[d]thiazole-2,6-diamine and hydrochloride thereof**

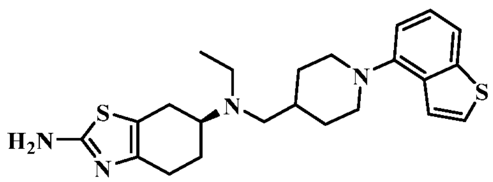

Synthesis scheme of (S)-N6-((1-(benzo[b]thiophen-4-yl)piperidin-4-yl)methyl)-N6-ethyl-4,5,6,7-tetrahydrobenzo[d]thiazole-2,6-diamine:

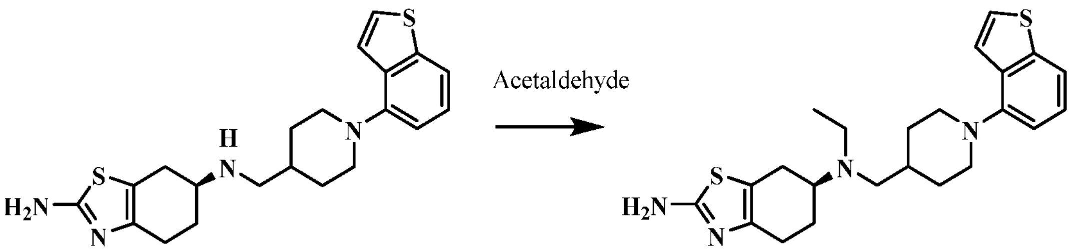


The target product (S)-N6-((1-(benzo[b]thiophen-4-yl)piperidin-4-yl)methyl)-4,5,6,7-tetrahydrobenzo[d]thiazole-2,6-diamine (0.25 mmol, 1 eq) produced in Example 6 and acetaldehyde (0.25 mmol, 1 eq) were taken as starting materials, and the synthesis method in Example 2 was employed to prepare the target product.

ESI-MS$[M+H]^+$: m/z 427.0.

Preparation of hydrochloride: Synthesis of (S)-N6-((1-(benzo[b]thiophen-4-yl)piperidin-4-yl)methyl)-N6-ethyl-4,5,6,7-tetrahydrobenzo[d]thiazole-2,6-diamine hydrochloride

(S)-N6-((1-(Benzo[b]thiophen-4-yl)piperidin-4-yl)methyl)-N6-ethyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 427.0.

$^1$H NMR (600 MHz, DMSO-$d_6$) δ 9.62 (s, 1H), 7.72 (dd, *J* = 5.5, 1.5 Hz, 1H), 7.63 (d, *J* = 8.0 Hz, 1H), 7.38 (d, *J* = 5.5 Hz, 1H), 7.29 (t, *J* = 7.8 Hz, 1H), 6.93 (d, *J* = 7.7 Hz, 1H), 3.80 - 3.68 (m, 1H), 3.49 - 3.44 (m, 3H), 3.32 - 3.20 (m, 1H), 3.11 - 2.97 (m, 3H), 2.93 - 2.81 (m, 1H), 2.77 - 2.70 (m, 1H), 2.68 (s, 1H), 2.65 - 2.62 (m, 1H), 2.62 - 2.59 (m, 1H), 2.41 - 2.39 (m, 1H), 2.37 - 2.33 (m, 1H), 2.15 - 2.08 (m, 1H), 2.04 - 1.98 (m, 1H), 1.98 - 1.94 (m, 1H), 1.60 - 1.52 (m, 2H), 1.37 - 1.33 (m, 3H).

**Example 14**

**(S)-N6-((1-(Benzo[b]thiophen-4-yl)piperidin-4-yl)methyl)-N6-methyl-4,5,6,7-tetrahydrobenzo[d]thiazole-2,6-diamine and hydrochloride thereof**

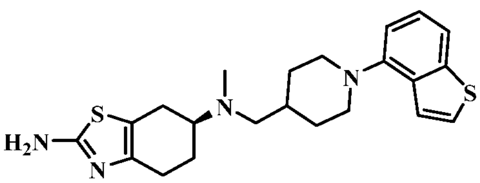

Synthesis scheme of (S)-N6-((1-(benzo[b]thiophen-4-yl)piperidin-4-yl)methyl)-N6-methyl-4,5,6,7-tetrahydrobenzo[d]thiazole-2,6-diamine:

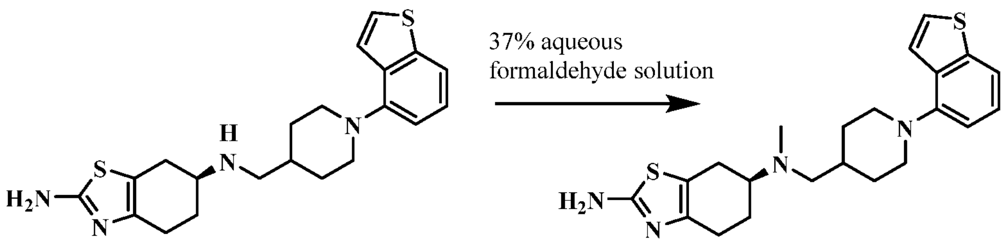


The target product (S)-$N^6$-((1-(benzo[b]thiophen-4-yl)piperidin-4-yl)methyl)-4,5,6,7-tetrahydrobenzo[d]thiazole-2,6-diamine (0.75 mmol, 1 eq) produced in Example 6 and a 37% aqueous formaldehyde solution (1.5 mmol, 2 eq) were taken and dissolved in a formic acid solution (6 mL), and the mixture was refluxed at 90 °C for 6 h. After the reaction was completed, the system was cooled to room temperature, and 10 mL of water was added. The reaction solution was made neutral with saturated $Na_2CO_3$ and then extracted with ethyl acetate (10 mL × 2). The organic layers were combined, washed with saturated brine, dried, and concentrated, and the residue was purified by column chromatography to obtain the target product.

ESI-MS$[M+H]^+$: m/z 413.0.

Preparation of hydrochloride: Synthesis of (S)-$N^6$-((1-(benzo[b]thiophen-4-yl)piperidin-4-yl)methyl)-$N^6$-methyl-4,5,6,7-tetrahydrobenzo[d]thiazole-2,6-diamine hydrochloride

(S)-$N^6$-((1-(Benzo[b]thiophen-4-yl)piperidin-4-yl)methyl)-$N^6$-methyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 413.0.

$^1H$ NMR (600 MHz, DMSO-$d_6$) δ 10.60 (s, 1H), 9.34 (s, 2H), 7.75 (d, J = 5.3 Hz, 1H), 7.70 - 7.67 (m, 1H), 7.46 - 7.43 (m, 1H), 7.32 (t, J = 8.0 Hz, 1H), 7.03 - 7.00 (m, 1H), 3.50 - 3.46 (m, 6H), 3.28 - 3.25 (m, 1H), 3.17 - 2.96 (m, 1H), 2.93 - 2.86 (m, 1H), 2.86 - 2.81 (m, 4H), 2.76 - 2.69 (m, 1H), 2.67 - 2.57 (m, 1H), 2.26 - 2.19 (m, 1H), 2.09 - 2.06 (m, 1H), 2.00 - 1.90 (m, 2H), 1.62 - 1.59 (m, 2H).

**Example 15**

**(S)-(4-(((2-Amino-4,5,6,7-tetrahydrobenzo[d]thiazol-6-yl)(propyl)amino)methyl)piperidin-1-yl)(3-chloro-4-fluorophenyl)methanone and hydrochloride thereof**

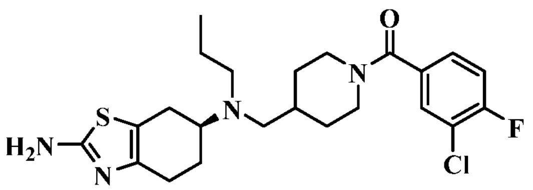

Synthesis scheme of (S)-(4-(((2-amino-4,5,6,7-tetrahydrobenzo[d]thiazol-6-yl)(propyl) amino)methyl)piperidin-1-yl)(3-chloro-4-fluorophenyl)methanone:

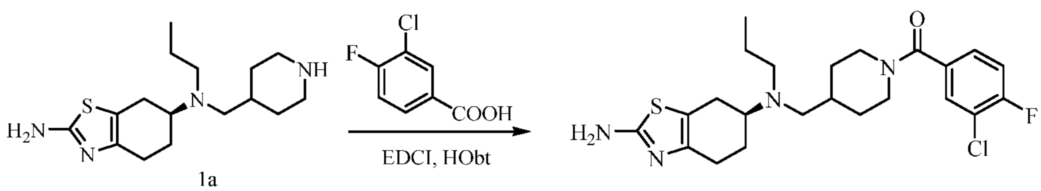


1a

3-Chloro-4-fluorobenzoic acid (2.7 mmol, 1.0 eq), EDCI (3.2 mmol, 1.2 eq), HObt (3.2 mmol, 1.2 eq), and

triethylamine (5.4 mmol, 2 eq) were taken and dissolved in dichloromethane (20 mL), and the mixture was stirred under an ice bath for 20 min. Intermediate **1a** (3.2 mmol, 1.2 eq) was added, and the mixture was allowed to react at room temperature for 1.5 h. After the reaction was completed, 20 mL of water was added, and the mixture was extracted with dichloromethane (30 mL × 3). The organic layer was washed with water and saturated brine, dried over $MgSO_4$, and then concentrated, and the residue was purified by column chromatography to obtain the target product. ESI-MS$[M+H]^+$: m/z 465.1.

<u>Preparation of hydrochloride: Synthesis of (S)-(4-(((2-amino-4,5,6,7-tetrahydrobenzo[d]thiazol-6-yl)(propyl)amino)methyl)piperidin-1-yl)(3-chloro-4-fluorophenyl)methanone hydrochloride</u>

(S)-(4-(((2-amino-4,5,6,7-tetrahydrobenzo[d]thiazol-6-yl)(propyl)amino)methyl)piperidin-1-yl)(3-chloro-4-fluorophenyl)methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 465.1.

$^1H$ NMR (600 MHz, DMSO-$d_6$) δ 10.72 (s, 1H), 9.41 (s, 2H), 7.64 - 7.60 (m, 1H), 7.54 - 7.48 (m, 1H), 7.43 - 7.39 (m, 1H), 4.45 - 4.42 (m, 1H), 3.80 - 3.62 (m, 2H), 3.62 - 3.42 (m, 2H), 3.40 - 3.34 (m, 1H), 3.23 - 3.11 (m, 2H), 3.11 - 2.98 (m, 2H), 3.00 - 2.75 (m, 1H), 2.75 - 2.66 (m, 1H), 2.66 - 2.55 (m, 1H), 2.53 - 2.49 (m, 3H), 2.44 - 2.31 (m, 1H), 2.23 - 1.90 (m, 2H), 1.90 - 1.71 (m, 1H), 1.29 - 1.19 (m, 2H), 0.94 - 0.88 (m, 3H).

**Example 16**

**(S)-(4-(2-((2-Amino-4,5,6,7-tetrahydrobenzo[d]thiazol-6-yl)(propyl)amino)ethyl)piperidin-1-yl)(3-chloro-4-fluorophenyl)methanone and hydrochloride thereof**

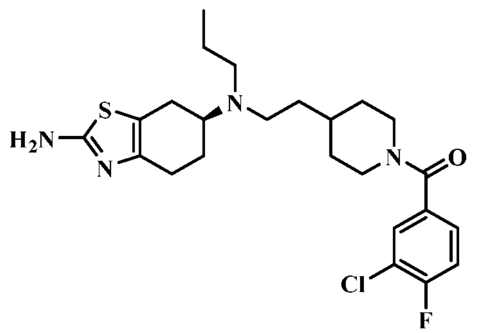

<u>Synthesis scheme of (S)-(4-(2-((2-amino-4,5,6,7-tetrahydrobenzo[d]thiazol-6-yl)(propyl) amino)ethyl)piperidin-1-yl)(3-chloro-4-fluorophenyl)methanone:</u>

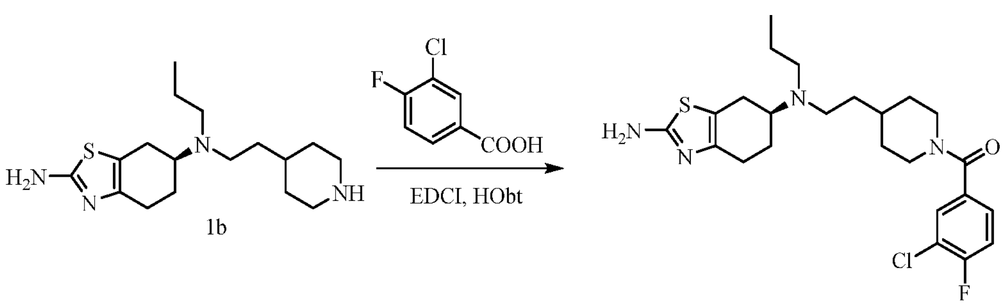


3-Chloro-4-fluorobenzoic acid (1.3 mmol, 1 eq), EDCI (1.6 mmol, 1.2 eq), HObt (1.6 mmol, 1.2 eq), and triethylamine (2.6 mmol, 2 eq) were taken and dissolved in dichloromethane, and the mixture was stirred under an ice bath for 20 min. Intermediate **1b** (1.6 mmol, 1.2 eq) was added, and the mixture was allowed to react at room temperature for 1.5 h. After the reaction was completed, 20 mL of water was added, and the mixture was extracted with dichloromethane

(30 mL × 3). The organic layer was washed with water and saturated brine, dried over $MgSO_4$, and then concentrated, and the residue was purified by column chromatography to obtain the target product.

ESI-MS[M+H]$^+$: m/z 479.1.

Preparation of hydrochloride: Synthesis of (S)-(4-(2-((2-amino-4,5,6,7-tetrahydrobenzo[d]thiazol-6-yl)(propyl)amino) ethyl)piperidin-1-yl)(3-chloro-4-fluorophenyl)methanone hydrochloride

(S)-(4-(2-((2-Amino-4,5,6,7-tetrahydrobenzo[d]thiazol-6-yl)(propyl)amino)ethyl)piperidin-1-yl)(3-chloro-4-fluorophenyl)methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS[M+H]$^+$: m/z 479.1.

$^1$H NMR (600 MHz, DMSO-$d_6$) δ 10.47 (s, 1H), 8.95 (s, 2H), 7.63 (dd, J = 7.2, 2.1 Hz, 1H), 7.50 (t, J = 8.9 Hz, 1H), 7.41 (ddd, J = 8.5, 4.7, 2.1 Hz, 1H), 4.44 - 4.41 (m, 1H), 3.69 - 3.66 (m, 1H), 3.61 - 3.44 (m, 3H), 3.26 - 3.17 (m, 1H), 3.17 - 3.07 (m, 1H), 3.07 - 3.04 (m, 1H), 3.04 - 2.97 (m, 1H), 2.90 - 2.81 (m, 1H), 2.80 - 2.70 (m, 1H), 2.70 - 2.65 (m, 1H), 2.65 - 2.52 (m, 1H), 2.34 - 2.31 (m, 1H),1.98 - 1.90 (m, 1H), 1.87 - 1.67 (m, 5H), 1.67 - 1.53 (m, 2H), 1.33 - 1.05 (m, 2H), 0.93 (t, J = 7.3 Hz, 3H).

**Example 17**

**(S)-(4-(((2-Amino-4,5,6,7-tetrahydrobenzo[d]thiazol-6-yl)(propyl)amino)methyl)piperidin-1-yl)(phenyl)methanone and hydrochloride thereof**

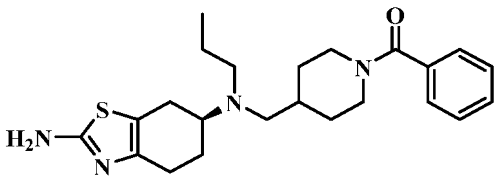

Synthesis scheme of (S)-(4-(((2-amino-4,5,6,7-tetrahydrobenzo[d]thiazol-6-yl)(propyl) amino)methyl)piperidin-1-yl) (phenyl)methanone:

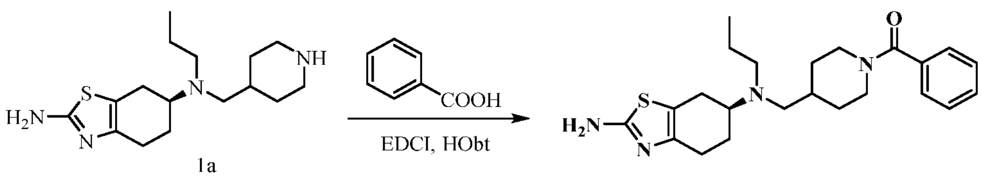


The synthesis method in Example 15 was employed, except that the starting material 3-chloro-4-fluorobenzoic acid was replaced by benzoic acid to prepare the target product. ESI-MS[M+H]$^+$: m/z 413.1.

Preparation of hydrochloride: Synthesis of (S)-(4-(((2-amino-4,5,6,7-tetrahydrobenzo[d]thiazol-6-yl)(propyl)amino) methyl)piperidin-1-yl)(phenyl)methanone hydrochloride

(S)-(4-(((2-Amino-4,5,6,7-tetrahydrobenzo[d]thiazol-6-yl)(propyl)amino)methyl)piperidin-1-yl)(phenyl)methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound. ESI-MS[M+H]$^+$: m/z 413.1.

$^1$H NMR (600 MHz, DMSO-$d_6$) δ 10.30 (s, 1H), 8.99 - 8.49 (m, 2H), 7.50 - 7.42 (m, 3H), 7.40 - 7.34 (m, 2H), 4.55 - 4.39 (m, 1H), 3.70 - 3.65 (m, 1H), 3.59 - 3.56 (m, 1H), 3.24 - 3.15 (m, 1H), 3.14 - 3.03 (m, 3H), 3.00 - 2.72 (m, 1H), 2.72 - 2.55 (m, 1H), 2.42 - 2.26 (m, 1H), 2.12 - 2.09 (m, 1H), 1.99 - 1.96 (m, 4H), 1.96 - 1.89 (m, 2H), 1.87 - 1.77 (m, 2H), 1.26 - 1.23 (m, 1H), 1.22 - 1.20 (m, 2H), 0.95 - 0.89 (m, 3H).

**Example 18**

**(S)-(4-(((2-Amino-4,5,6,7-tetrahydrobenzo[d]thiazol-6-yl)(propyl)amino)methyl)piperidin-1-yl)(6-chloro-5-fluoropyridin-2-yl)methanone and hydrochloride thereof**

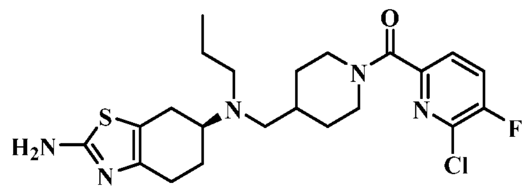

Synthesis scheme of (S)-(4-(((2-amino-4,5,6,7-tetrahydrobenzo[d]thiazol-6-yl)(propyl) amino)methyl)piperidin-1-yl)(6-chloro-5-fluoropyridin-2-yl)methanone:

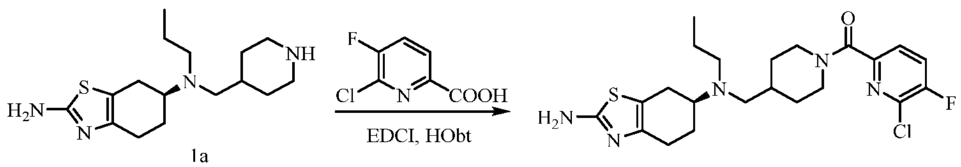


1a

The synthesis method in Example 15 was employed, except that the starting material 3-chloro-4-fluorobenzoic acid was replaced by 2-chloro-3-fluoropyridine-6-carboxylic acid to prepare the target product.
ESI-MS$[M+H]^+$: m/z 466.0.

Preparation of hydrochloride: Synthesis of (S)-(4-(((2-amino-4,5,6,7-tetrahydrobenzo [d]thiazol-6-yl)(propyl)amino) methyl)piperidin-1-yl)(6-chloro-5-fluoropyridin-2-yl)methanone hydrochloride

(S)-(4-(((2-Amino-4,5,6,7-tetrahydrobenzo[d]thiazol-6-yl)(propyl)amino)methyl)piperidin-1-yl)(6-chloro-5-fluoropyridin-2-yl)methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.
ESI-MS$[M+H]^+$: m/z 466.0.
$^1H$ NMR (600 MHz, DMSO-$d_6$) δ 10.21 (s, 1H), 9.07 (s, 2H), 8.07 (t, J = 8.4 Hz, 1H), 7.67 (dd, J = 8.4, 3.4 Hz, 1H), 4.45 (d, J = 12.7 Hz, 1H), 3.69 - 3.63 (m, 2H), 3.21 (s, 1H), 3.15 - 3.07 (m, 4H), 3.07 - 3.02 (m, 1H), 3.02 - 2.93 (m, 1H), 2.93 - 2.74 (m, 1H), 2.70 - 2.65 (m, 1H), 2.64 - 2.53 (m, 1H), 2.40 - 2.27 (m, 1H), 2.20 - 2.06 (m, 1H), 2.05 - 1.89 (m, 1H), 1.90 - 1.71 (m, 2H), 1.27 - 1.21 (m, 4H), 0.95 - 0.88 (m, 3H).

**Example 19**

**(S)-(4-(((2-Amino-4,5,6,7-tetrahydrobenzo[d]thiazol-6-yl)(propyl)amino)methyl)piperidin-1-yl)(6-fluoropyridin-3-yl)methanone and hydrochloride thereof**

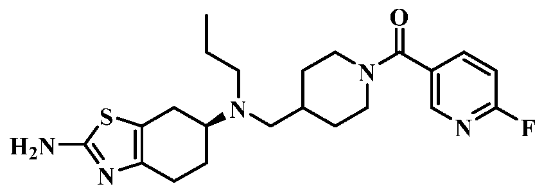

Synthesis scheme of (S)-(4-(((2-amino-4,5,6,7-tetrahydrobenzo[d]thiazol-6-yl)(propyl)amino)methyl)piperidin-1-yl)(6-fluoropyridin-3-yl)methanone:

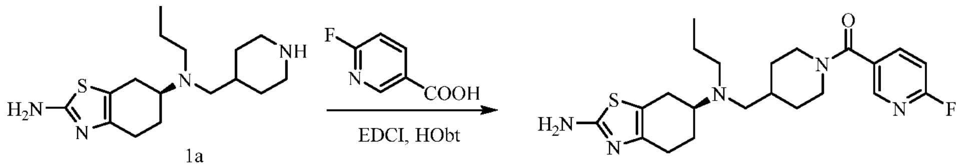


6-Fluoronicotinic acid (2.7 mmol, 1 eq), EDCI (3.2 mmol, 1.2 eq), HObt (3.2 mmol, 1.2 eq), triethylamine (5.4 mmol, 2 eq), and intermediate 1a (3.2 mmol, 1.2 eq) were dissolved in DMF, and the mixture was allowed to react at -10 °C for 5 h. After the reaction was completed, 20 mL of water was added, and the mixture was extracted with dichloromethane (30 mL × 3). The organic layer was washed with water and saturated brine, dried over $MgSO_4$, and then concentrated, and the residue was purified by column chromatography to obtain the target product.

ESI-MS[M+H]$^+$: m/z 432.1.

Preparation of hydrochloride: Synthesis of (S)-(4-(((2-amino-4,5,6,7-tetrahydrobenzo[d]thiazol-6-yl)(propyl)amino) methyl)piperidin-1-yl)(6-fluoropyridin-3-yl)methanone hydrochloride

(S)-(4-(((2-Amino-4,5,6,7-tetrahydrobenzo[d]thiazol-6-yl)(propyl)amino)methyl)piperidin-1-yl)(6-fluoropyridin-3-yl)methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS[M+H]$^+$: m/z 432.1.

$^1$H NMR (600 MHz, DMSO-$d_6$) δ 10.61 (s, 1H), 9.31 (s, 2H), 8.30 (d, J = 2.4 Hz, 1H), 8.03 (td, J = 8.2, 2.4 Hz, 1H), 7.29 (dt, J = 8.6, 2.1 Hz, 1H), 4.61 - 4.34 (m, 1H), 3.68 - 3.65 (m, 3H), 3.62 - 3.46 (m, 6H), 3.13 - 3.03 (m, 3H), 3.00 - 2.75 (m, 1H), 2.72 - 2.57 (m, 1H), 2.43 - 2.30 (m, 1H), 2.18 - 2.06 (m, 2H), 2.04 - 1.72 (m, 1H), 1.29 - 1.18 (m, 3H), 0.91 (td, J = 7.3, 2.7 Hz, 3H).

**Example 20**

**(S)-5-(4-(((2-Amino-4,5,6,7-tetrahydrobenzo[d]thiazol-6-yl)(propyl)amino)methyl)piperidine-1-carbonyl)pyridinecarbonitrile and hydrochloride thereof**

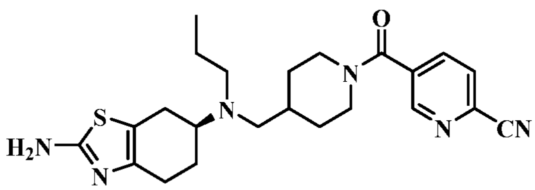

Synthesis scheme of (S)-5-(4-(((2-amino-4,5,6,7-tetrahydrobenzo[d]thiazol-6-yl)(propyl)amino)methyl)piperidine-1-carbonyl)pyridinecarbonitrile:

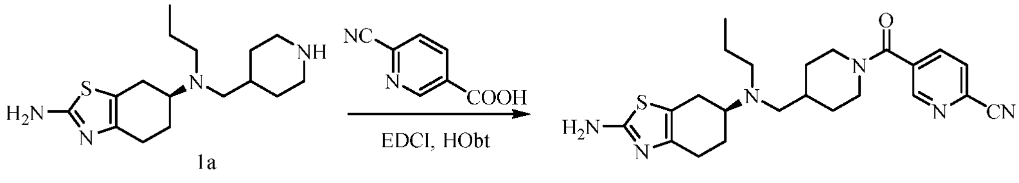


The synthesis method in Example 15 was employed, except that the starting material 3-chloro-4-fluorobenzoic

acid was replaced by 6-cyanonicotinic acid to prepare the target product.

6-Cyanonicotinic acid (4.0 mmol, 1 eq), EDCI (4.8 mmol, 1.2 eq), HObt (4.8 mmol, 1.2 eq), triethylamine (8 mmol, 2 eq), and intermediate 1a (4.8 mmol, 1.2 eq) were dissolved in DMF, and the mixture was allowed to react at -10 °C for 4 h. After the reaction was completed, 20 mL of water was added, and the mixture was extracted with dichloromethane (30 mL × 3). The organic layer was washed with water and saturated brine, dried over $MgSO_4$, and then concentrated, and the residue was purified by column chromatography to obtain the target product.

ESI-MS[M+H]$^+$: m/z 439.1.

Preparation of hydrochloride: Synthesis of (S)-5-(4-(((2-amino-4,5,6,7-tetrahydrobenzo[d]thiazol-6-yl)(propyl)amino) methyl)piperidine-1-carbonyl)pyridinecarbonitrile hydrochloride

(S)-5-(4-(((2-Amino-4,5,6,7-tetrahydrobenzo[d]thiazol-6-yl)(propyl)amino)methyl)piperidine-1-carbonyl)pyridinecarbonitrile was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS[M+H]$^+$: m/z 439.1.

$^1$H NMR (600 MHz, DMSO-$d_6$) δ10.45 (s, 1H), 9.14 (s, 2H), 8.77 (d, J = 2.1 Hz, 1H), 8.15 (dt, J = 8.0, 1.2 Hz, 1H), 8.10 - 8.05 (m, 1H), 4.50 - 4.45 (m, 1H), 3.77 - 3.62 (m, 1H), 3.24 - 3.16 (m, 1H), 3.16 - 3.01 (m, 3H), 2.97 - 2.94 (m, 1H), 2.87 - 2.80 (m, 1H), 2.75 - 2.54 (m, 1H), 2.41 - 2.33 (m, 1H), 2.21 - 2.07 (m, 2H), 2.07 - 1.89 (m, 1H), 1.89 - 1.71 (m, 2H), 1.34 - 1.27 (m, 1H), 1.28 - 1.22 (m, 5H), 1.21 - 1.15 (m, 1H), 0.95 - 0.89 (m, 3H).

**Example 21**

**(S)-(4-(((2-Amino-4,5,6,7-tetrahydrobenzo[d]thiazol-6-yl)(propyl)amino)methyl)piperidin-1-yl)(1H-indol-2-yl) methanone and hydrochloride thereof**

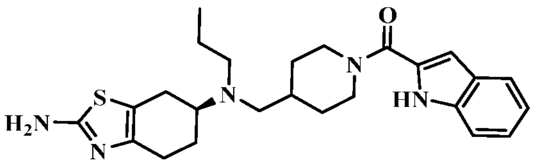

Synthesis scheme of (S)-(4-(((2-amino-4,5,6,7-tetrahydrobenzo[d]thiazol-6-yl)(propyl)amino)methyl)piperidin-1-yl)(1H-indol-2-yl)methanone:

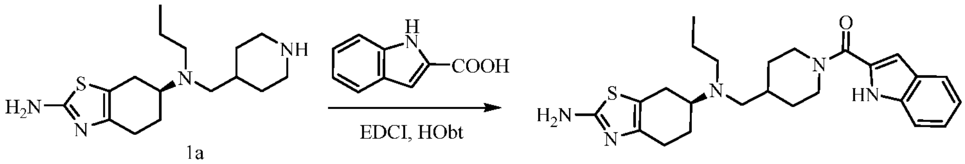


The synthesis method in Example 15 was employed, except that the starting material 3-chloro-4-fluorobenzoic acid was replaced by 2-indolecarboxylic acid to prepare the target product.

ESI-MS[M+H]$^+$: m/z 452.2.

Preparation of hydrochloride: Synthesis of (S)-(4-(((2-amino-4,5,6,7-tetrahydrobenzo[d]thiazol-6-yl)(propyl)amino) methyl)piperidin-1-yl)(1H-indol-2-yl)methanone hydrochloride

(S)-(4-(((2-Amino-4,5,6,7-tetrahydrobenzo[d]thiazol-6-yl)(propyl)amino)methyl)piperidin-1-yl)(1H-indol-2-yl) methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS[M+H]$^+$: m/z 452.2.

$^1H$ NMR (600 MHz, $D_2O$) δ 7.75 (d, J = 8.0 Hz, 1H), 7.56 (d, J = 8.3 Hz, 1H), 7.36 (t, J = 7.7 Hz, 1H), 7.21 (t, J = 7.5 Hz, 1H), 6.94 (s, 1H), 4.83 - 4.82 (m, 1H), 4.82 - 4.79 (m, 3H), 4.76 - 4.74 (m, 1H), 4.52 (d, J = 13.2 Hz, 2H), 3.84 - 3.81 (m, 1H), 3.41 - 3.27 (m, 1H), 3.28 - 3.04 (m, 1H), 3.02 - 2.97 (m, 1H), 2.86 - 2.83 (m, 1H), 2.78 - 2.73 (m, 1H), 2.39 - 2.13 (m, 2H), 2.04 - 1.87 (m, 3H), 1.82 - 1.75 (m, 2H), 1.43 - 1.29 (m, 2H), 1.00 (t, J = 7.3 Hz, 3H).

**Example 22**

**(S)-(4-(((2-Amino-4,5,6,7-tetrahydrobenzo[d]thiazol-6-yl)(propyl)amino)methyl)piperidin-1-yl)(benzo[b]thiophen-2-yl)methanone and hydrochloride thereof**

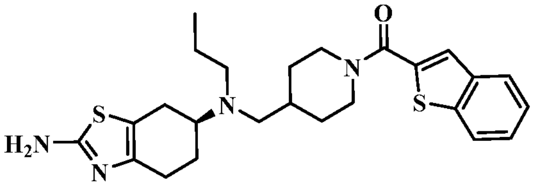

Synthesis scheme of (S)-(4-(((2-amino-4,5,6,7-tetrahydrobenzo[d]thiazol-6-yl)(propyl)amino)methyl)piperidin-1-yl)(benzo[b]thiophen-2-yl)methanone:

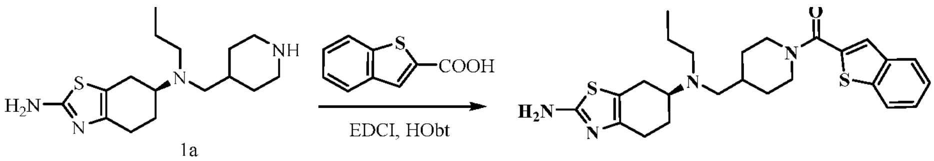


The synthesis method in Example 15 was employed, except that the starting material 3-chloro-4-fluorobenzoic acid was replaced by benzothiophene-2-carboxylic acid to prepare the target product.
ESI-MS[M+H]$^+$: m/z 469.1.

Preparation of hydrochloride: Synthesis of (S)-(4-(((2-amino-4,5,6,7-tetrahydrobenzo[d]thiazol-6-yl)(propyl)amino)methyl)piperidin-1-yl)(benzo[b]thiophen-2-yl)methanone hydrochloride

(S)-(4-(((2-Amino-4,5,6,7-tetrahydrobenzo[d]thiazol-6-yl)(propyl)amino)methyl)piperidin-1-yl)(benzo[b]thiophen-2-yl)methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.
ESI-MS[M+H]$^+$: m/z 469.1.
$^1H$ NMR (600 MHz, DMSO-$d_6$) δ 10.21 (s, 1H), 9.06 (s, 2H), 8.05 - 8.00 (m, 1H), 7.96 - 7.91 (m, 1H), 7.70 (s, 1H), 7.49 - 7.42 (m, 2H), 4.42 - 4.24 (m, 3H), 3.71 - 3.66 (m, 1H), 3.33 - 3.15 (m, 1H), 3.14 - 3.05 (m, 4H), 3.01 - 2.96 (m, 1H), 2.92 - 2.79 (m, 1H), 2.73 - 2.55 (m, 1H), 2.41 - 2.30 (m, 1H), 2.23 - 2.07 (m, 2H), 2.05 - 1.89 (m, 2H), 1.87 - 1.72 (m, 2H), 1.31 - 1.22 (m, 3H), 0.92 (td, J = 7.3, 2.4 Hz, 3H).

**Example 23**

**(S)-(4-(((2-Amino-4,5,6,7-tetrahydrobenzo[d]thiazol-6-yl)(propyl)amino)methyl)piperidin-1-yl)(naphthalen-2-yl)methanone and hydrochloride thereof**

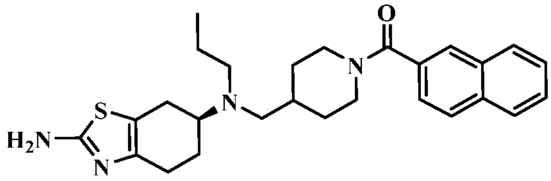

Synthesis scheme of (S)-(4-(((2-amino-4,5,6,7-tetrahydrobenzo[d]thiazol-6-yl)(propyl)amino)methyl)piperidin-1-yl)(naphthalen-2-yl)methanone:

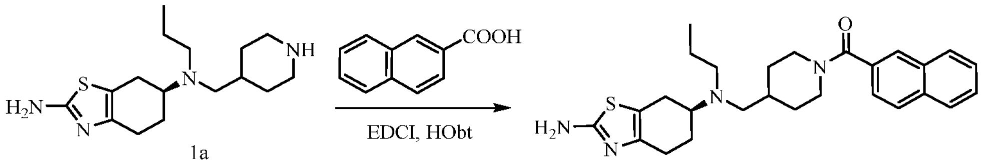


The synthesis method in Example 15 was employed, except that the starting material 3-chloro-4-fluorobenzoic acid was replaced by 2-naphthoic acid to prepare the target product.

ESI-MS$[M+H]^+$: m/z 463.2.

Preparation of hydrochloride: Synthesis of (S)-(4-(((2-amino-4,5,6,7-tetrahydrobenzo[d]thiazol-6-yl)(propyl)amino)methyl)piperidin-1-yl)(naphthalen-2-yl)methanone hydrochloride

(S)-(4-(((2-Amino-4,5,6,7-tetrahydrobenzo[d]thiazol-6-yl)(propyl)amino)methyl)piperidin-1-yl)(naphthalen-2-yl)methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 463.2.

$^1H$ NMR (600 MHz, DMSO-$d_6$) δ 10.45 (s, 1H), 9.27 (s, 2H), 8.00 - 7.98 (m, 3H), 7.95 (d, J = 1.6 Hz, 1H), 7.61 - 7.58 (m, 2H), 7.49 (dd, J = 8.4, 1.7 Hz, 1H), 4.63 - 4.42 (m, 1H), 3.76 - 3.61 (m, 3H), 3.43 - 3.37 (m, 3H), 3.22 - 3.19 (m, 1H), 3.12 - 3.06 (m, 4H), 2.99 - 2.96 (m, 1H), 2.90 - 2.80 (m, 1H), 2.77 - 2.54 (m, 1H), 2.45 - 2.26 (m, 1H), 2.23 - 2.07 (m, 1H), 2.08 - 1.89 (m, 1H), 1.89 - 1.71 (m, 1H), 1.39 - 1.19 (m, 3H), 0.92 (td, J = 7.3, 2.6 Hz, 3H).

**Example 24**

**(S)-(4-(((2-Amino-4,5,6,7-tetrahydrobenzo[d]thiazol-6-yl)(propyl)amino)methyl)piperidin-1-yl)(thiophen-2-yl)methanone and hydrochloride thereof**

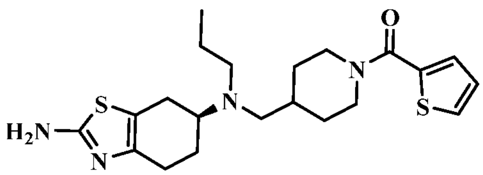

Synthesis scheme of (S)-(4-(((2-amino-4,5,6,7-tetrahydrobenzo[d]thiazol-6-yl)(propyl)amino)methyl)piperidin-1-yl)(thiophen-2-yl)methanone:

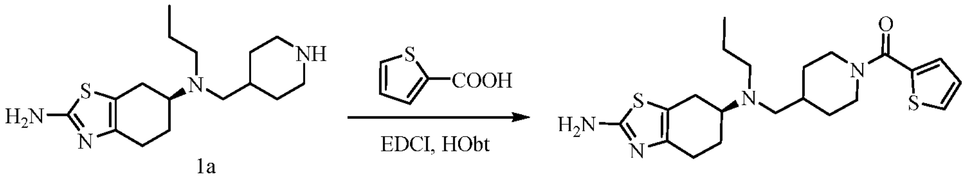


The synthesis method in Example 15 was employed, except that the starting material 3-chloro-4-fluorobenzoic acid was replaced by 2-thiophenecarboxylic acid to prepare the target product.
ESI-MS[M+H]$^+$: m/z 419.1.

Preparation of hydrochloride: Synthesis of (S)-(4-(((2-amino-4,5,6,7-tetrahydrobenzo[d]thiazol-6-yl)(propyl)amino)methyl)piperidin-1-yl)(thiophen-2-yl)methanone hydrochloride

(S)-(4-(((2-Amino-4,5,6,7-tetrahydrobenzo[d]thiazol-6-yl)(propyl)amino)methyl)piperidin-1-yl)(thiophen-2-yl)methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.
ESI-MS[M+H]$^+$: m/z 419.1.
$^1$H NMR (400 MHz, DMSO) δ 9.97 (s, 1H), 8.93(s, 2H), 7.79 (dd, J = 5.0, 1.1 Hz, 1H), 7.41 (dd, J = 3.7, 1.2 Hz, 1H), 7.20 - 7.14 (m, 1H), 4.39 - 4.21 (m, 2H), 3.79 - 3.64 (m, 1H), 3.33 - 3.19 (m, 3H), 3.18 - 3.05 (m, 4H), 3.05 - 2.97 (m, 2H), 2.94 - 2.56 (m, 1H), 2.15 - 2.07 (m, 2H), 1.99 - 1.92 (m, 2H), 1.84 - 1.78 (m, 2H), 1.33 - 1.27 (m, 1H), 1.27 - 1.23 (m, 2H), 0.99 - 0.92 (m, 3H).

## Example 25

**(S)-(4-(((2-Amino-4,5,6,7-tetrahydrobenzo[d]thiazol-6-yl)(propyl)amino)methyl)piperidin-1-yl)(furan-2-yl)methanone and hydrochloride thereof**

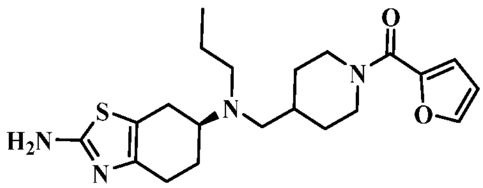

Synthesis scheme of (S)-(4-(((2-amino-4,5,6,7-tetrahydrobenzo[d]thiazol-6-yl)(propyl)amino)methyl)piperidin-1-yl)(furan-2-yl)methanone:

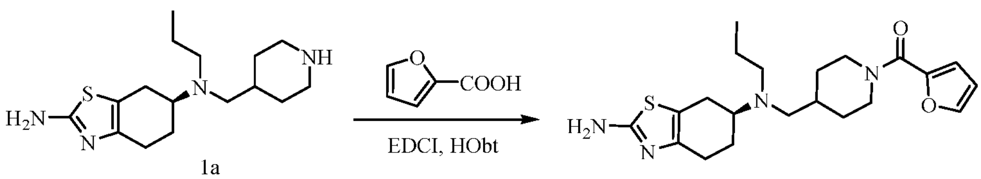


The synthesis method in Example 15 was employed, except that the starting material 3-chloro-4-fluorobenzoic acid was replaced by 2-furancarboxylic acid to prepare the target product.
ESI-MS[M+H]$^+$: m/z 403.0.

Preparation of hydrochloride: Synthesis of (S)-(4-(((2-amino-4,5,6,7-tetrahydrobenzo[d]thiazol-6-yl)(propyl)amino)methyl)piperidin-1-yl)(furan-2-yl)methanone hydrochloride

(S)-(4-(((2-Amino-4,5,6,7-tetrahydrobenzo[d]thiazol-6-yl)(propyl)amino)methyl)piperidin-1-yl)(furan-2-yl)methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 403.0.

$^1H$ NMR (600 MHz, DMSO-$d_6$) δ 9.95 (s, 1H), 7.84 (d, J = 1.6 Hz, 1H), 6.96 (d, J = 3.4 Hz, 1H), 6.63 (dt, J = 3.0, 1.2 Hz, 1H), 4.35 - 4.32 (m, 2H), 3.76 - 3.63 (m, 1H), 3.23 - 3.18 (m, 1H), 3.16 - 3.02 (m, 1H), 3.01 - 2.94 (m, 1H), 2.90 - 2.78 (m, 1H), 2.70 - 2.65 (m, 1H), 2.65 - 2.54 (m, 1H), 2.42 - 2.32 (m, 1H), 2.30 - 2.27 (m, 1H), 2.12 - 2.10 (m, 2H), 2.10 - 2.04 (m, 1H), 1.97 - 1.94 (m, 1H), 1.94 - 1.89 (m, 1H), 1.83 - 1.78 (m, 2H), 1.78 - 1.75 (m, 1H), 1.26 - 1.20 (m, 3H), 0.93 (td, J = 7.3, 2.3 Hz, 3H).

**Example 26**

**(S)-(4-(((2-Amino-4,5,6,7-tetrahydrobenzo[d]thiazol-6-yl)(propyl)amino)methyl)piperidin-1-yl)(1H-pyrrol-2-yl) methanone and hydrochloride thereof**

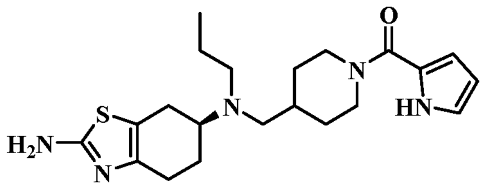

Synthesis scheme of (S)-(4-(((2-amino-4,5,6,7-tetrahydrobenzo[d]thiazol-6-yl)(propyl)amino)methyl)piperidin-1-yl)(1H-pyrrol-2-yl)methanone:

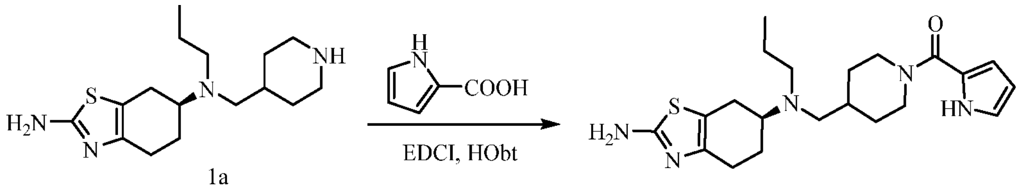


The synthesis method in Example 15 was employed, except that the starting material 3-chloro-4-fluorobenzoic acid was replaced by 2-pyrrolecarboxylic acid to prepare the target product.

ESI-MS$[M+H]^+$: m/z 402.0.

Preparation of hydrochloride: Synthesis of (S)-(4-(((2-amino-4,5,6,7-tetrahydrobenzo[d]thiazol-6-yl)(propyl)amino) methyl)piperidin-1-yl)(1H-pyrrol-2-yl)methanone hydrochloride

(S)-(4-(((2-Amino-4,5,6,7-tetrahydrobenzo[d]thiazol-6-yl)(propyl)amino)methyl) piperidin-1-yl)(1H-pyrrol-2-yl) methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 402.0.

$^1H$ NMR (600 MHz, DMSO-$d_6$) δ 11.41 (s, 1H), 10.11 (s, 1H), 8.86 (s, 2H), 6.90 - 6.85 (m, 1H), 6.48 - 6.44 (m, 1H), 6.14 - 6.10 (m, 1H), 4.45 - 4.40 (m, 2H), 3.71 - 3.67 (m, 1H), 3.24 - 3.17 (m, 1H), 3.14 - 3.06 (m, 2H), 3.01 - 2.94 (m, 2H), 2.91 - 2.79 (m, 1H), 2.71 - 2.65 (m, 1H), 2.64 - 2.53 (m, 1H), 2.39 - 2.29 (m, 1H), 2.13 - 2.05 (m, 2H), 2.02 - 1.90 (m, 2H), 1.88 - 1.70 (m, 3H), 1.27 - 1.19 (m, 2H), 1.19 - 1.14 (m, 1H), 0.93 (td, J = 7.3, 2.2 Hz, 3H).

**Example 27**

**(S)-(4-(((2-Amino-4,5,6,7-tetrahydrobenzo[d]thiazol-6-yl)(propyl)amino)methyl)piperidin-1-yl)(thiazol-5-yl) methanone and hydrochloride thereof**

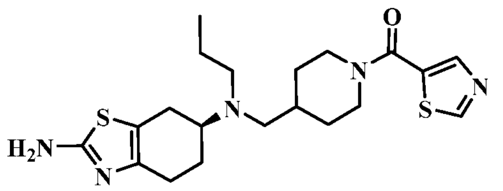

Synthesis scheme of (S)-(4-(((2-amino-4,5,6,7-tetrahydrobenzo[d]thiazol-6-yl)(propyl)amino)methyl)piperidin-1-yl)(thiazol-5-yl)methanone:

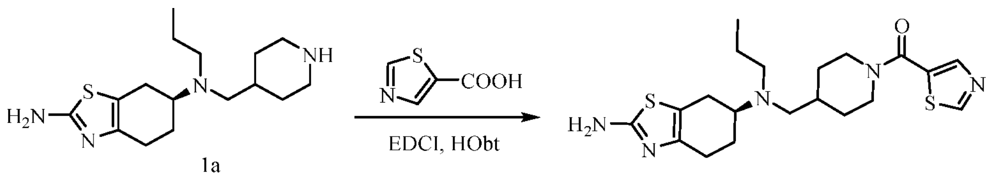


The synthesis method in Example 15 was employed, except that the starting material 3-chloro-4-fluorobenzoic acid was replaced by 5-thiazolecarboxylic acid to prepare the target product.

ESI-MS$[M+H]^+$: m/z 420.1.

Preparation of hydrochloride: Synthesis of (S)-(4-(((2-amino-4,5,6,7-tetrahydrobenzo [d]thiazol-6-yl)(propyl)amino)methyl)piperidin-1-yl)(thiazol-5-yl)methanone hydrochloride

(S)-(4-(((2-Amino-4,5,6,7-tetrahydrobenzo[d]thiazol-6-yl)(propyl)amino)methyl)piperidin-1-yl)(thiazol-5-yl) methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 420.1.

$^1H$ NMR (600 MHz, DMSO-$d_6$) δ 10.49 (s, 1H), 9.45 - 9.38 (m, 2H), 9.24 (s, 1H), 8.17 (s, 1H), 4.43 - 4.40 (m, 1H), 3.46 - 3.42 (m, 1H), 3.23 - 3.17 (m, 1H), 3.16 - 3.03 (m, 3H), 3.01 - 2.93 (m, 1H), 2.93 - 2.80 (m, 1H), 2.74 - 2.67 (m, 1H), 2.66 - 2.58 (m, 1H), 2.42 - 2.34 (m, 1H), 2.21 - 2.10 (m, 2H), 2.05 - 1.88 (m, 2H), 1.88 - 1.75 (m, 2H), 1.30 - 1.20 (m, 5H), 0.92 (t, J = 7.4 Hz, 3H).

**Example 28**

**(S)-(4-(((2-Amino-4,5,6,7-tetrahydrobenzo[d]thiazol-6-yl)(propyl)amino)methyl)piperidin-1-yl)(1-methyl-1H-pyrazol-4-yl)methanone**

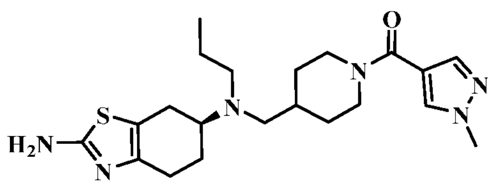

Synthesis scheme of (S)-(4-(((2-amino-4,5,6,7-tetrahydrobenzo[d]thiazol-6-yl)(propyl) amino1-yl)(1-methyl-1H-pyrazol-4-yl)methanone:

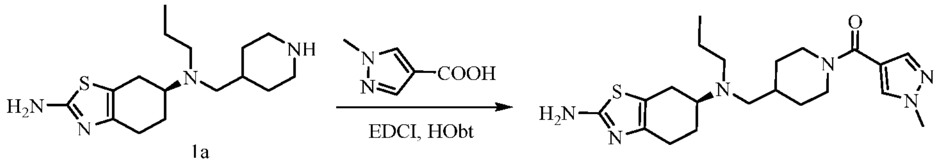


1a

The synthesis method in Example 15 was employed, except that the starting material 3-chloro-4-fluorobenzoic acid was replaced by 1-methylpyrazole-4-carboxylic acid to prepare the target product.
1-Methylpyrazole-4-carboxylic acid (2.7 mmol, 1 eq), EDCI (3.2 mmol, 1.2 eq), HObt (3.2 mmol, 1.2 eq), triethylamine (5.4 mmol, 2 eq), and intermediate 1a (3.2 mmol, 1.2 eq) were dissolved in DMF, and the mixture was allowed to react at -10 °C for 8 h. After the reaction was completed, 20 mL of water was added, and the mixture was extracted with dichloromethane (30 mL × 3). The organic layer was washed with water and saturated brine, dried over $MgSO_4$, and then concentrated, and the residue was purified by column chromatography to obtain the target product.
ESI-MS[M+H]$^+$: m/z 417.1.
$^1$H NMR (600 MHz, $CDCl_3$) δ 7.58 (s, 1H), 7.50 (s, 1H), 4.53 (s, 2H), 3.82 (s, 3H), 3.79 - 3.67 (m, 1H), 2.93 - 2.85 (m, 1H), 2.64 - 2.57 (m, 1H), 2.55 - 2.50 (m, 1H), 2.50 - 2.37 (m, 2H), 2.37 - 2.30 (m, 2H), 2.29 - 2.24 (m, 1H), 2.24 - 2.16 (m, 1H), 1.87 - 1.82 (m, 1H), 1.81 - 1.73 (m, 2H), 1.62 - 1.52 (m, 2H), 1.37 - 1.28 (m, 3H), 1.16 (s, 2H), 1.02 - 0.99 (m, 2H), 0.78 (t, J = 7.3 Hz, 3H).

## Example 29

**(S)-(4-(((2-Amino-4,5,6,7-tetrahydrobenzo[d]thiazol-6-yl)(propyl)amino)methyl)piperidin-1-yl)(cyclohexyl) methanone and hydrochloride thereof**

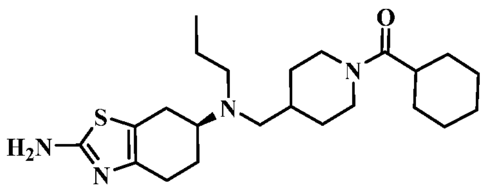

Synthesis scheme of (S)-(4-(((2-amino-4,5,6,7-tetrahydrobenzo[d]thiazol-6-yl)(propyl) amino)methyl)piperidin-1-yl)(cyclohexyl)methanone:

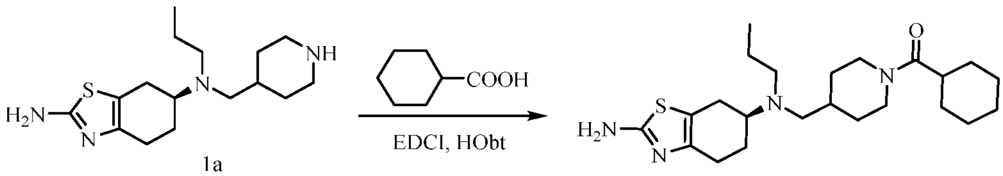


1a

The synthesis method in Example 15 was employed, except that the starting material 3-chloro-4-fluorobenzoic acid was replaced by cyclohexanecarboxylic acid to prepare the target product.
ESI-MS[M+H]$^+$: m/z 419.2.

Preparation of hydrochloride: Synthesis of (S)-(4-(((2-amino-4,5,6,7-tetrahydrobenzo [d]thiazol-6-yl)(propyl)amino) methyl)piperidin-1-yl)(cyclohexyl)methanone hydrochloride

(S)-(4-(((2-Amino-4,5,6,7-tetrahydrobenzo[d]thiazol-6-yl)(propyl)amino)methyl)piperidin-1-yl)(cyclohexyl)methanone was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 419.2.

$^1H$ NMR (600 MHz, DMSO-$d_6$) δ 10.45 (s, 1H), 9.30 (s, 2H), 4.47 - 4.30 (m, 1H), 4.03 - 3.85 (m, 1H), 3.58 - 3.44 (m, 5H), 3.18 - 3.15 (m, 1H), 3.12 - 3.03 (m, 2H), 3.02 - 2.76 (m, 1H), 2.72 - 2.66 (m, 1H), 2.65 - 2.53 (m, 1H), 2.49 - 2.41 (m, 1H), 2.38 - 2.32 (m, 1H), 2.19 - 2.11 (m, 1H), 2.08 - 1.96 (m, 1H), 1.88 - 1.79 (m, 2H), 1.74 - 1.63 (m, 2H), 1.63 - 1.58 (m, 3H), 1.38 - 1.23 (m, 5H), 1.20 - 1.07 (m, 3H), 1.03 - 0.99 (m, 1H), 0.91 (td, J = 7.3, 2.2 Hz, 3H).

### Example 30

**(S)-$N^6$-((1-(Phenylsulfonyl)piperidin-4-yl)methyl)-$N^6$-propyl-4,5,6,7-tetrahydrobenzo[d]thiazole-2,6-diamine and hydrochloride thereof**

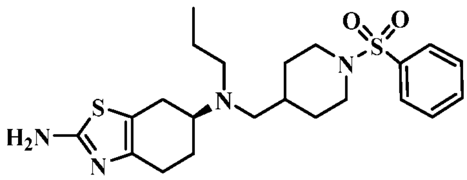

Synthesis scheme of (S)-$N^6$-((1-(phenylsulfonyl)piperidin-4-yl)methyl)-$N^6$-propyl-4,5,6,7-tetrahydrobenzo[d]thiazole-2,6-diamine:

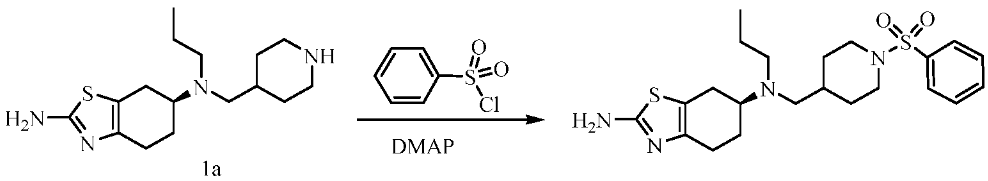


Intermediate **1a** (0.65 mmol, 1 eq) was taken and dissolved in DCM (6 mL), and triethylamine (1.95 mmol, 3 eq), DMAP (2 mg, catalytic amount), and benzenesulfonyl chloride (0.65 mmol, 1 eq) were added under an ice bath. The mixture was stirred for 2 h. After the reaction was completed, 10 mL of water was added, and the mixture was extracted with dichloromethane (20 mL × 3). The organic layer was washed with water and saturated brine, dried over $MgSO_4$, and then concentrated, and the residue was purified by column chromatography to obtain the target product.

ESI-MS$[M+H]^+$: m/z 449.0.

Preparation of hydrochloride: Synthesis of (S)-$N^6$-((1-(phenylsulfonyl)piperidin-4-yl)methyl)-$N^6$-propyl-4,5,6,7-tetrahydrobenzo[d]thiazole-2,6-diamine hydrochloride

(S)-$N^6$-((1-(Phenylsulfonyl)piperidin-4-yl)methyl)-$N^6$-propyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.

ESI-MS$[M+H]^+$: m/z 449.0.

$^1H$ NMR (600 MHz, DMSO-$d_6$) δ 10.22 (s, 1H), 9.00 (s, 2H), 7.77 - 7.71 (m, 3H), 7.66 (t, J = 7.6 Hz, 2H), 3.71 - 3.58 (m, 3H), 3.18 - 3.09 (m, 1H), 3.05 - 2.99 (m, 3H), 2.93 - 2.88 (m, 1H), 2.85 - 2.75 (m, 1H), 2.69 - 2.53 (m, 1H), 2.34 - 2.25 (m, 1H), 2.20 - 2.13 (m, 1H), 2.16 - 2.05 (m, 1H), 1.96 - 1.89 (m, 1H), 1.82 - 1.63 (m, 4H), 1.33 - 1.19 (m, 4H), 0.87 (td, J = 7.5, 2.1 Hz, 3H).

### Example 31

**(S)-N$^6$-((1-((3-Chloro-4-fluorophenyl)sulfonyl)piperidin-4-yl)methyl)-N$^6$-propyl-4,5,6,7-tetrahydrobenzo[d]thiazole-2,6-diamine and hydrochloride thereof**

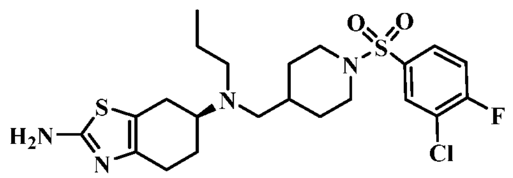

Synthesis scheme of (S)-N$^6$-((1-((3-chloro-4-fluorophenyl)sulfonyl)piperidin-4-yl)methyl)-N$^6$-propyl-4,5,6,7-tetrahydrobenzo[d]thiazole-2,6-diamine:

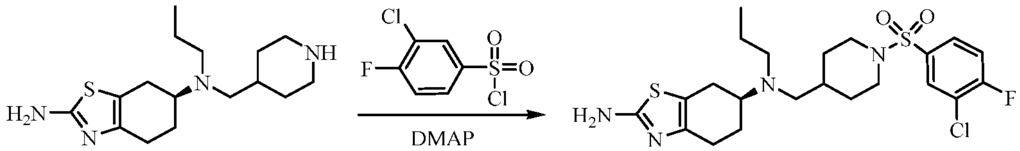


1a

The synthesis method in Example 30 was employed, except that the starting material benzenesulfonyl chloride was replaced by 3-chloro-4-fluorobenzenesulfonyl chloride to prepare the target product.
ESI-MS[M+H]$^+$: m/z 501.0.

Preparation of hydrochloride: Synthesis of (S)-N$^6$-((1-((3-chloro-4-fluorophenyl) sulfonyl)piperidin-4-yl)methyl)-N$^6$-propyl-4,5,6,7-tetrahydrobenzo[d]thiazole-2,6-diamine hydrochloride

(S)-N$^6$-((1-((3-Chloro-4-fluorophenyl)sulfonyl)piperidin-4-yl)methyl)-N$^6$-propyl-4,5,6,7-tetrahydrobenzo[d]thiazole-2,6-diamine was taken and dissolved in ethyl acetate (5-10 mL), and HCl EA (2 M) was added dropwise to adjust the pH to below 3, resulting in the precipitation of a solid. The mixture was stirred at room temperature for 1-5 h, and subjected to suction filtration to obtain the hydrochloride of the compound.
ESI-MS[M+H]$^+$: m/z 501.0.
$^1$H NMR (600 MHz, DMSO-$d_6$) δ 10.10 (s, 1H), 8.56 (s, 2H), 8.00 - 7.95 (m, 1H), 7.82 - 7.77 (m, 1H), 7.72 (t, J = 8.8 Hz, 1H), 3.70 - 3.60 (m, 4H), 3.17 - 3.11 (m, 1H), 3.10 - 2.95 (m, 2H), 2.95 - 2.89 (m, 1H), 2.85 - 2.75 (m, 1H), 2.69 - 2.51 (m, 1H), 2.37 - 2.15 (m, 3H), 2.14 - 2.03 (m, 1H), 1.98 - 1.84 (m, 2H), 1.81 - 1.78 (m, 2H), 1.75 - 1.71 (m, 2H), 1.29 - 1.24 (m, 2H), 0.88 (td, J = 7.3, 2.3 Hz, 3H).

**Example 32**

**(S)-4-(((2-Amino-4,5,6,7-tetrahydrobenzo[d]thiazol-6-yl)(propyl)amino)methyl)-N,N-dimethylpiperidine-1-carboxamide**

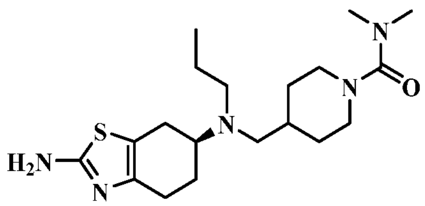

Synthesis scheme of (S)-4-(((2-amino-4,5,6,7-tetrahydrobenzo[d]thiazol-6-yl)(propyl) amino)methyl)-N,N-dimethylpiperidine-1-carboxamide:

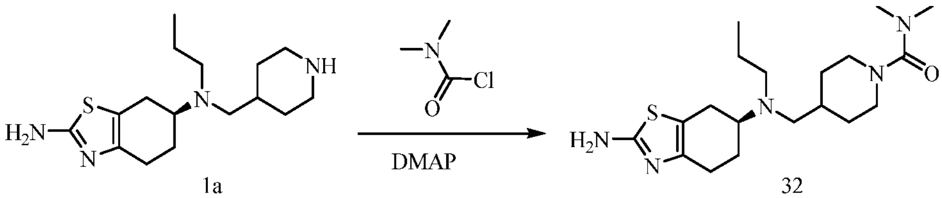


1a 32

The synthesis method in Example 30 was employed, except that the starting material benzenesulfonyl chloride was replaced by dimethylcarbamoyl chloride to prepare the target product.

ESI-MS$[M+H]^+$: m/z 380.2.

$^1$H NMR (600 MHz, $CDCl_3$) δ 4.80 (s, 2H), 3.60 - 3.54 (m, 2H), 2.92 - 2.84 (m, 1H), 2.71 (s, 6H), 2.66 - 2.56 (m, 2H), 2.55 - 2.49 (m, 1H), 2.44 - 2.37 (m, 1H), 2.37 - 2.27 (m, 2H), 2.27 - 2.21 (m, 1H), 2.21 - 2.15 (m, 1H), 1.88 - 1.81 (m, 1H), 1.77 - 1.74 (m, 1H), 1.72 - 1.66 (m, 1H), 1.61 - 1.51 (m, 1H), 1.48 - 1.37 (m, 1H), 1.36 - 1.27 (m, 2H), 1.25 - 1.09 (m, 2H), 1.03 - 0.92 (m, 2H), 0.78 (t, J = 7.3 Hz, 3H).

**Example 33**

**(S)-(4-(((2-Amino-4,5,6,7-tetrahydrobenzo[d]thiazol-6-yl)(propyl)amino)methyl)piperidin-1-yl)(morpholino) methanone**

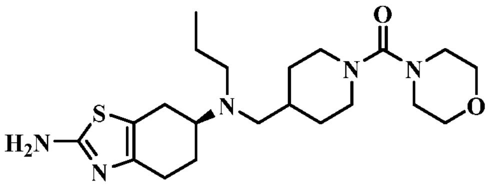

Synthesis scheme of (S)-(4-(((2-amino-4,5,6,7-tetrahydrobenzo[d]thiazol-6-yl)(propyl) amino)methyl)piperidin-1-yl) (morpholino)methanone:

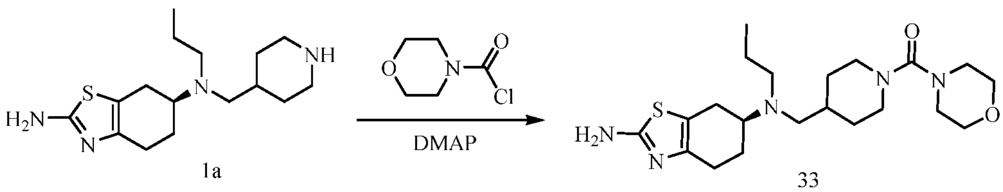


1a 33

The synthesis method in Example 30 was employed, except that the starting material benzenesulfonyl chloride was replaced by 4-morpholinecarbonyl chloride to prepare the target product.

ESI-MS$[M+H]^+$: m/z 422.1.

$^1$H NMR (600 MHz, $CDCl_3$) δ 4.86 (s, 2H), 3.67 - 3.53 (m, 8H), 3.16 - 3.13 (m, 4H), 2.91 - 2.83 (m, 1H), 2.68 - 2.62 (m, 2H), 2.62 - 2.55 (m, 1H), 2.54 - 2.49 (m, 1H), 2.49 - 2.37 (m, 1H), 2.37 - 2.27 (m, 2H), 2.27 - 2.22 (m, 1H), 2.21 - 2.15 (m, 1H), 1.87 - 1.81 (m, 1H), 1.74 - 1.66 (m, 2H), 1.61 - 1.51 (m, 1H), 1.36 - 1.26 (m, 2H), 1.03 - 0.91 (m, 2H), 0.78 (t, J = 7.3 Hz, 3H).

**Comparative Example 1**

**(S)-N⁶-(2-(4-(2,3-Dichlorophenyl)piperazin-1-yl)ethyl)-N⁶-propyl-4,5,6,7-tetrahydrobenzo[d]thiazole-2,6-diamine hydrochloride**

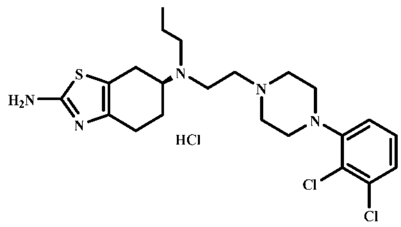

Synthesis scheme:

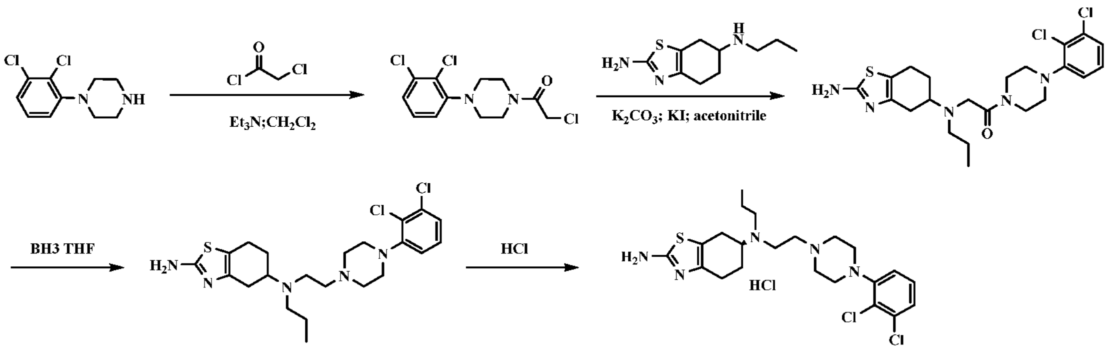


Referring to the literature (Biswas S. J Med Chem. 2008;51(10):3005-3019.), Comparative Example 1 was prepared according to the route described above.

The following Comparative Examples 2 and 3 were prepared by referring to Example 30 or Example 19 of patent JP2005104885A.

**Comparative Example 2**

**Methyl 4-(((2-amino-4,5,6,7-tetrahydrobenzo[d]thiazol-6-yl)(propyl)amino)methyl)piperidine-1-carboxylate hydrochloride**

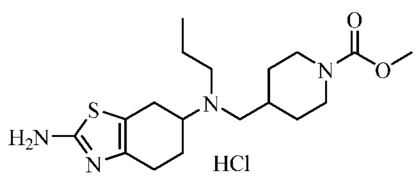

**Comparative Example 3**

**Methyl (R)-4-(((2-amino-4,5,6,7-tetrahydrobenzo[d]thiazol-6-yl)(propyl)amino)methyl)piperidine-1-carboxylate hydrochloride**

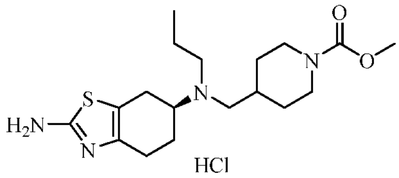

**Biological Assay Evaluation**

The present invention is further described below using test examples, but these examples are not intended to limit the scope of the present invention.

**Test Example *1. In Vitro* $5HT_{1A}$ Receptor Function Experiment for Compounds of the Present Invention**

1.1 Experimental materials

1.1.1 Cell information: HEK293/$5HT_{1A}$: a stably transfected cell strain HEK293/$5HT_{1A}$ constructed by adopting $5HT_{1A}$ receptor lentivirus to infect HEK293; growth culture medium: DMEM, 10% FBS, and 0.6 μg/mL puro; and cryopreservation culture medium: 70% culture medium, 20% FBS, and 10% DMSO.

1.1.2 Experimental reagent consumables

| Reagent | Supplier | Model |
|---|---|---|
| 5HT | Sigma | H9523 |
| Forskolin | Sigma | F6886 |
| IBMX | Sigma | I5879 |
| cAMP Gi Dynamic kits | Cisbio | 62AM9PEB |
| HBSS | Gibco | 14175095 |
| DMSO | Sigma | D8418 |
| ProxiPlate-384 Plus | PerkinELmer | 6008208 |

1.1.3 Experimental instruments

| Name | Supplier | Model |
|---|---|---|
| Cell counter | Countstar | BioTech |
| EnVision multimode plate reader | PerkinElmer | 2105 |
| Milli-Q ultrapure water instrument | Millipore | IQ 7000 |
| Centrifuge | Cence | TDZ5-WS |
| Picoliter micropipette | Tecan | D300e |

1.2 Experimental procedures

(1) Compound preparation: The test compounds and 5HT were diluted with stimulation buffer to a concentration of 0.1 mM for later use.
(2) Experimental buffer preparation: The 5× stimulation buffer was diluted to 1× with $ddH_2O$, and IBMX was added to

a final concentration of 0.5 mM. The mixture was mixed well for later use.

(3) The cells were digested with trypsin, and centrifugation was performed after the digestion was terminated. The cell pellet was resuspended in 10 mL of pre-warmed HBSS and centrifuged again. Then, 1 mL of stimulation buffer was added for resuspension, and 20 μL of the cell suspension was taken for cell counting.

(4) An appropriate amount of the cell suspension was taken and diluted to $0.6 \times 10^6$ cells/mL, and 5 μL of the diluted cell suspension was added to each well of a cell plate and centrifuged at 1000 rpm for 1 min.

(5) Compound dilution and transfer using bravo: The compounds were diluted 4-fold to obtain 8 concentration points in the compound plate. The diluted compounds in the whole plate were then transferred to a 384-well white plate using bravo and centrifuged at 1000 rpm for 1 min.

(6) The experimental plate was sealed and incubated at room temperature for 15 min.

(7) Forskolin, prepared with DMSO, was added using Tecan D300e, with a mother liquor concentration of 0.2 mM and a final concentration of 1 μM, followed by centrifugation at 1000 rpm for 1 min.

(8) The plate was sealed and incubated at room temperature for 45 min.

(9) cAMP standard curve preparation: A 4-fold serial dilution was performed to obtain 8 concentration points from a starting concentration of 2848 nM. Then, 10 μL of each dilution was taken and added to the experimental plate, with a maximum concentration of 712 nM.

(10) 5 μL of cAMP-d2 solution (stock solution diluted at 1:20 with lysis buffer) was added to the experimental plate, and the plate was centrifuged at 1000 rpm for 1 min.

(11) Then, 5 μL of Anti-cAMP-Cryptate solution (stock solution diluted at 1:20 with lysis buffer) was added to the experimental plate, and the plate was centrifuged at 1000 rpm for 1 min.

(12) The experimental plate was incubated at room temperature for 1 h and centrifuged at 1000 rpm for 1 min before reading.

(13) Plate reading was performed using Envision, with excitation light at 320 nm and emission lights at 620 nm and 665 nm. The derived data were analyzed, and the maximum activation rates $E_{max}$ and $EC_{50}$ of the compounds were calculated.

1.3 Experimental results: See Table 1.

Table 1. Results of 5-$HT_{1A}$ receptor function experiment for compounds of the present invention

| Example No. | 5-$HT_{1A}$ | |
|---|---|---|
| | $EC_{50}$ (nM) | $E_{max}$ (%) |
| 9 | 30.22 | 100.94 |
| 11 | 130.50 | 92.64 |
| 12 | 31.43 | 115.13 |
| 13 | 35.94 | 117.65 |
| 15 | 4.70 | 85.08 |
| 16 | 142.90 | 93.67 |
| 17 | 51.60 | 113.80 |
| 18 | 48.02 | 115.17 |
| 19 | 184.50 | 99.51 |
| 20 | 131.00 | 115.25 |
| 22 | 99.27 | 114.81 |
| 23 | 202.40 | 101.28 |
| 24 | 75.70 | 96.54 |
| 25 | 41.93 | 112.47 |
| 26 | 16.39 | 103.01 |
| 27 | 87.33 | 99.89 |
| 28[a] | 121.60 | 102.58 |

(continued)

| Example No. | 5-$HT_{1A}$ | |
|---|---|---|
| | $EC_{50}$ (nM) | $E_{max}$ (%) |
| 29 | 107.00 | 118.45 |
| 30 | 39.95 | 124.60 |
| 31 | 124.00 | 119.96 |
| 32[a] | 25.75 | 102.33 |
| 33[a] | 162.40 | 92.71 |
| Comparative Example 1 | 203.50 | 99.13 |
| Comparative Example 2 | 246.50 | 95.41 |
| Comparative Example 3 | 203.30 | 97.05 |
| Note: In the above table, a represents the free base form of the compound; and the others represent the hydrochloride/dihydrochloride salt form of the compound. | | |

1.4 Experimental conclusion:

As can be seen from the results in Table 1 above, the compounds of the present invention can act on 5-$HT_{1A}$ receptors and show good 5-$HT_{1A}$ receptor agonistic activity.

**Test Example 2. *In Vitro* Dopamine $D_{2L}$ Receptor Function Experiment for Compounds of the Present Invention**

2.1 Experimental materials

2.1.1 Cell information

| Cell | Supplier |
|---|---|
| HEK293/$D_{2L}$ | GenScript Biotech Co., Ltd. |

2.1.2 Experimental reagent consumables

| Reagent | Supplier | Model |
|---|---|---|
| Forskolin | Sigma | F6886 |
| IBMX | Sigma | I5879 |
| Dopamine | Sigma | H8502 |
| cAMP Gi Dynamic kits | Cisbio | 62AM9PEB |
| HBSS | Gibco | 14175095 |
| DMSO | Sigma | D8418 |
| ProxiPlate-384 Plus | PerkinELmer | 6008208 |

2.1.3 Experimental instruments

| Name | Supplier | Model |
|---|---|---|
| Cell counter | Countstar | BioTech |
| EnVision multimode plate reader | PerkinElmer | 2105 |
| Milli-Q ultrapure water instrument | Millipore | IQ 7000 |
| Centrifuge | Cence | TDZ5-WS |
| Picoliter micropipette | Tecan | D300e |

2.2 Experimental procedures

(1) Compound preparation: The test compounds and dopamine were diluted to a concentration of 200 nM for later use.
(2) Experimental buffer preparation: The 5× stimulation buffer was diluted to 1× with $ddH_2O$, and IBMX was added to a final concentration of 0.5 mM. The mixture was mixed well for later use.
(3) The cells were digested with trypsin, and centrifugation was performed after the digestion was terminated. The cell pellet was resuspended in 10 mL of pre-warmed HBSS and centrifuged again. Then, 1 mL of stimulation buffer was added for resuspension, and 20 μL of the cell suspension was taken for cell counting.
(4) An appropriate amount of the cell suspension was taken and diluted to $0.4 \times 10^6$ cells/mL, and 5 μL of the diluted cell suspension was added to each well of a cell plate and centrifuged at 1000 rpm for 1 min.
(5) Compound dilution and transfer using bravo: The compounds were diluted 4-fold to obtain 8 concentration points in the compound plate. The diluted compounds in the whole plate were then transferred to a 384-well white plate using bravo and centrifuged at 1000 rpm for 1 min.
(6) The experimental plate was sealed and incubated at room temperature for 15 min.
(7) Forskolin, prepared with DMSO, was added at 25.1 nL/well using Tecan D300e, with a mother liquor concentration of 0.2 mM and a final concentration of 0.25 μM, followed by centrifugation at 1000 rpm for 1 min.
(8) The plate was sealed and incubated at room temperature for 45 min.
(9) cAMP standard curve preparation: A 4-fold serial dilution was performed to obtain 8 concentration points from a starting concentration of 2848 nM. Then, 10 μL of each dilution was taken and added to the experimental plate, with a maximum concentration of 712 nM.
(10) 5 μL of cAMP-d2 solution (stock solution diluted at 1:20 with lysis buffer) was added to the experimental plate, and the plate was centrifuged at 1000 rpm for 1 min.
(11) Then, 5 μL of Anti-cAMP-Cryptate solution (stock solution diluted at 1:20 with lysis buffer) was added to the experimental plate, and the plate was centrifuged at 1000 rpm for 1 min.
(12) The experimental plate was incubated at room temperature for 45 min and centrifuged at 1000 rpm for 1 min before reading.
(13) Plate reading was performed using Envision, with excitation light at 320 nm and emission lights at 620 nm and 665 nm. The derived data were analyzed, and the maximum activation rates $E_{max}$ and $EC_{50}$ of the compounds were calculated.

2.3 Experimental results: See Table 2.

Table 2. Results of $D_2$ receptor function experiment for compounds of the present invention

| Example No. | D2L | |
|---|---|---|
| | $EC_{50}$ (nM) | $E_{max}$ (%) |
| 15 | 0.06 | 99.9 |
| 16 | 0.38 | 115.1 |
| 18 | 0.37 | 101.7 |
| 19 | 0.15 | 102.1 |
| 20 | 0.28 | 103.0 |

(continued)

| Example No. | D2L | |
|---|---|---|
| | $EC_{50}$ (nM) | $E_{max}$ (%) |
| 22 | 0.07 | 103.2 |
| 23 | 0.07 | 101.7 |
| 24 | 0.16 | 103.0 |
| 27 | 0.19 | 101.4 |
| 28[a] | 0.22 | 100.5 |
| 30 | 0.23 | 101.3 |
| Comparative Example 1 | 0.25 | 100.7 |
| Comparative Example 2 | 1.68 | 112.3 |
| Comparative Example 3 | 0.95 | 112.2 |
| Note: In the above table, a represents the free base form of the compound; and the others represent the hydrochloride/dihydrochloride salt form of the compound. | | |

2.4 Experimental conclusion:

As can be seen from the results in Table 2 above, the compounds of the present invention can act on dopamine $D_2$ receptors and show good dopamine $D_2$ receptor (partial) agonistic activity.

**Test Example *3. In Vitro* Dopamine $D_3$ Receptor Function Experiment for Compounds of the Present Invention**

3.1 Experimental materials

3.1.1 Cell information: CHO-K1/D3/CRE constructed by referring to the method in Example 5 of CN114369578A.

3.1.2 Experimental reagent consumables

| Reagent | Supplier | Model |
|---|---|---|
| Forskolin | Sigma | F6886 |
| IBMX | Sigma | I5879 |
| Dopamine | Sigma | H8502 |
| cAMP Gi Dynamic kits | Cisbio | 62AM9PEB |
| HBSS | Gibco | 14175095 |
| DMSO | Sigma | D8418 |
| ProxiPlate-384 Plus | PerkinELmer | 6008208 |

3.1.3 Experimental instruments

| Name | Supplier | Model |
|---|---|---|
| Cell counter | Countstar | BioTech |
| EnVision multimode plate reader | PerkinElmer | 2105 |
| Milli-Q ultrapure water instrument | Millipore | IQ 7000 |

(continued)

| Name | Supplier | Model |
|---|---|---|
| Centrifuge | Cence | TDZ5-WS |
| Picoliter micropipette | Tecan | D300e |

3.2 Experimental procedures

The first day: cell plating

(1) The cultured cells were digested with trypsin, and after the digestion was terminated with culture medium, the cell suspension was transferred to a centrifuge tube and centrifuged at 750 rpm for 5 min.
(2) The supernatant was discarded, and the pellet was resuspended in an appropriate amount of plating medium. Then, 20 μL of the cell suspension was taken and counted using a cell counter.
(3) An appropriate amount of the cell suspension was taken and diluted to $0.5 \times 10^6$ cells/mL, and 20 μL of the diluted cell suspension was added to each well of a cell plate (the cell density was 10000 cells/well).
(4) The cell plate was incubated at 37 °C with 5% $CO_2$ overnight.

The following day: experimental detection

(1) The test compounds were diluted to 0.2 mM with DMSO, and the reference compound dopamine was diluted to 0.02 mM.

(2) Compound dilution (4-fold dilution at 8 concentration points) was performed using Bravo, with the starting concentrations of the test compounds and the reference compound being 5 μM and 500 nM, respectively. Then, 5 μL of each dilution was taken and added to a cell plate, with the final reaction concentrations of the test compounds and the reference compound being 1 μM and 100 nM, respectively. The positive control well contained 100 nM dopamine, and the negative control well contained an equal volume of DMSO.

(3) The plate was centrifuged at 1000 rpm for 1 min and incubated at 37 °C with 5% $CO_2$ for 30 min.

(4) 40 nL of 0.2 mM forskolin DMSO solution was transferred to the cell plate using Tecan-D300e, with a final concentration of 0.4 μM. The blank group had cells added without forskolin.

(5) The plate was centrifuged at 1000 rpm for 1 min and incubated at 37 °C with 5% $CO_2$ for 4 h.

(6) The lysis solution was taken out 30 min in advance, thawed in a water bath at normal atmospheric temperature, and returned to room temperature. An appropriate amount of the substrate was taken and diluted with the lysis solution at a 1:50 ratio, and the dilution was mixed well for later use.

(7) 20 μL of detection reagent was added, and the plate was centrifuged at 1000 rpm for 1 min.

(8) After incubation at room temperature for 3 min, plate reading was performed using Envision. The program selected was an ultrasensitive chemiluminescence detection program. The derived data were analyzed, and the maximum activation rates $E_{max}$ and $EC_{50}$ of the compounds were calculated.

3.3 Experimental results: See Table 3.

Table 3. Results of $D_3$ receptor function experiment for compounds of the present invention

| Example No. | $D_3$ | |
|---|---|---|
| | $EC_{50}$ (nM) | $E_{max}$ (%) |
| 7 | 2.9 | 121.5 |
| 9 | 2.1 | 110.7 |

(continued)

| Example No. | $D_3$ | |
|---|---|---|
| | $EC_{50}$ (nM) | $E_{max}$ (%) |
| 11 | 3.3 | 109.2 |
| 13 | 3.0 | 133.1 |
| 15 | 1.4 | 117.1 |
| 16 | 0.9 | 120.0 |
| 17 | 1.1 | 109.0 |
| 18 | 2.1 | 104.6 |
| 19 | 2.4 | 108.9 |
| 20 | 2.2 | 109.4 |
| 21 | 1.3 | 105.1 |
| 22 | 1.9 | 110.0 |
| 23 | 1.4 | 99.3 |
| 24 | 1.5 | 103.4 |
| 25 | 1.3 | 106.1 |
| 26 | 1.2 | 109.4 |
| 27 | 0.2 | 112.9 |
| 28[a] | 2.9 | 102.0 |
| 29 | 2.8 | 110.8 |
| 30 | 2.1 | 108.8 |
| 31 | 3.6 | 102.7 |
| 32[a] | 1.3 | 120.1 |
| 33[a] | 1.1 | 106.8 |
| Note: In the above table, a represents the free base form of the compound; and the others represent the hydrochloride/dihydrochloride salt form of the compound. | | |

3.4 Experimental conclusion:

As can be seen from the results in Table 3 above, the compounds of the present invention can act on dopamine $D_3$ receptors and show good dopamine $D_3$ receptor (partial) agonistic activity.

**Test Example 4. *In Vitro* Liver Microsome Experiment for Compounds of the Present Invention**

4.1 Experimental objective

This experiment aimed to evaluate the phase I metabolic stability of the compounds of the present invention in CD-1 mouse, SD rat, and human liver microsomes.

4.2 Experimental materials

4.2.1 Experimental reagents

| Reagent | Supplier | Model |
|---|---|---|
| Dipotassium hydrogen phosphate trihydrate | Greagent | 01031670 |

(continued)

| Reagent | Supplier | Model |
|---|---|---|
| Potassium dihydrogen phosphate | Greagent | 01115129 |
| Phosphoric acid | Greagent | 01113527 |
| Testosterone | Dr.E | 01279087 |
| Dimethyl sulfoxide | Sigma | D8418 |
| Buspirone | TRC | B689850 |
| Acetonitrile | Fisher | A9984 |
| CD-1 mouse liver microsome | RILD | LQDL LM-XS-02M |
| SD rat liver microsome | RILD | DMXD LM-DS-02M |
| Human liver microsome | Corning | 452117 |
| NADP | Aladdin | N113163 |
| G6P | Shanghai Yuanye | S11024 |
| $MgCl_2$ | Greagent | 01115966 |
| G6PDH | Shanghai Yuanye | S10078 |

4.2.2 Experimental instruments

| Name | Supplier | Model |
|---|---|---|
| Liquid chromatograph | SCIEX | Exion LC$^{tm}$ |
| Mass spectrometer | SCIEX | Triple Qvad 5500 |
| Centrifuge | Thermo Fisher | 75009915 |
| Shaker | Thermo Scientific | 88882006 |

4.3 Experimental procedures

(1) Buffer preparation: 73.21 g of dipotassium hydrogen phosphate trihydrate and 10.78 g of potassium dihydrogen phosphate were dissolved in 4000 mL of ultrapure water. The pH of the solution was adjusted to within 7.40 ± 0.10 using 10% phosphoric acid or 1 M potassium hydroxide, resulting in a final concentration of 100 mM.
(2) Solution preparation and dilution: Stock solutions of the test compounds at a concentration of 10 mM were prepared in dimethyl sulfoxide (DMSO) and stored at 4 °C, and they were diluted into 100 μM working solutions with pure acetonitrile when used. A stock solution of the control compound testosterone at a concentration of 10 mM was prepared in DMSO and stored at -20 °C, and it was diluted into a 400 μM working solution with pure acetonitrile when used.
(3) Termination solution preparation: The termination solution was acetonitrile containing the internal standard buspirone. The prepared termination solution was stored in a refrigerator at 2-8 °C.
(4) Liver microsome solution preparation: Microsomes from various species (CD-1 mouse, SD rat, and human) were diluted into a 20× working solution using 100 mM potassium phosphate buffer. The final concentration of the microsomes in the reaction system was 0.5 mg/mL.
(5) Reduced nicotinamide adenine dinucleotide phosphate (NADPH) regeneration system preparation: Appropriate amounts of nicotinamide adenine dinucleotide phosphate (NADP), glucose-6-phosphate (G6P), magnesium chloride ($MgCl_2$), and glucose-6-phosphate dehydrogenase (G6PDH) were weighed to prepare stock solutions at concentrations of 65.33 mM, 330 mM, 300 mM, and 250 Units/mL, respectively. The four stock solutions described above were each added to an appropriate amount of buffer, and the mixture was mixed well by gently inverting up and down. The final concentrations of NADP, G6P, $MgCl_2$, and G6PDH in the NADPH regeneration system were 2.65 mM, 10.2

mM, 6.12 mM, and 2.45 Units/mL, respectively.

(6) Incubation process: The incubation was completed in 96-well plates. Several incubation plates were prepared and named T0, T5, T10, T20, T40, T60, PB60, and NCF60, respectively. The first 6 plates corresponded to reaction time points of 0, 5, 10, 20, 40, and 60 min, respectively. The NCF60 plate was incubated for 60 min with potassium phosphate buffer replacing the NADPH regeneration system solution. The PB60 plate was incubated for 60 min with potassium phosphate buffer replacing the liver microsomes. Samples under all conditions were prepared in triplicate.

2 μL of the test compound or control compound and 100 μL of microsome working solution (containing 1 mg/mL liver microsome protein) were added to each of the T0, T5, T10, T20, T40, T60, and NCF60 plates, and 2 μL of the test compound and 100 μL of potassium phosphate buffer were added to the PB60 plate. The plates described above were then pre-incubated in a 37 °C water bath for about 5 min.

After the pre-incubation, 600 μL of the termination solution was first added to the T0 samples, followed by the addition of 98 μL of NADPH regeneration system working solution. The plate was sealed, shaken, and then allowed to wait for simultaneous processing with subsequent samples. Except for the NCF60 plate where 98 μL of potassium phosphate buffer was added to each sample well, 98 μL of NADPH regeneration system working solution was added to each sample well of the other incubation plates to initiate the reaction. In the reaction system, the final concentrations of the test compound and the control compound were 1 μM and 4 μM, respectively, the concentration of the liver microsomes was 0.5 mg/mL, and the final concentrations of DMSO and acetonitrile were 0.01% (v/v) and 0.99% (v/v), respectively.

After incubation for an appropriate time (such as 5, 10, 20, 40, and 60 min), 600 μL of the termination solution containing the internal standard was added to each test compound sample and control compound well to terminate the reaction. The plates were sealed, shaken well, and then centrifuged at 4000 ×g and 4 °C for 15 min. The supernatant was taken and transferred to a 96-well sample reception plate, diluted with an appropriate amount of pure water, shaken well, and used for LC-MS/MS analysis. Data processing was performed using the software Analyst 7.1 (Sciex, Framingham, Massachusetts, USA).

4.4 Experimental results: See Table 4.

Table 4. Results of *in vitro* liver microsome stability test

| Example No. | Liver microsome species | $T_{1/2}$ (min) | $CL_{int(mic)}$ (μL/min/mg protein) | $CL_{int(liver)}$ (mL/min/K g) | Clearance rate classification |
|---|---|---|---|---|---|
| 9 | CD-1 mouse | 28.2 | 49.1 | 194.5 | Medium |
| | SD rat | 28.0 | 49.5 | 89.1 | Medium |
| | Human | 37.4 | 37.1 | 33.4 | Medium |
| 17 | CD-1 mouse | 79.8 | 17.4 | 68.8 | Medium |
| | SD rat | 32.43 | 42.7 | 76.9 | Medium |
| | Human | 45.0 | 30.8 | 27.7 | Medium |
| 24 | CD-1 mouse | 46.9 | 29.6 | 117.1 | Medium |
| | SD rat | 33.2 | 41.8 | 75.2 | Medium |
| | Human | 36.4 | 38.1 | 34.3 | Medium |
| 27 | CD-1 mouse | 85.80 | 16.2 | 64.0 | Medium |
| | SD rat | 97.86 | 14.2 | 25.5 | Medium |
| | Human | 84.94 | 16.3 | 14.7 | Medium |
| 28[a] | CD-1 mouse | 394.3 | 13.9 | 13.4 | Low |
| | SD rat | 890.9 | 2.8 | 2.7 | Low |
| | Human | 1180.3 | 1.1 | 1.0 | Low |
| 32[a] | CD-1 mouse | 120.6 6 | 11.5 | 45.5 | Medium |
| | SD rat | 64.81 | 21.4 | 38.5 | Medium |
| | Human | 120.8 6 | 11.5 | 10.3 | Medium |

(continued)

| Example No. | Liver microsome species | $T_{1/2}$ (min) | $CL_{int(mic)}$ (μL/min/mg protein) | $CL_{int(liver)}$ (mL/min/K g) | Clearance rate classification |
|---|---|---|---|---|---|
| 33[a] | CD-1 mouse | 109.7 4 | 12.6 | 50.0 | Medium |
| | SD rat | 91.41 | 15.2 | 27.3 | Medium |
| | Human | 485.1 3 | 2.9 | 2.6 | Low |
| Note: The clearance rate classification criteria are shown in the following table: | | | | | |

| Classification criteria | $CL_{int(liver)}$ (mL/min/kg) | | |
|---|---|---|---|
| | Human | SD rat | CD-1 mouse |
| Low | ≤8.9 | ≤23.7 | ≤38.6 |
| Medium | $8.9<CL_{int(liver)}\leq 48.3$ | $23.7<CL_{int(liver)}\leq 128.8$ | $38.6<CL_{int(liver)}\leq 210.0$ |
| High | >48.3 | > 128.8 | >210.0 |

4.5 Experimental conclusion

As can be seen from the results in Table 4 above, the compounds of the present invention have a medium clearance rate in SD rat and CD-1 mouse liver microsomes, and have a low-to-medium clearance rate in human liver microsomes. Based on the pharmacodynamic activity and pharmacological mechanism of the series of compounds, a slow metabolic rate is beneficial for the compounds to exert the pharmacodynamic activity, so the compounds of the present invention have a superior effect in the aspect of metabolic rate.

**Test Example *5. In Vitro* hERG Experiment for Compounds of the Present Invention**

5.1 Experimental materials

5.1.1 Experimental reagents

| Chemical reagent | Supplier | Cat. No. | Batch No. |
|---|---|---|---|
| DMEM | Biochannel | BC-M-004 | BC20210128 |
| FBS | LONSERA | S711-001S | Q708773 |
| TrypLE™ Express | Gibco | 12604-021 | 2167270 |
| Magnesium chloride | Sigma | M2393 | BCCD9633 |
| Sodium chloride | Sigma | S5886 | SLCF3558 |
| Potassium chloride | Sigma | P5405 | SLBM3653V |
| Ethylene glycol bis(2-aminoethyl ether)tetraacetic acid (EGTA) | Sigma | E3889 | SLCB4238 |
| 4-Hydroxyethyl piperazine ethanesulfonic acid (HEPES) | Sigma | V900477 | WXBD0664V |
| Potassium aspartate | Sigma | A6558 | SLBC2171V |
| Glucose | Sigma | G8270 | WXBD3622V |
| Calcium chloride | Sigma | C7902 | SLBM0710V |
| Sodium dihydrogen phosphate | admas | 81292B | P1533702 |
| Sodium adenosine triphosphate | Sigma | A2383 | SLBP6246V |

(continued)

| Chemical reagent | Supplier | Cat. No. | Batch No. |
|---|---|---|---|
| Phosphate buffer | Takara | T900 | AJ1P033 |
| 5.1.2 Experimental instruments | | | |
| Name | Supplier | Model | |
| Amplifier | HEKA | BioTech | |
| Micromanipulator | Sutter Instruments | 2105 | |
| Micropipette puller | Sutter Instruments | IQ 7000 | |
| Microscope | Olympus | TDZ5-WS | |
| Capillary glass tube | Sutter Instruments | D300e | |
| Data acquisition and analysis software | PatchMaster | Igor Pro 6.0/ GraphPad Prism 5.0 | |

5.1.3 Electrophysiological detection solutions

Extracellular fluid: 140 mM NaCl, 3.5 mM KCl, 1 mM $MgCl_2$, 2 mM $CaCl_2$, 10 mM Glucose, 10 mM HEPES, and 1.25 mM $NaH_2PO_4$, with the pH adjusted to 7.4 by NaOH. Intracellular fluid: 20 mM KCl, 115 mM K-Aspartic, 1 mM $MgCl_2$, 5 mM EGTA, 10 mM HEPES, and 2 mM $Na_2$-ATP, with the pH adjusted to 7.2 by KOH.

5.2 Patch clamping method

Under an inverted microscope, recording electrodes were controlled by a glass electrode micromanipulator (micromanipulation) to contact with the cell. A negative voltage was applied to form a GΩ seal. After forming the GΩ seal, a rapid capacitance compensation was given. Under the continuous negative voltage, the cell membrane was ruptured to establish a whole-cell recording mode. In the whole-cell recording mode, a slow capacitance compensation was given, and the values of membrane capacitance and series resistance were recorded.

Voltage stimulation scheme for cellular hERG potassium current: the cell membrane clamping voltage was -80 mV; the voltage was first elevated from -80 mV to +30 mV, held for 2.5 s, and rapidly changed to and held at -50 mV for 4 s, thus exciting the hERG channel tail current. Data were acquired every 10 s. Leak current detection was performed at -50 mV.

The coverslip with seeded cells was placed in the recording chamber of the inverted microscope. Negative control and test compounds flowed through the recording chamber in an ascending order of concentration by gravity perfusion to quickly act on the cells. During the recording, the extracellular fluid was continuously circulated by a vacuum pump. The current detected in a cell in the negative control was used as the background for the cell. Each compound concentration was allowed to act for 5 min or until the current was stabilized. All experiments were performed at room temperature.

5.3 Data analysis

The current of each compound concentration was first normalized according to (peak tail current compound)/(-peak tail current vehicle), and then the corresponding inhibition rate was calculated according to (1 - (peak tail current compound)/(peak tail current vehicle)). Basic statistics were calculated for each concentration, including mean, standard deviation (SD), standard error (SE), and replicates (n). The dose-dependent curve was fitted with the following equation and the half-maximal inhibitory concentrations ($IC_{50}$) of the test compounds were calculated:

$$\text{inhibition} = 1/(1 + (IC_{50}/C)\text{\^{}}h)$$

where C represents the test compound concentration, $IC_{50}$ represents the half-maximal inhibitory concentration, and h represents the Hill coefficient. Curve fitting and calculation of $IC_{50}$ were performed by GraphPad Prism 5.0 software.

5.4 Experimental results: See Table 5.

Table 5. Results of inhibitory activity experiment for compounds of the present invention on hERG potassium channel receptor

| Example No. | hERG $IC_{50}$ ($\mu M$) |
|---|---|
| 28[a] | 23.3 |
| 32[a] | 17.8 |
| 33[a] | 20.2 |
| Comparative Example 2 | 4.7 |
| Comparative Example 3 | 5.1 |
| Note: In the above table, a represents the free base form of the compound; and the others represent the hydrochloride/dihydrochloride salt form of the compound. | |

5.5 Experimental conclusion

As can be seen from the hERG inhibition test results in Table 5 above, most of the compounds described herein have weak or essentially no inhibitory activity on the hERG potassium channel. The hERG inhibition $IC_{50}$ values for Examples 28, 32, and 33 were all at 20 $\mu M$, with a functional activity 1000-fold or more lower than that of the pharmacodynamic target. Therefore, the potential cardiotoxicity risk of the series of compounds of the present invention is extremely low.

**Test Example 6. Test of Effects on Tacrine-Induced Jaw Tremor Behavior in Rats by Compounds of the Present Invention**

6.1 Experimental scheme

6.1.1 Experimental materials

Test compounds: The free bases of Examples 28, 32, and 33 in the present invention, which were self-prepared.
Positive control compound: Rotigotine.
Molding drug: Tacrine, SIGMA, A3773-1G.
Vehicle: Normal saline, Cisen Pharmaceutical Co., Ltd., 2111060723.

6.1.2 Experimental instruments

| Name | Model | Manufacturer |
|---|---|---|
| Precision electronic balance | SECURA225D-1CN | Sartorius Scientific Instruments (Beijing) Co., Ltd. |
| Electronic balance | YH-C 10001 | Dongyang Yingheng Intelligent Equipment Co., Ltd. |
| Counter | F60157 | Niuxiang Flagship Store |

6.1.3 Experimental animals

Sprague Dawley rats, male, 10/group, Shanghai Slac Laboratory Animal Co., Ltd.

6.1.4 Administration information

Drug preparation: The test compound was taken, added to the vehicle, and subjected to ultrasonication.
Administration route and method: Administration was performed by intraperitoneal injection.

Administration frequency and duration: single administration.

6.2 Experimental procedures

The rats were stratified according to body weight and then randomly divided into a model group, an administration group, and a control group. The rats were acclimatized for three days before the experiment. On the day of the experiment, the vehicle or drug was first injected intraperitoneally, followed by an intraperitoneal injection of tacrine at a dose of 5 mg/kg (body weight) after 60 min. After administration, the rats were placed in a transparent observation box, and the jaw tremor counting was started 10 min later. The continuous counting duration was 5 min, and the jaw tremor frequency in the rats within 5 min was recorded.

6.3 Data processing and statistics

The experimental data were expressed as $\bar{x} \pm$ SEM. Inhibition rate (%) = 100% $\times$ (jaw tremor frequency in the rats of the model group - jaw tremor frequency in the rats of the administration group)/jaw tremor frequency in the rats of the model group, where *: $p < 0.05$, **: $p < 0.01$, and ***: $p < 0.001$ vs vehicle.

6.4 Experimental results: See FIG. 1 (amelioration of tacrine-induced jaw tremors in rats by the compounds of the present invention at different concentrations).

6.5 Experimental conclusion

The tacrine-induced jaw tremor model test in rats was performed on the free bases of Examples 28, 32, and 33, with the anti-Parkinson's drug rotigotine used as a positive control drug, so as to investigate their potential anti-Parkinson's tremor symptom effects. The results are shown in FIGs. 1-3, compared to the model group, the positive control rotigotine group (10 mg/kg), and Examples 28 (1 mg/kg, 3 mg/kg, and 10 mg/kg), 32 (1 mg/kg, 3 mg/kg, and 10 mg/kg), and 33 (1 mg/kg, 3 mg/kg, and 10 mg/kg) significantly inhibited tacrine-induced jaw tremors in rats, with the minimum effective doses of Examples 28, 32, and 33 being lower than that of the positive control drug. The results suggest that the series of compounds of the present invention have the effect of ameliorating Parkinson's tremor symptoms.

Although specific embodiments of the present invention have been described in detail, various modifications and substitutions can be made to the details of the technical solutions of the present invention by those skilled in the art according to all the teachings that have been disclosed, and these changes are all encompassed within the protection scope of the present invention. The full scope of the present invention is given by the appended claims and any equivalent thereof.

## Claims

1. A compound of general formula (I), a stereoisomer thereof, a tautomer thereof, or a pharmaceutically acceptable salt thereof:

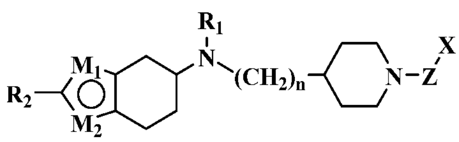

(I)

wherein

X is selected from amino, $C_{1-6}$ alkyl, $C_{2-6}$ alkenyl, $C_{2-6}$ alkynyl, $C_{3-8}$ cycloalkyl, 3- to 8-membered heterocyclyl comprising 1-4 heteroatoms selected from N, O, or S, $C_{6-10}$ aryl, 5- to 10-membered heteroaryl comprising 1-4 heteroatoms selected from N, O, or S, $C_{6-10}$ aryl-fused $C_{3-8}$ cycloalkyl, $C_{6-10}$ aryl-fused 3- to 8-membered heterocyclyl comprising 1-4 heteroatoms selected from N, O, or S, $C_{6-10}$ aryl-fused 5- to 10-membered heteroaryl comprising 1-4 heteroatoms selected from N, O, or S, (5- to 10-membered heteroaryl comprising 1-4 heteroatoms selected from N, O, or S)-fused $C_{3-8}$ cycloalkyl, or (5- to 10-membered heteroaryl comprising 1-4 heteroatoms selected from N, O, or S)-fused 3- to 8-membered heterocyclyl comprising 1-4 heteroatoms selected from N, O, or S, and optionally further substituted with one or more R;

R is selected from deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy, wherein the $C_{1-6}$ alkyl, $C_{1-6}$ haloalkyl, $C_{1-6}$ alkoxy, or $C_{1-6}$ haloalkoxy is optionally further substituted with one or more substituents selected from deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy;

Z is selected from a bond, $-(CR_{aa}R_{bb})_m-$, $-C(O)(CR_{aa}R_{bb})_m-$, $-O(CR_{aa}R_{bb})_m-$, $-(CR_{aa}R_{bb})_mO-$, $-(CR_{aa}R_{bb})_mN(R_{cc})-$, $-S(O)(CR_{aa}R_{bb})_m-$, $-S(O)_2(CR_{aa}R_{bb})_m-$, $-C(O)(CR_{aa}R_{bb})_mN(R_{cc})-$, $-C(S)(CR_{aa}R_{bb})_mN(R_{cc})-$, or $-C(S)(CR_{aa}R_{bb})_m-$;

$M_1$ and $M_2$ are each independently selected from $CR_{aa}$, N, O, or S;

$R_{aa}$ and $R_{bb}$ are each independently selected from hydrogen, deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy, wherein the $C_{1-6}$ alkyl, $C_{1-6}$ haloalkyl, $C_{1-6}$ alkoxy, or $C_{1-6}$ haloalkoxy is optionally further substituted with one or more substituents selected from deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy;

$R_{cc}$ is selected from hydrogen, deuterium, $C_{1-6}$ alkyl, $C_{1-6}$ haloalkyl, $C_{1-6}$ alkoxy, or $C_{1-6}$ haloalkoxy, wherein the $C_{1-6}$ alkyl, $C_{1-6}$ haloalkyl, $C_{1-6}$ alkoxy, or $C_{1-6}$ haloalkoxy is optionally further substituted with one or more substituents selected from deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy;

$R_1$ is selected from hydrogen, deuterium, $C_{1-6}$ alkyl, $C_{2-6}$ alkenyl, $C_{2-6}$ alkynyl, $C_{1-6}$ haloalkyl, $C_{1-6}$ alkoxy, or $C_{1-6}$ haloalkoxy, wherein the $C_{1-6}$ alkyl, $C_{2-6}$ alkenyl, $C_{2-6}$ alkynyl, $C_{1-6}$ haloalkyl, $C_{1-6}$ alkoxy, or $C_{1-6}$ haloalkoxy is optionally further substituted with one or more substituents selected from deuterium, halogen, hydroxyl, amino, sulfydryl, nitryl, cyano, $C_{1-6}$ alkyl, $C_{1-6}$ haloalkyl, $C_{1-6}$ alkoxy, or $C_{1-6}$ haloalkoxy;

$R_2$ is selected from hydroxyl, amino, sulfydryl, $C_{1-6}$ alkyl, $C_{1-6}$ haloalkyl, $C_{1-6}$ alkoxy, or $C_{1-6}$ haloalkoxy;

n is selected from 0, 1, 2, 3, or 4; and

m is selected from 0, 1, 2, 3, 4, 5, or 6.

**2.** The compound, the stereoisomer thereof, the tautomer thereof, or the pharmaceutically acceptable salt thereof according to claim 1, wherein one or more of the following conditions are met:

(1)

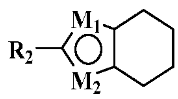

is selected from the following groups:

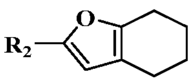, 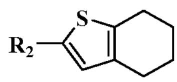,

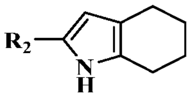, 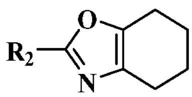, 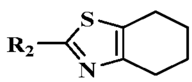, or 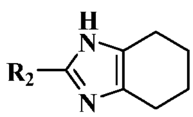,

preferably

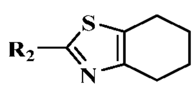;

(2) X is selected from amino, $C_{3-8}$ cycloalkyl, 3- to 8-membered heterocyclyl comprising 1-4 heteroatoms selected from N, O, or S, $C_{6-10}$ aryl, 5- to 10-membered heteroaryl comprising 1-4 heteroatoms selected from N, O, or S, or $C_{6-10}$ aryl-fused 5- to 10-membered heteroaryl comprising 1-4 heteroatoms selected from N, O, or S, and optionally further substituted with one or more R, preferably X is selected from amino, $C_{4-6}$ cycloalkyl, 4- to 6-membered heterocyclyl comprising 1-3 heteroatoms selected from N, O, or S, $C_{6-10}$ aryl, 5- to 10-membered heteroaryl comprising 1-4 heteroatoms selected from N, O, or S, or benzo-fused 5- to 6-membered heteroaryl comprising 1-3 heteroatoms selected from N, O, or S, and optionally further substituted with one or more R, and

more preferably X is selected from the following groups:

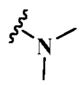, 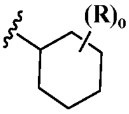, 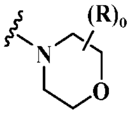,

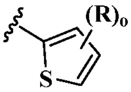, 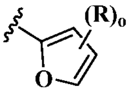, 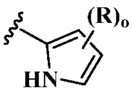, 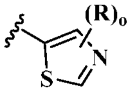, 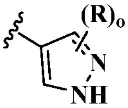, 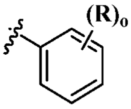, 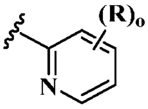,

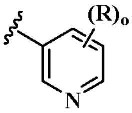, 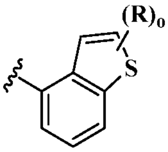, 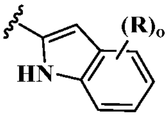, 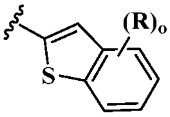, or 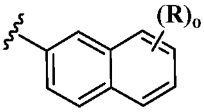,

wherein o is selected from 0, 1, 2, 3, 4, or 5;

(3) R is selected from hydrogen, deuterium, halogen, hydroxyl, amino, cyano, $C_{1-6}$ alkyl, $C_{1-6}$ haloalkyl, $C_{1-6}$ alkoxy, or $C_{1-6}$ haloalkoxy, preferably hydrogen, deuterium, halogen, cyano, or $C_{1-6}$ alkyl, more preferably hydrogen, deuterium, fluorine, chlorine, bromine, cyano, or $C_{1-3}$ alkyl, and further preferably hydrogen, deuterium, fluorine, chlorine, cyano, or methyl;

(4) Z is selected from a bond, $-(CH_2)_m-$, -C(O)-, $-C(O)(CH_2)_m-$, $-S(O)_2-$, $-S(O)_2(CH_2)_m-$, or $-C(O)N(CH_3)-$, preferably a bond, -C(O)-, $-S(O)_2-$, or $-C(O)N(CH_3)-$;

(5) $R_1$ is selected from hydrogen, deuterium, $C_{1-6}$ alkyl, $C_{2-6}$ alkenyl, $C_{2-6}$ alkynyl, or $C_{1-6}$ alkoxy, preferably hydrogen, deuterium, or $C_{1-6}$ alkyl, more preferably hydrogen, deuterium, or $C_{1-3}$ alkyl, and further preferably hydrogen, deuterium, methyl, ethyl, or propyl;

(6) $R_2$ is selected from hydroxyl, amino, $C_{1-6}$ alkyl, or $C_{1-6}$ haloalkyl, preferably hydroxyl, amino, $C_{1-3}$ alkyl, or $C_{1-3}$ haloalkyl, more preferably hydroxyl or amino, and further preferably amino;

(7) n is selected from 0, 1, 2, or 3, preferably 0, 1, or 2; and

(8) m is selected from 0, 1, 2, or 3, preferably 0 or 1.

**3.** The compound, the stereoisomer thereof, the tautomer thereof, or the pharmaceutically acceptable salt thereof according to claim 1 or 2, wherein general formula (I) further has a structure represented by general formula (II):

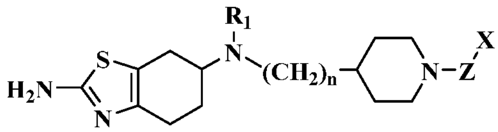

**(II)**

wherein

X, Z, $R_1$, and n are as defined in claim 1 or 2.

**4.** The compound, the stereoisomer thereof, the tautomer thereof, or the pharmaceutically acceptable salt thereof according to claim 3, wherein general formula (I) further has a structure represented by general formula (III):

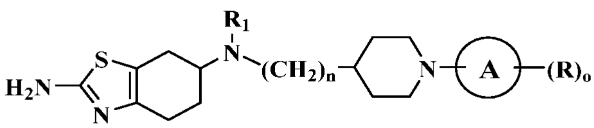

**(III)**

wherein

ring A is selected from $C_{6-10}$ aryl, 5- to 10-membered heteroaryl comprising 1-4 heteroatoms selected from N, O, or S, or $C_{6-10}$ aryl-fused 5- to 10-membered heteroaryl comprising 1-4 heteroatoms selected from N, O, or S, preferably $C_{6-10}$ aryl or benzo-fused 5- to 6-membered heteroaryl comprising 1-3 heteroatoms selected from N, O, or S, and more preferably phenyl or benzothienyl;

R is selected from hydrogen, deuterium, halogen, hydroxyl, amino, cyano, $C_{1-6}$ alkyl, $C_{1-6}$ haloalkyl, $C_{1-6}$ alkoxy, or $C_{1-6}$ haloalkoxy, preferably hydrogen, deuterium, halogen, cyano, or $C_{1-6}$ alkyl, more preferably hydrogen, deuterium, fluorine, chlorine, bromine, cyano, or $C_{1-3}$ alkyl, and further preferably hydrogen, deuterium, fluorine, chlorine, cyano, or methyl;

o is selected from 0, 1, 2, or 3, preferably 0, 1, or 2; and

$R_1$ and n are as defined in claim 3.

**5.** The compound, the stereoisomer thereof, the tautomer thereof, or the pharmaceutically acceptable salt thereof according to claim 4, wherein

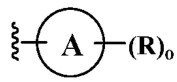

in general formula (III) is selected from the following groups:

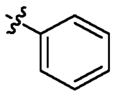,

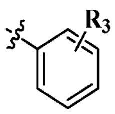, 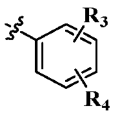, 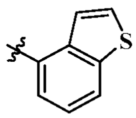, 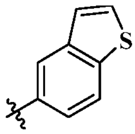, 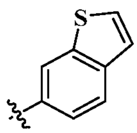, or 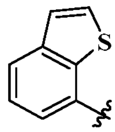,

preferably

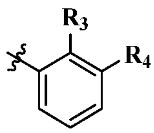 or 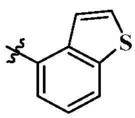,

wherein

$R_3$ and $R_4$ are each independently selected from halogen, hydroxyl, amino, cyano, $C_{1-6}$ alkyl, $C_{1-6}$ haloalkyl, $C_{1-6}$ alkoxy, or $C_{1-6}$ haloalkoxy, preferably halogen, cyano, or $C_{1-6}$ alkyl, more preferably fluorine, chlorine, or bromine, and further preferably chlorine.

**6.** The compound, the stereoisomer thereof, the tautomer thereof, or the pharmaceutically acceptable salt thereof according to claim 3, wherein general formula (I) further has a structure represented by general formula (IV):

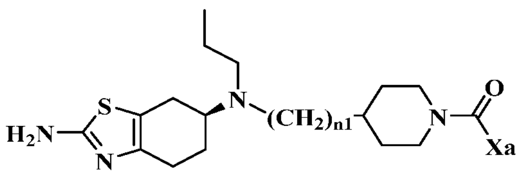

**(IV)**

wherein

Xa is selected from amino, $C_{3-8}$ cycloalkyl, 3- to 8-membered heterocyclyl comprising 1-4 heteroatoms selected from N, O, or S, $C_{6-10}$ aryl, or 5- to 10-membered heteroaryl comprising 1-4 heteroatoms selected from N, O, or S,

and optionally further substituted with one or more $R_a$, preferably Xa is selected from amino, $C_{4-6}$ cycloalkyl, 4- to 6-membered heterocyclyl comprising 1-3 heteroatoms selected from N, O, or S, $C_{6-10}$ aryl, or 5- to 10-membered heteroaryl comprising 1-4 heteroatoms selected from N, O, or S, and optionally further substituted with one or more $R_a$, and more preferably Xa is selected from the following groups:

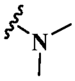 , 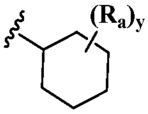 , 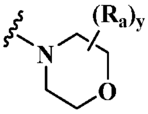 , 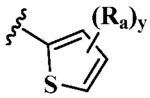 , 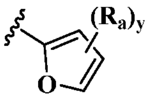 ,

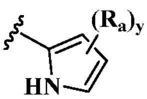 , 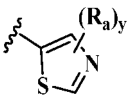 , 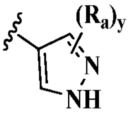 , 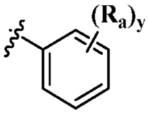 , 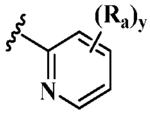 , 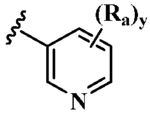 ,

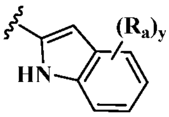 , 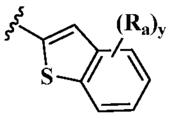 , or 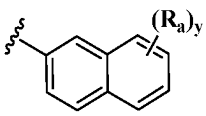 ,

wherein y is selected from 0, 1, 2, or 3, preferably 0, 1, or 2;
$R_a$ is selected from hydrogen, deuterium, halogen, hydroxyl, amino, cyano, $C_{1-6}$ alkyl, $C_{1-6}$ haloalkyl, $C_{1-6}$ alkoxy, or $C_{1-6}$ haloalkoxy, preferably hydrogen, deuterium, halogen, cyano, or $C_{1-6}$ alkyl, more preferably hydrogen, deuterium, fluorine, chlorine, bromine, cyano, or $C_{1-3}$ alkyl, and further preferably hydrogen, deuterium, fluorine, chlorine, cyano, or methyl; and
n1 is selected from 0, 1, 2, 3, or 4, preferably 0, 1, or 2, and more preferably 1.

**7.** The compound, the stereoisomer thereof, the tautomer thereof, or the pharmaceutically acceptable salt thereof according to claim 6, wherein

Xa is selected from the following groups:

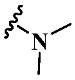 , 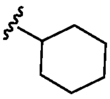 , 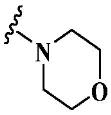 , 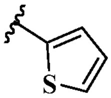 ,

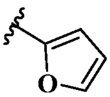 , 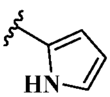 , 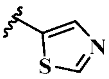 , 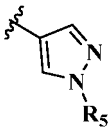 , 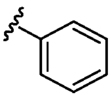 , 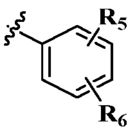 , 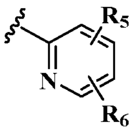 , 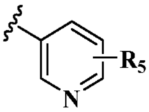 ,

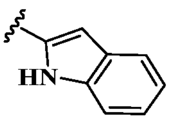 , 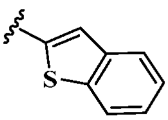 , or 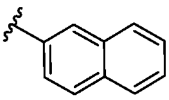 ,

wherein $R_5$ and $R_6$ are each independently selected from halogen, hydroxyl, amino, cyano, $C_{1-6}$ alkyl, $C_{1-6}$ haloalkyl, $C_{1-6}$ alkoxy, or $C_{1-6}$ haloalkoxy, preferably halogen, cyano, or $C_{1-6}$ alkyl, more preferably fluorine, chlorine, bromine, cyano, or $C_{1-3}$ alkyl, and further preferably fluorine, chlorine, cyano, or methyl.

**8.** The compound, the stereoisomer thereof, the tautomer thereof, or the pharmaceutically acceptable salt thereof according to any one of claims 1-7, selected from the following compounds:

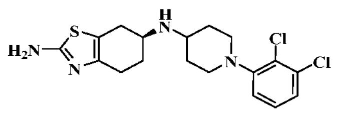
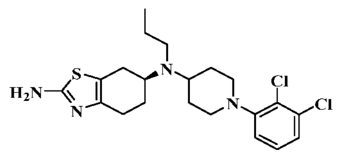
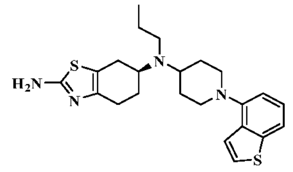

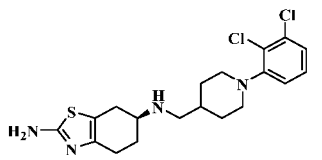
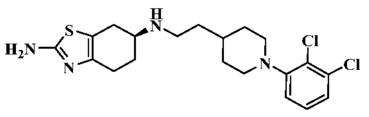
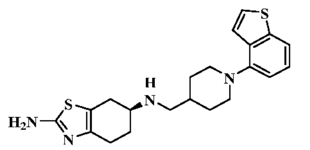

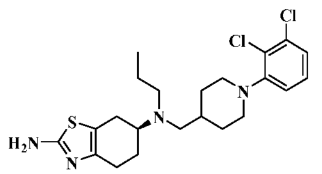
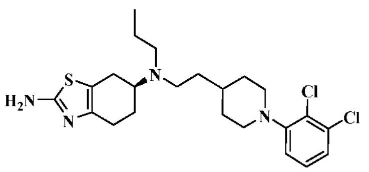
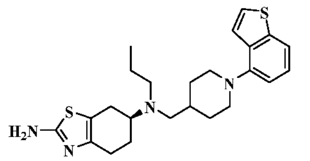

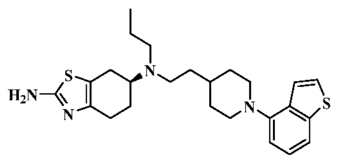
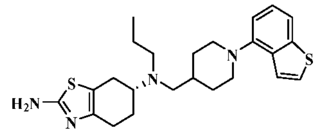
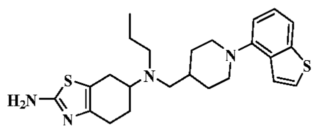

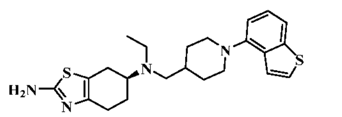
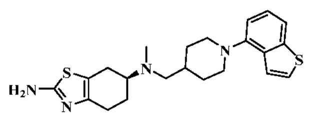
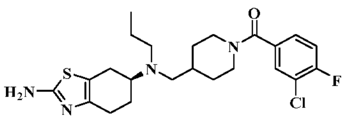

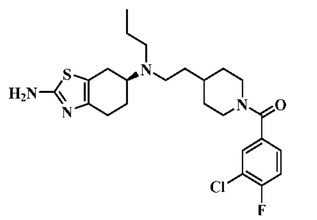
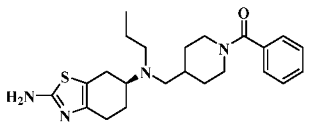
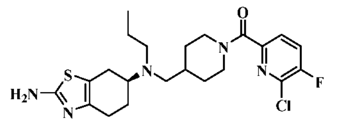

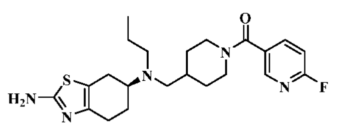
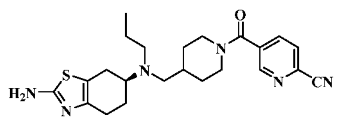
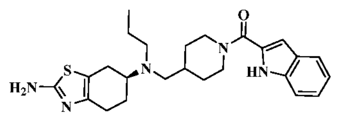

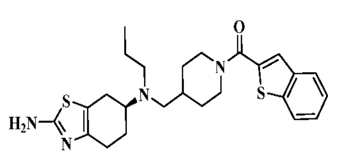
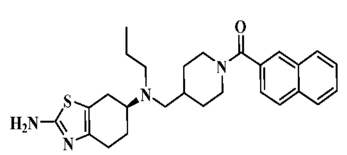
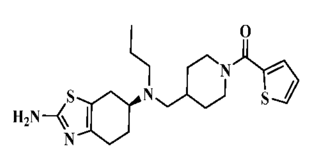

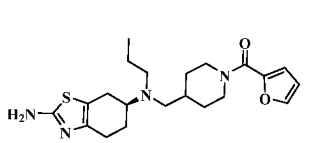
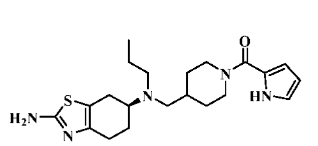
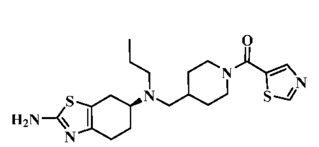

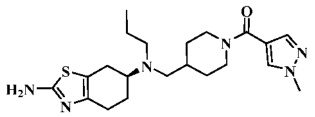 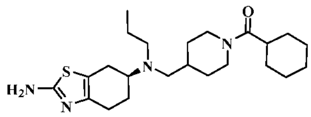 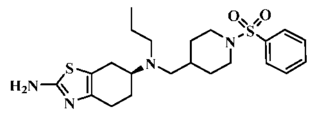

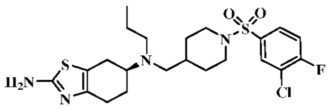 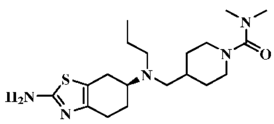 or 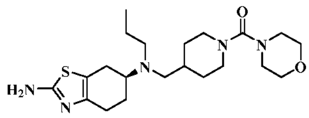 .

9. A compound of general formula (V), a stereoisomer thereof, a tautomer thereof, or a pharmaceutically acceptable salt thereof:

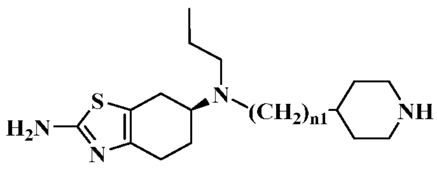

(V)

wherein
n1 is selected from 0, 1, 2, 3, or 4, preferably 0, 1, or 2, and more preferably 1.

10. A method for preparing the compound, the stereoisomer thereof, the tautomer thereof, or the pharmaceutically acceptable salt thereof according to claim 6, comprising the following step:

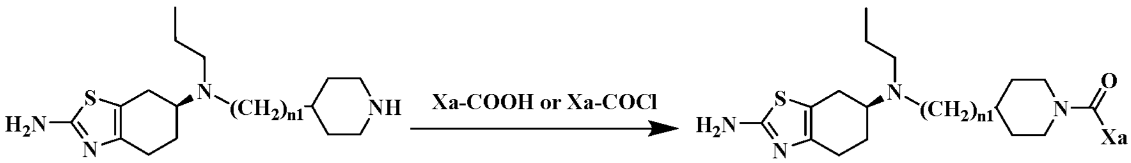


(V) (IV)

conducting a condensation reaction of a compound of general formula (V) with a substituted carboxylic acid or acyl chloride to obtain the compound of general formula (IV), the stereoisomer thereof, the tautomer thereof, or the pharmaceutically acceptable salt thereof,
wherein n1 and Xa are as described in claim 6.

11. A pharmaceutical composition, comprising a therapeutically effective amount of the compound, the stereoisomer thereof, the tautomer thereof, or the pharmaceutically acceptable salt thereof according to any one of claims 1-8, and at least one pharmaceutical adjuvant of a pharmaceutically acceptable carrier, diluent or excipient.

12. Use of the compound, the stereoisomer thereof, the tautomer thereof, or the pharmaceutically acceptable salt thereof according to any one of claims 1-8, or the pharmaceutical composition according to claim 11 in the preparation of a medicament involving or regulating a 5-hydroxytryptamine receptor and/or a dopamine receptor, preferably in the preparation of a medicament involving or regulating a 5-$HT_{1A}$ receptor, a dopamine $D_2$ receptor, and/or a dopamine $D_3$ receptor.

13. Use of the compound, the stereoisomer thereof, the tautomer thereof, or the pharmaceutically acceptable salt thereof according to any one of claims 1-8, or the pharmaceutical composition according to claim 11 in the preparation of a medicament for treating a central nervous system disease.

14. The use according to claim 13, wherein the central nervous system disease is selected from one or more of Parkinson's disease, schizophrenia, bipolar disorder, depression, anxiety, mania, Huntington's disease, Alzheimer's disease, senile dementia, dementia of the Alzheimer's type, memory disorder, loss of executive function, vascular

dementia, neuropathic pain and dysfunctional diseases related to intelligence, learning, or memory, glaucoma, age-related macular degeneration, optic neuritis, ischemic disorder, and retinal edema, preferably Parkinson's disease.

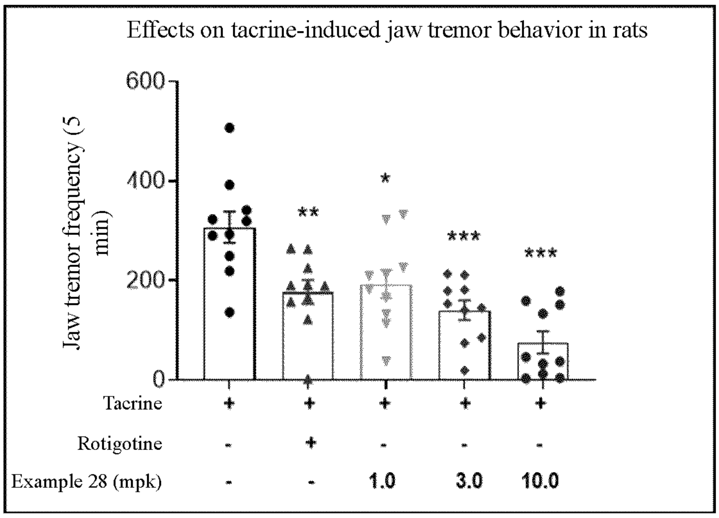


FIG. 1

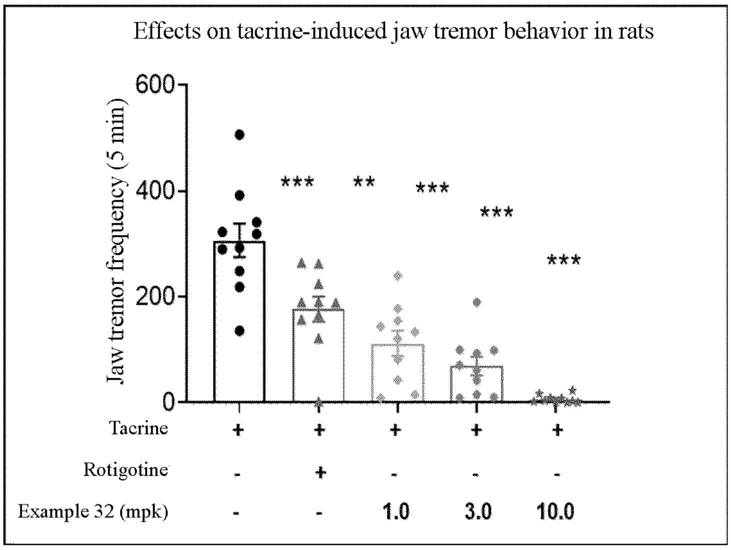


FIG. 2

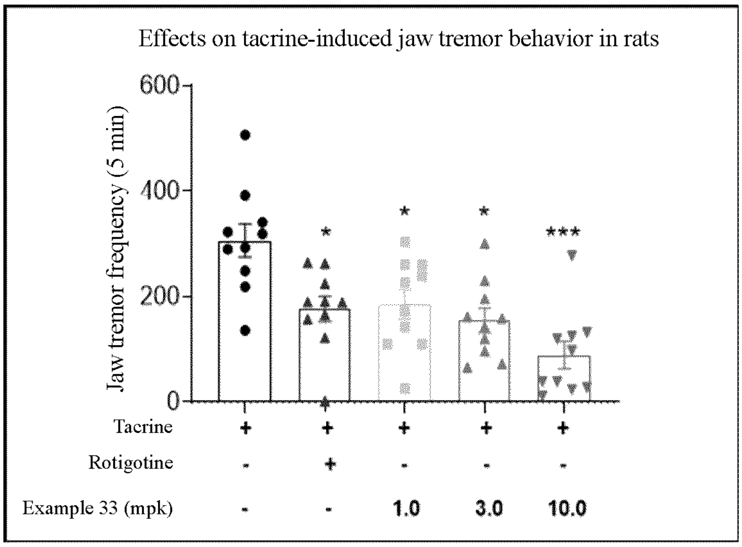


FIG. 3

## INTERNATIONAL SEARCH REPORT

International application No.
**PCT/CN2023/111410**

**A. CLASSIFICATION OF SUBJECT MATTER**

C07D 417/12(2006.01)i; C07D211/06(2006.01)i; C07D277/60(2006.01)i; C07D221/00(2006.01)i; A61K31/39(2006.01)i; A61K31/4427(2006.01)i; A61P25/16(2006.01)i; A61P25/24(2006.01)i; A61P25/22(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, ENTXT, DWPI, CJFD, CNKI, Caplus(STN), Registry(STN): 江苏恩华药业, 噻唑, 环己烷, 哌啶, 氨基, 多巴胺, 帕金森, thiazole, cyclohexane, piperidine, amino, dopamine, Parkinson's, 结构检索, structural research

**C. DOCUMENTS CONSIDERED TO BE RELEVANT**

| Category* | Citation of document, with indication, where appropriate, of the relevant passages | Relevant to claim No. |
|---|---|---|
| A | US 2005222175 A1 (DHANOA, Dale S. et al.) 06 October 2005 (2005-10-06)<br>claims 1-45, and description, page 56, compound B18 | 1-14 |
| A | CN 113121417 A (SUZHOU SUNCADIA BIOPHARMACEUTICALS CO., LTD. et al.) 16 July 2021 (2021-07-16)<br>entire document | 1-14 |
| A | US 2020276196 A1 (SUNSHINE LAKE PHARMA CO., LTD. et al.) 03 September 2020 (2020-09-03)<br>entire document | 1-14 |
| A | CN 1884262 A (POISON MEDICINE INSTITUTE OF PLA MILITARY MEDICALACADEMY OF SCIENCES) 27 December 2006 (2006-12-27)<br>entire document | 1-14 |
| A | US 2009062261 A1 (SHIONOGI & CO., LTD.) 05 March 2009 (2009-03-05)<br>entire document | 1-14 |
| A | CN 101990535 A (MERCK PATENT GMBH) 23 March 2011 (2011-03-23)<br>entire document | 1-14 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **03 November 2023** | **10 November 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/111410**

**C. DOCUMENTS CONSIDERED TO BE RELEVANT**

| Category* | Citation of document, with indication, where appropriate, of the relevant passages | Relevant to claim No. |
|---|---|---|
| A | CN 1518547 A (WYETH LLC) 04 August 2004 (2004-08-04)<br>entire document | 1-14 |

Form PCT/ISA/210 (second sheet) (July 2022)

**INTERNATIONAL SEARCH REPORT**
**Information on patent family members**

International application No.
**PCT/CN2023/111410**

| Patent document cited in search report | | | Publication date (day/month/year) | Patent family member(s) | | | Publication date (day/month/year) |
|---|---|---|---|---|---|---|---|
| US | 2005222175 | A1 | 06 October 2005 | ES | 2351110 | T3 | 31 January 2011 |
| | | | | US | 2011082157 | A1 | 07 April 2011 |
| CN | 113121417 | A | 16 July 2021 | None | | | |
| US | 2020276196 | A1 | 03 September 2020 | CA | 3077383 | A1 | 04 April 2019 |
| | | | | US | 11285153 | B2 | 29 March 2022 |
| | | | | WO | 2019062662 | A1 | 04 April 2019 |
| | | | | EP | 3687989 | A1 | 05 August 2020 |
| | | | | EP | 3687989 | A4 | 19 May 2021 |
| | | | | JP | 2020535205 | A | 03 December 2020 |
| | | | | JP | 7282082 | B2 | 26 May 2023 |
| | | | | AU | 2018340376 | A1 | 26 March 2020 |
| | | | | AU | 2018340376 | B2 | 01 September 2022 |
| CN | 1884262 | A | 27 December 2006 | None | | | |
| US | 2009062261 | A1 | 05 March 2009 | WO | 2007099828 | A1 | 07 September 2007 |
| | | | | EP | 2520567 | A2 | 07 November 2012 |
| | | | | EP | 2520567 | A3 | 12 December 2012 |
| | | | | EP | 1988077 | A1 | 05 November 2008 |
| | | | | EP | 1988077 | A4 | 02 September 2009 |
| | | | | US | 7935706 | B2 | 03 May 2011 |
| | | | | JPWO | 2007099828 | A1 | 16 July 2009 |
| | | | | US | 2011172415 | A1 | 14 July 2011 |
| | | | | TW | 200800182 | A | 01 January 2008 |
| CN | 101990535 | A | 23 March 2011 | SI | 2262785 | T1 | 29 May 2015 |
| | | | | MX | 2010011292 | A | 10 November 2010 |
| | | | | HK | 1155741 | A1 | 25 May 2012 |
| | | | | ECSP | 10010615 | A | 30 December 2010 |
| | | | | CY | 1116333 | T1 | 08 February 2017 |
| | | | | ES | 2531006 | T3 | 09 March 2015 |
| | | | | WO | 2009127321 | A1 | 22 October 2009 |
| | | | | UA | 104140 | C2 | 10 January 2014 |
| | | | | PT | 2262785 | E | 29 April 2015 |
| | | | | HRP | 20150319 | T1 | 19 June 2015 |
| | | | | IL | 208322 | A0 | 30 December 2010 |
| | | | | IL | 208322 | A | 29 February 2016 |
| | | | | PL | 2262785 | T3 | 30 April 2015 |
| | | | | DK | 2262785 | T3 | 16 February 2015 |
| | | | | EA | 201001647 | A1 | 30 June 2011 |
| | | | | EA | 020454 | B1 | 28 November 2014 |
| | | | | EP | 2110374 | A1 | 21 October 2009 |
| | | | | BRPI | 0911243 | A2 | 06 October 2015 |
| | | | | BRPI | 0911243 | B1 | 08 October 2019 |
| | | | | BRPI | 0911243 | B8 | 25 May 2021 |
| | | | | JP | 2014237676 | A | 18 December 2014 |
| | | | | JP | 5997730 | B2 | 28 September 2016 |
| | | | | AR | 071207 | A1 | 02 June 2010 |
| | | | | ZA | 201008243 | B | 25 April 2012 |
| | | | | US | 2011105475 | A1 | 05 May 2011 |
| | | | | US | 8575161 | B2 | 05 November 2013 |
| | | | | MY | 156530 | A | 26 February 2016 |

Form PCT/ISA/210 (patent family annex) (July 2022)

**INTERNATIONAL SEARCH REPORT**
**Information on patent family members**

International application No.
**PCT/CN2023/111410**

| Patent document cited in search report | Publication date (day/month/year) | Patent family member(s) | Publication date (day/month/year) |
|---|---|---|---|
| | | JP 2011516589 A | 26 May 2011 |
| | | JP 5616326 B2 | 29 October 2014 |
| | | NZ 589327 A | 29 June 2012 |
| | | KR 20110002478 A | 07 January 2011 |
| | | KR 101662853 B1 | 05 October 2016 |
| | | EP 2262785 A1 | 22 December 2010 |
| | | EP 2262785 B1 | 14 January 2015 |
| | | AU 2009238015 A1 | 22 October 2009 |
| | | AU 2009238015 B2 | 12 September 2013 |
| | | CA 2721591 A1 | 22 October 2009 |
| | | CA 2721591 C | 09 August 2016 |
| | | CO 6311099 A2 | 22 August 2011 |
| CN 1518547 A | 04 August 2004 | EA 200301142 A1 | 26 February 2004 |
| | | HU 0303958 A2 | 28 April 2004 |
| | | AR 035235 A1 | 05 May 2004 |
| | | US 2003069278 A1 | 10 April 2003 |
| | | US 6815456 B2 | 09 November 2004 |
| | | US 2005065186 A1 | 24 March 2005 |
| | | NO 20034648 D0 | 17 October 2003 |
| | | NO 20034648 L | 20 November 2003 |
| | | ECSP 034810 A | 01 December 2003 |
| | | WO 02085892 A1 | 31 October 2002 |
| | | KR 20030088508 A | 19 November 2003 |
| | | IL 158445 A0 | 12 May 2004 |
| | | JP 2004526787 A | 02 September 2004 |
| | | JP 4323810 B2 | 02 September 2009 |
| | | EP 1385842 A1 | 04 February 2004 |
| | | ECSP 034811 A | 01 December 2003 |
| | | PL 367297 A1 | 21 February 2005 |
| | | BR 0209056 A | 10 August 2004 |
| | | MXPA 03009476 A | 12 February 2004 |
| | | CA 2444095 A1 | 31 October 2002 |
| | | ZA 200309004 B | 21 February 2005 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2022109619064
- EP 0771563 A2
- US 5693654 A
- WO 9220333 A
- JP 2005104885 A


### Non-patent literature cited in the description


- **LILIENFELD, D. E.** *Neuroepidemiology*, 1993, vol. 12, 219-228
- **ARMSTRONG MJ**. *JAMA*, 2020, vol. 323 (6), 548-560
- **JOST WH**. *J Neurol.*, February 2003, vol. 250 (1), 128-30
- **HALLIDAY, G.M**. *Mov. Disord*, 2014, vol. 29, 634-650
- **GALLAGHER, D.A.** *Neurobiol. Dis.*, 2012, vol. 46, 581-589
- **SCHAPIRA, A. H.** *Neurol. Clin.*, 2009, vol. 27, 583-603
- **TITOVA N.** *Med J Aust*, 2018, vol. 208, 404-409
- **TITOVA N.** *J Neural Transm (Vienna)*, 2017, vol. 124, 907-914
- **JANKOVIC J**. *Journal of Neurology, Neurosurgery & Psychiatry*, 2020, vol. 91, 795-808
- **ARMSTRONG MJ.** *JAMA*, 2020, vol. 323 (6), 548-560
- **MARSDEN, C. D**. *Lancet*, 1976, vol. 307, 292-296
- **HISAHARA S**. *Int J Med Chem.*, 2011, vol. 2011, 403039
- **JANKOVIC J.** *Journal of Neurology, Neurosurgery & Psychiatry*, 2020, vol. 91, 795-808
- **TORTI M.** *Drugs*, May 2019, vol. 79 (7), 693-703
- **CERRI S**. *Expert Opin Investig Drugs.*, July 2017, vol. 26 (7), 777-791
- **OHNO Y**. *Prog Neurobiol*, November 2015, vol. 134, 104-21
- **MIYAZAKI I.** *Curr Med Chem.*, 2016, vol. 23 (7), 686-700
- **MARTIN et al.** *Ophthalmol.*, 1988, vol. 95, 1221-1226
- **MALLORGA** ; **SUGRUE**. *Curr. Eye Res.*, 1987, vol. 6, 527-532
- **CHIDLOW et al.** *Invest. Ophthalmol. Vis. Sci.*, 1995, vol. 36, 2238-2245
- **TOBIN** ; **OSBORNE**. *J. Neurochem.*, 1989, vol. 53, 686-601
- **TOBIN et al.** *J. Neurosci*
- **BARNET** ; **OSBORNE**. *Exp. Eye Res.*, 1993, vol. 57, 209-216
- **CHIDLOW et al.** *Invest. Ophthalmol. Vis. Sci.*
- **OSBORNE** ; **CHIDLOW**. *Ophthalmologica*, 1996, vol. 210, 308-314
- **OSBORNE** ; **CHIDLOW**. *Ophthalmologica*, 1996, vol. 210, 308-319
- **WANG et al.** *Curr. Eye Res.*, 1997, vol. 16, 679-775
- **CABEDO N.** *Journal of Medicinal Chemistry*, 2001, vol. 44 (11), 1794-1801
- **NOUREDDINE EL AOUAD.** *European Journal of Medicinal Chemistry*, vol. 44 (11), 4616-4621
- **OHNO Y**. *Prog Neurobiol.*, November 2015, vol. 134, 104-21
- **SHIMIZU S.** *Aging Dis*, February 2013, vol. 4 (1), 1-13
- **WANG, Q**. *Neuropharmacology*, 2019, vol. 148, 1-10
- **JONES CA**. *Eur Neuropsychopharmacol.*, August 2010, vol. 20 (8), 582-93
- **GLENNON, J. C**. *Synapse*, 2006, vol. 60, 599-608
- **BISWAS S.** *J Med Chem.*, 2008, vol. 51 (10), 3005-3019
- **BISWAS S**. *J Med Chem.*, 2008, vol. 51 (10), 3005-3019